# EUROPEAN PATENT APPLICATION

(11) **EP 4 194 435 A1**
(43) Date of publication of application: **14.06.2023**
(21) Application number: 21853226.5
(22) Date of filing: 06.08.2021
(51) Int. Cl.: C07C 49/753, A61K 31/075, A61K 31/122, A61K 31/13, A61K 31/16, A61K 31/27, A61K 31/275, A61P 13/10, A61P 21/00, A61P 25/00, A61P 25/02, A61P 25/04, A61P 25/28, A61P 29/02, A61P 43/00, C07C 235/78, C07C 255/31, C07C 271/12

(54) **NOVEL CYCLOALKENONE COMPOUND OR SALT THEREOF**

(30) Priority: 07.08.2020 JP 2020135328
(71) Applicant: Taiho Pharmaceutical Co., Ltd., Chiyoda-ku, Tokyo 101-8444 (JP)
(72) Inventor: SHIGENO, Kazuhiko, Tsukuba-shi, Ibaraki 300-2611 (JP); NOMA, Takahisa, Tokyo 101-0047 (JP); HAKOZAKI, Atsushi, Tsukuba-shi, Ibaraki 300-2611 (JP)
(74) Representative: Hoffmann Eitle
(86) International application number: PCT/JP2021/029246
(87) International publication number: WO 2022/030606

(57) **Abstract**

Provided are a compound represented by formula (I) or a salt thereof, a nerve growth promoter containing the compound or a salt thereof as an active ingredient, a pharmaceutical composition containing the compound or a salt thereof, a therapeutic agent for neurodegenerative diseases containing the compound or a salt thereof as an active ingredient, a therapeutic agent for amyotrophic lateral sclerosis containing the compound or a salt thereof as an active ingredient, a therapeutic agent for pain containing the compound or a salt thereof as an active ingredient, and a therapeutic agent for lower urinary tract dysfunction containing the compound or a salt thereof as an active ingredient.

## Description

### Technical Field

The present disclosure relates to a novel cycloalkenone compound or a salt thereof having a nerve growth stimulation effect, and to a pharmaceutical composition containing the compound or a salt thereof as an active ingredient.

### Background Art

Neurodegenerative diseases are broadly divided into central and peripheral disorders according to the site of damaged nerves. Typical diseases of the central nervous system include Alzheimer's disease, amyotrophic lateral sclerosis, and cerebrospinal cord injury. Typical diseases involving peripheral nerve damage include sensory neuropathy such as neuropathic pain and hypoesthesia, and dysautonomia accompanied by constipation or dysuria. Although the pathogenesis of the condition in neurodegenerative diseases varies, the diseases are thought to involve the degeneration or atrophy of neurites and neuronal cell death. For those diseases, there is currently a paucity of fundamental therapeutic agents that ameliorate neuropathy.

PTL 1 discloses that 3-(15-hydroxypentadecyl)-2,4,4-trimethylcyclohex-2-en-1-one has a nerve growth stimulation effect and is useful as a medical drug for prevention or treatment of brain diseases such as dementia. PTL 2 discloses that the compound is useful as a therapeutic agent for dysuria. However, 3-(15-hydroxypentadecyl)-2,4,4-trimethylcyclohex-2-en-1-one is unsatisfactory in pharmacokinetics when usually prescribed (PTL 3).

### Citation List

### Patent Literature

PTL 1: WO1999/008987A
PTL 2: WO2002/066024A
PTL 3: WO2013/147072A

### Summary of Invention

### Technical Problem

An object of the present disclosure is to provide a novel compound with excellent neurite outgrowth action or a salt thereof and a pharmaceutical composition containing the compound or a salt thereof.

### Solution to Problem

The present inventors conducted extensive research to achieve the object and found that a cycloalkenone compound represented by formula (I) has a nerve growth stimulation effect (neurite outgrowth action and/or action of increasing the proportion of neurite-bearing cells) and is useful as a medical drug for treating diseases that are ameliorated by the promotion of nerve growth (including neurodegenerative diseases), pain, and/or lower urinary tract dysfunction.

Specifically, the present disclosure provides the following [1] to [15].
[1] A compound represented by the following formula (I) or a salt thereof: 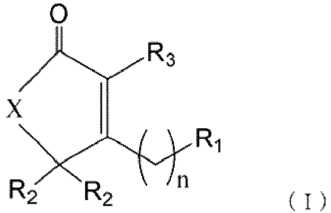 wherein
   X represents -CH₂ -, -CH(CH₃)-, -CH₂-CH₂-, or -CH=CH-,
   R₂ represents -CH₂OR₄, -CH₂OCON(R₅)₂, -CONHR₆, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OR₇, or a halogen atom,
   R₄ represents CₘH₂ₘ₊₁,
   R₅s are identical or different and each represent a hydrogen atom or a methyl group,
   R₆ represents a hydrogen atom or a methyl group,
   R₇ represents a C1-C3 alkyl group,
   m represents an integer of 1 to 14, and
   n represents an integer of 1 to 15,
   with the proviso that when R₄ is a linear alkyl group, the sum of m and n is an integer of 12 to 17, and when R₄ is a branched alkyl group, the sum of m_{b} and n is an integer of 12 to 17, wherein m_{b} is the number of carbon atoms of the longest linear chain of the branched alkyl group.
[2] A compound represented by the following formula (I) or a salt thereof: 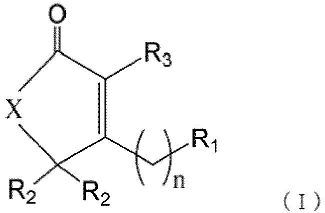 wherein
   X represents -CH₂ -, -CH(CH₃)-, -CH₂-CH₂-, or -CH=CH-,
   R₁ represents -CH₂OR₄, -CH₂OCON(R₅)₂, -CONHR₆, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OR₇, or a halogen atom,
   R₄ represents CₘH₂ₘ₊₁,
   R₅s are identical or different and each represent a hydrogen atom or a methyl group,
   R₆ represents a hydrogen atom or a methyl group,
   R₇ represents a C1-C3 alkyl group,
   m represents an integer of 1 to 14, and
   n represents an integer of 1 to 14,
   with the proviso that when R₄ is a linear alkyl group, the sum of m and n is an integer of 12 to 15, and when R₄ is a branched alkyl group, the sum of m_{b} and n is an integer of 12 to 15, wherein m_{b} is the number of carbon atoms of the longest linear chain of the branched alkyl group.
[3] A compound represented by the following formula (I) or a salt thereof: 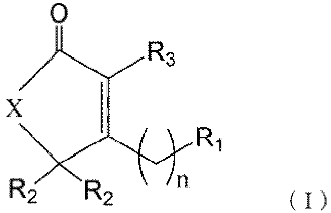 wherein
   X represents -CH₂-, -CH(CH₃)-, -CH₂-CH₂-, or -CH=CH-,
   R₁ represents -CH₂OR₄, -CH₂OCON(CH₃)₂, -CONHR₆, or a cyano group,
   R₂s are identical or different and each represent a hydrogen atom or a methyl group, or two R₂s may form a cyclopropyl group together with the adjacent carbon atom, and
   R₃ represents a hydrogen atom, a methyl group, -CH₂OCH₃, or an iodine atom,
   with the proviso that when R₂s are identical or different and each represent a hydrogen atom or a methyl group, R₃ represents a hydrogen atom, a methyl group, -CH₂OCH₃, or an iodine atom, and when two R₂s form a cyclopropyl group together with the adjacent carbon atom, R₃ represents a methyl group, -CH₂OCH₃, or an iodine atom,
   R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12, and
   R₆ represents a hydrogen atom or a methyl group.
[4] The compound or a salt thereof according to any one of [1] to [3], wherein X represents -CH₂-CH₂-, -CH₂-CH₂-, or -CH=CH- in formula (I).
[5] The compound or a salt thereof according to any one of [1] to [4],
   wherein in formula (I),
   R₂ represents -CH₂OR₄, -CONH₂, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OCH₃, or an iodine atom, with the proviso that when R₃ is a hydrogen atom, R₂s are a hydrogen atom, when R₃ is a methyl group, -CH₂OCH₃, or an iodine atom, R₂s are identical or different and each represent a hydrogen atom or a methyl group, or two R₂s may form a cyclopropyl group together with the adjacent carbon atom, and
   R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12.
[6] The compound or a salt thereof according to any one of [1] to [5], wherein in formula (I),
   X represents -CH₂- or -CH₂-CH₂-,
   R₁ represents -CH₂OR₄ or -CONH₂,
   R₂s represent a methyl group,
   R₃ represents a methyl group or -CH₂OCH₃, and
   R₄ represents a methyl group, an ethyl group, or an isopropyl group, with the proviso that when R₄ is a methyl group, n represents an integer of 11 to 14, and when R₄ is an ethyl group or an isopropyl group, n represents an integer of 11 to 13.
[7] The compound or a salt thereof according to any one of [1] to [6], wherein X represents -CH₂-CH₂- in formula (I) .
[8] The compound or a salt thereof according to any one of [1] to [7], which is any one of the following compounds (1) to (10):
   (1) 3-(12-methoxydodecyl)-2,4,4-trimethylcyclohex-2-en-1-one,
   (2) 3-(12-ethoxydodecyl)-2,4,4-trimethylcyclohex-2-en-1-one,
   (3) 3-(12-isopropoxydodecyl)-2,4,4-trimethylcyclohex-2-en-1-one,
   (4) 3-(13-methoxytridecyl)-2,4,4-trimethylcyclohex-2-en-1-one,
   (5) 3-(13-ethoxytridecyl)-2,4,4-trimethylcyclohex-2-en-1-one,
   (6) 3-(13-isopropoxytridecyl)-2,4,4-trimethylcyclohex-2-en-1-one,
   (7) 3-(14-methoxytetradecyl)-2,4,4-trimethylcyclohex-2-en-1-one,
   (8) 3-(14-ethoxytetradecyl)-2,4,4-trimethylcyclohex-2-en-1-one,
   (9) 3-(14-isopropoxytetradecyl)-2,4,4-trimethylcyclohex-2-en-1-one, and
   (10) 3-(15-methoxypentadecyl)-2,4,4-trimethylcyclohex-2-en-1-one.
[9] The compound or a salt thereof according to any one of [1] to [8], wherein the compound is 3-(15-methoxypentadecyl)-2,4,4-trimethylcyclohex-2-en-1-one.
[10] A nerve growth promoter comprising the compound or a salt thereof of any one of [1] to [9] as an active ingredient.
[11] A therapeutic agent for a neurodegenerative disease, comprising the compound or a salt thereof of any one of [1] to [9] as an active ingredient.
[12] A therapeutic agent for amyotrophic lateral sclerosis, comprising the compound or a salt thereof of any one of [1] to [9] as an active ingredient.
[13] A therapeutic agent for pain, comprising the compound or a salt thereof of any one of [1] to [9] as an active ingredient.
[14] A therapeutic agent for a lower urinary tract dysfunction, comprising the compound or a salt thereof of any one of [1] to [9] as an active ingredient.
[15] A pharmaceutical composition comprising
   the compound or a salt thereof of any one of [1] to [9], and
   a pharmaceutical carrier.

### Advantageous Effects of Invention

The present disclosure provides a novel compound represented by formula (I) or a salt thereof, which is useful as a compound with a nerve growth stimulation effect. The present disclosure also provides a novel compound represented by formula (I) or a salt thereof, which is useful as a compound with excellent pharmacokinetics and/or action of improving voiding function.

### Brief Description of Drawings

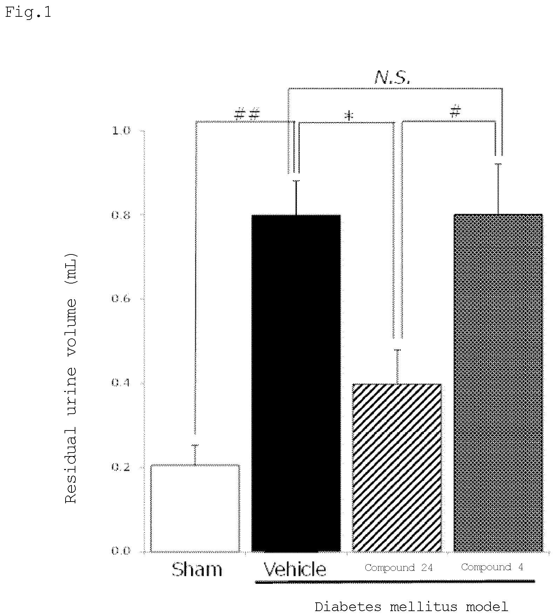

Fig. 1: A graph of residual urine volume of each group. The values represent mean ± S.E. The number of each group is 10, 10, 12, and 12 from left to right.
*: p<0.05, there is a significant difference compared with the vehicle in Dunnett's test
NS: there is no significant difference compared with the vehicle in Dunnett's test,
#: p<0.05, there is a significant difference compared with compound 24 in Student's t-test
##: p<0.01, there is a significant difference compared with the vehicle in Student's t-test

### Description of Embodiments

The compound represented by formula (I) according to the present disclosure is a novel compound that has not been disclosed in any prior literature.

In the compound represented by formula (I) of the present disclosure, X represents -CH₂ -, -CH(CH₃)-, -CH₂-CH₂-, or -CH=CH-, and preferably -CH₂-, -CH(CH₃)-, or -CH₂-CH₂-.

In an embodiment of the present disclosure, X represents -CH₂ -, -CH₂-CH₂ -, or -CH=CH-, preferably -CH₂ - or -CH₂-CH₂-, and more preferably -CH₂-CH₂- in the compound represented by formula (I) of the present disclosure.

In the compound represented by formula (I) of the present disclosure, R₂ represents -CH₂OR₄, -CH₂OCON(R₅)₂, -CONHR₆, or a cyano group. R₂ preferably represents -CH₂OR₄, -CH₂OCON(CH₃)₂, -CONHR₆, or a cyano group, more preferably -CH₂OR₄, -CONH₂, or a cyano group, more preferably -CH₂OCH₃, -CH₂OC₂H₅, -CH₂OCH(CH₃)₂, -CH₂OCH₂CH₂CH₃, -CONH₂, or a cyano group, more preferably -CH₂OCH₃, -CH₂OC₂H₅, -CH₂OCH(CH₃)₂, or -CH₂OCH₂CH₂CH₃, and most preferably -CH₂OCH₃.

In the compound represented by formula (I) of the present disclosure, R₂s are identical or different and each represent a hydrogen atom or a methyl group, or two R₂s may form a cyclopropyl group together with the adjacent carbon atom. R₂s are preferably identical or different and each represent a hydrogen atom or a methyl group, more preferably a methyl group.

In the compound represented by formula (I) of the present disclosure, R₃ represents a hydrogen atom, a methyl group, -CH₂OR₈, or a halogen atom.

The halogen atom represented by R₃ is, for example, a fluorine atom, a chlorine atom, a bromine atom, or an iodine atom, and preferably an iodine atom.

R₃ is preferably a hydrogen atom, a methyl group, -CH₂OCH₃, or a halogen atom, more preferably a hydrogen atom, a methyl group, -CH₂OCH₃, or an iodine atom, more preferably a methyl group, -CH₂OCH₃, or an iodine atom, more preferably a methyl group, or -CH₂OCH₃, and most preferably a methyl group.

In an embodiment of the present disclosure, when R₂s are identical or different and each represent a hydrogen atom or a methyl group, R₃ is a hydrogen atom, a methyl group, -CH₂OCH₃, or an iodine atom. In another embodiment of the present disclosure, when two R₂s form a cyclopropyl group together with the adjacent carbon atom, R₃ is a methyl group, -CH₂OCH₃, or an iodine atom; and when R₂s are identical or different and each represent a hydrogen atom or a methyl group, R₃ is preferably a hydrogen atom, a methyl group, -CH₂OCH₃, or an iodine atom. In another embodiment of the present disclosure, when two R₂s form a cyclopropyl group together with the adjacent carbon atom, R₃ is a methyl group.

In the compound represented by formula (I) of the present disclosure, R₄ is CₘH₂ₘ₊₁. R₄ is preferably a methyl group, an ethyl group, an n-propyl group, or an isopropyl group, more preferably a methyl group, an ethyl group, or an isopropyl group, and more preferably a methyl group.

In the compound represented by formula (I) of the present disclosure, m represents an integer of 1 to 14, and preferably an integer of 1 to 3.

In the compound represented by formula (I) of the present disclosure, n represents an integer of 1 to 15, preferably an integer of 1 to 14, and more preferably an integer of 11 to 14. Preferably, when R₄ is a methyl group, n is an integer of 11 to 14. Preferably, when R₄ is an ethyl group or an isopropyl group, n is an integer of 11 to 13. Preferably, when R₄ is an n-propyl group, n is 11 or 12.

In the compound represented by formula (I) of the present disclosure, when R₄ is a linear alkyl group, the sum of m and n is an integer of 12 to 17; and when R₄ is a branched alkyl group, the sum of m_{b} and n is an integer of 12 to 17, wherein m_{b} is the number of carbon atoms of the longest linear chain of the branched alkyl group. More preferably, when R₄ is a linear alkyl group, the sum of m and n is an integer of 12 to 15; and when R₄ is a branched alkyl group, the sum of m_{b} and n is an integer of 12 to 15, wherein m_{b} is the number of carbon atoms of the longest linear chain of the branched alkyl group.

In the compound represented by formula (I) of the present disclosure, when R₄ is a branched alkyl group, m_{b} represents an integer indicating the number of carbon atoms of the longest linear chain of the branched alkyl group. For example, when m is 3, and R₄ is an isopropyl group, m_{b} is 2. When m is 4, and R₄ is a sec-butyl group or an isobutyl group, m_{b} is 3. When m is 4, and R₄ is a tert-butyl group, m_{b} is 2.

In the compound represented by formula (I) of the present disclosure, R₅s are identical or different and each represent a hydrogen atom or a methyl group. Each R₅ is preferably a methyl group.

In the compound represented by formula (I) of the present disclosure, R₆ represents a hydrogen atom or a methyl group. R₆ is preferably a hydrogen atom.

In the compound represented by formula (I) of the present disclosure, R₇ represents a C1-C3 alkyl group, preferably a methyl group, an ethyl group, or an isopropyl group, and more preferably a methyl group.

Preferable compounds among those represented by formula (I) satisfy the following:
X represents -CH₂-, -CH₂-CH₂-, or -CH=CH-,
R₁ represents -CH₂OR₄, -CH₂OCON (CH₃)₂, -CONHR₆, or a cyano group,
R₂s are identical or different and each represent a hydrogen atom or a methyl group, or two R₂s may form a cyclopropyl group together with the adjacent carbon atom, and
R₃ represents a hydrogen atom, a methyl group, -CH₂OCH₃, or an iodine atom,
with the proviso that
when R₂s are identical or different and each represent a hydrogen atom or a methyl group, R₃ represents a hydrogen atom, a methyl group, -CH₂OCH₃, or an iodine atom,
when two R₂s form a cyclopropyl group together with the adjacent carbon atom, R₃ represents a methyl group, -CH₂OCH₃, or an iodine atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13,
when R₄ is an n-propyl group, n is 11 or 12, and R₆ represents a hydrogen atom or a methyl group.

More preferable compounds among those represented by formula (I) satisfy the following:
X represents -CH₂-, -CH₂-CH₂-, or -CH=CH-,
R₂ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, R₂ is a hydrogen atom,
when R₃ is a methyl group, -CH₂OCH₃, or an iodine atom, R₂s are identical or different and each represent a hydrogen atom or a methyl group, or two R₂s may form a cyclopropyl group together with the adjacent carbon atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

More preferable compounds among those represented by formula (I) satisfy the following:
X represents -CH₂- or -CH₂-CH₂-,
R₂ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, each R₂ is a hydrogen atom,
when R₃ is a methyl group, -CH₂OCH₃, or an iodine atom, R₂s are identical or different and each represent a hydrogen atom or a methyl group, or two R₂s may form a cyclopropyl group together with the adjacent carbon atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

More preferable compounds among those represented by formula (I) satisfy the following:
X represents -CH₂-CH₂-,
R₂ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, each R₂ is a hydrogen atom, and when R₃ is a methyl group, -CH₂OCH₃, or an iodine atom, R₂s are identical or different and each represent a hydrogen atom or a methyl group, or two R₂s may form a cyclopropyl group together with the adjacent carbon atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

More preferable compounds among those represented by formula (I) satisfy the following:
X represents -CH₂-CH₂-,
R₂ represents -CH₂OR₄ or -CONH₂,
each R₂ represents a methyl group,
R₃ represents a methyl group,
R₄ represents a methyl group, an ethyl group, or an isopropyl group,
with the proviso that
when R₄ is a methyl group, n represents an integer of 11 to 14, and
when R₄ is an ethyl group or an isopropyl group, n represents an integer of 11 to 13.

More preferable compounds among those represented by formula (I) satisfy the following:
X represents -CH₂-CH₂-,
R₁ represents -CH₂OR₄,
each R₂ represents a methyl group,
R₃ represents a methyl group,
R₄ represents a methyl group, an ethyl group, or an isopropyl group,
with the proviso that
when R₄ is a methyl group, n represents an integer of 11 to 14, and
when R₄ is an ethyl group or an isopropyl group, n represents an integer of 11 to 13.

In an embodiment of the present disclosure, preferable compounds represented by formula (I) satisfy the following:
X represents -CH₂-CH₂-,
R₁ represents -CH₂OR₄, -CH₂OCON(CH₃)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OR₇, or an iodine atom,
with the proviso that
when R₂s are identical or different and each represent a hydrogen atom or a methyl group, R₃ represents a hydrogen atom, a methyl group, -CH₂OR₇, or a halogen atom, and when two R₂s form a cyclopropyl group together with the adjacent carbon atom, R₃ represents a methyl group, -CH₂OR₇, or a halogen atom,
R₄ represents CₘH₂ₘ₊₁,
R₆ represents a hydrogen atom or a methyl group,
R₇ represents a C1-C3 alkyl group,
m represents an integer of 1 to 14,
n represents an integer of 1 to 14,
with the proviso that
when m is an integer of 1 to 14, and R₄ is a linear alkyl group, the sum of m and n is an integer of 12 to 15, and
when m is an integer of 1 to 14, and R₄ is a branched alkyl group, and the sum of m_{b} and n is an integer of 12 to 15, wherein m_{b} is the number of carbon atoms of the longest linear chain of the branched alkyl group.

In an embodiment of the present disclosure, preferable compounds represented by formula (I) satisfy the following:
X represents -CH₂-CH₂-,
R₁ represents -CH₂OR₄, -CH₂OCON(CH₃)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₂s are identical or different and each represent a hydrogen atom or a methyl group, R₃ represents a hydrogen atom, a methyl group, -CH₂OCH₃, or an iodine atom, and
when two R₂s form a cyclopropyl group together with the adjacent carbon atom, R₃ represents a methyl group, -CH₂OCH₃, or an iodine atom,
R₄ represents CₘH₂ₘ₊₁,
R₆ represents a hydrogen atom or a methyl group,
m represents an integer of 1 to 14,
n represents an integer of 1 to 14,
with the proviso that
when m is an integer of 1 to 14, and R₄ is a linear alkyl group, the sum of m and n is an integer of 12 to 15, and
when m is an integer of 1 to 14, and R₄ is a branched alkyl group, the sum of m_{b} and n is an integer of 12 to 15, wherein m_{b} is the number of carbon atoms of the longest linear chain of the branched alkyl group.

In an embodiment of the present disclosure, preferable compounds represented by formula (I) satisfy the following:
X represents -CH₂-CH₂-,
R₁ represents -CH₂OR₄, -CH₂OCON(CH₃)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₂s are identical or different and each represent a hydrogen atom or a methyl group, R₃ represents a hydrogen atom, a methyl group, -CH₂OCH₃, or an iodine atom, and
when two R₂s form a cyclopropyl group together with the adjacent carbon atom, R₃ represents a methyl group, -CH₂OCH₃, or an iodine atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13,
when R₄ is an n-propyl group, n is 11 or 12, and R₆ represents a hydrogen atom or a methyl group.

In an embodiment of the present disclosure, more preferable compounds represented by formula (I) satisfy the following:
X represents -CH₂-CH₂-,
R₁ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, each R₂ is a hydrogen atom,
when R₃ is a methyl group, -CH₂OCH₃, or an iodine atom, R₂s are identical or different and each represent a hydrogen atom or a methyl group, or two R₂s may form a cyclopropyl group together
with the adjacent carbon atom, and
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

In an embodiment of the present disclosure, preferable compounds represented by formula (I) satisfy the following:
X represents -CH₂-, -CH(CH₃)-, -CH₂-CH₂-, or -CH=CH-,
R₁ represents -CH₂OR₄, -CH₂OCON(CH₃)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₂s are identical or different and each represent a hydrogen atom or a methyl group, R₃ represents a hydrogen atom, a methyl group, -CH₂OCH₃, or an iodine atom, and
when two R₂s form a cyclopropyl group together with the adjacent carbon atom, R₃ represents a methyl group, -CH₂OCH₃, or an iodine atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13,
when R₄ is an n-propyl group, n is 11 or 12, and R₆ represents a hydrogen atom or a methyl group.

In an embodiment of the present disclosure, preferable compounds represented by formula (I) satisfy the following:
X represents -CH₂-, -CH(CH₃)-, -CH₂-CH₂-, or -CH=CH-,
R₁ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, each R₂ is a hydrogen atom,
when R₃ is a methyl group, -CH₂OCH₃, or an iodine atom, R₂s are identical or different and each represent a hydrogen atom or a methyl group, or two R₂s may form a cyclopropyl group together with the adjacent carbon atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

In an embodiment of the present disclosure, preferable compounds represented by formula (I) satisfy the following:
X represents -CH₂-, -CH(CH₃)-, or -CH₂-CH₂-,
R₁ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom,
with the proviso that
when R₃ is a hydrogen atom, each R₂ is a hydrogen atom,
when R₃ is a methyl group, -CH₂OCH₃, or an iodine atom, R₂s are identical or different and each represent a hydrogen atom or a methyl group, or two R₂s may form a cyclopropyl group together with the adjacent carbon atom,
R₄ represents a methyl group, an ethyl group, an n-propyl group, or an isopropyl group,
with the proviso that
when R₄ is a methyl group, n represents an integer of 11 to 14, when R₄ is an ethyl group or an isopropyl group, n represents an integer of 11 to 13, and
when R₄ is an n-propyl group, n is 11 or 12.

Examples of specific compounds of the present disclosure include, but are not limited to, the compounds produced in the Examples below.

Examples of preferable compounds of the present disclosure are the following:
(1) 3-(12-methoxydodecyl)-2,4,4-trimethylcyclohex-2-en-1-one,
(2) 3-(12-ethoxydodecyl)-2,4,4-trimethylcyclohex-2-en-1-one,
(3) 3-(12-isopropoxydodecyl)-2,4,4-trimethylcyclohex-2-en-1-one,
(4) 3-(13-methoxytridecyl)-2,4,4-trimethylcyclohex-2-en-1-one,
(5) 3-(13-ethoxytridecyl)-2,4,4-trimethylcyclohex-2-en-1-one,
(6) 3-(13-isopropoxytridecyl)-2,4,4-trimethylcyclohex-2-en-1-one,
(7) 3-(14-methoxytetradecyl)-2,4,4-trimethylcyclohex-2-en-1-one,
(8) 3-(14-ethoxytetradecyl)-2,4,4-trimethylcyclohex-2-en-1-one,
(9) 3-(14-isopropoxytetradecyl)-2,4,4-trimethylcyclohex-2-en-1-one,
(10) 3-(15-methoxypentadecyl)-2,4,4-trimethylcyclohex-2-en-1-one.

Among the compounds represented by formula (I), the most preferable compound is 3-(15-methoxypentadecyl)-2,4,4-trimethylcyclohex-2-en-1-one.

The following describes a method for producing the compound according to the present disclosure.

Compound (I) of the present disclosure can be produced, for example, according to the following production method or the method described in the Examples. However, the production method for compound (I) is not limited to these reaction examples.

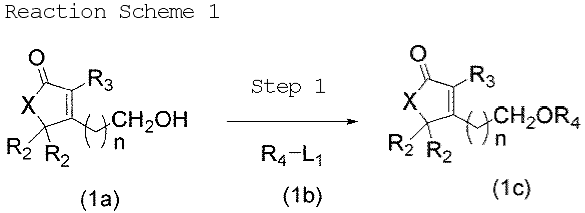

In reaction scheme 1, X, R₂, R₃, R₄, and n are as defined above, and L₁ represents a leaving group.

In the method shown by reaction scheme 1, the hydroxyl group of the compound having a hydroxyl group represented by formula (1a) is reacted with the compound having a leaving functional group represented by formula (1b) to induce an ether derivative (1c).

Specifically, L₁ in compound (1b) can be any group as long as it is a leaving functional group. Examples include a chlorine atom, a bromine atom, an iodine atom, a methanesulfonyloxy group, a benzenesulfonyloxy group, and a p-toluenesulfonyloxy group.

In an appropriate solvent, the reaction is allowed to proceed with 0.5 to 50 mol, preferably 0.8 to 20 mol of the compound represented by formula (1b), per mol of the hydroxy compound represented by formula (1a), in the presence of 0.5 to 10 mol, preferably 0.8 to 5 mol, of a base, per mol of the compound represented by formula (1a), at -50°C to 100°C, preferably -20°C to 80°C, to obtain the ether compound represented by formula (1c).

The appropriate solvent is not particularly limited as long as the solvent does not affect the reaction. Examples include dichloromethane, chloroform, ethyl acetate, acetonitrile, tetrahydrofuran, dioxane, diethyl ether, diisopropyl ether, benzene, toluene, N,N-dimethylformamide, N,N-dimethylacetamide, N-methylpyrrolidone, and dimethylsulfoxide. These can be used singly or in a combination.

Examples of bases for use include inorganic bases, such as lithium hydroxide, sodium hydroxide, potassium hydroxide, calcium hydroxide, barium hydroxide, lithium carbonate, sodium carbonate, potassium carbonate, sodium hydride, and potassium hydride, organic bases, such as pyridine, lutidine, collidine, 4-(N,N-dimethylamino)pyridine, triethylamine, diisopropylethylamine, 1,5-diazabicyclo[4.3.0]non-5-ene, 1,8-diazabicyclo[5.4.0]undec-7-ene, potassium tert-butoxide, and sodium tert-butoxide, silver oxide, and silver carbonate.

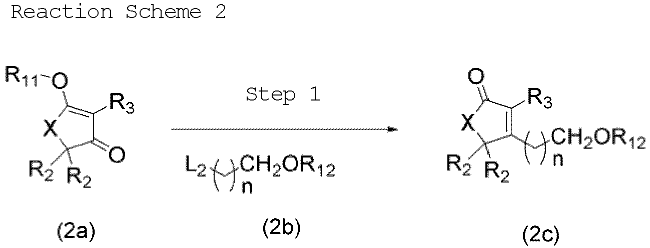

In reaction scheme 2, X, R₂, R₃, and n are as defined above, L₂ represents a halogen atom, R₁₁ represents a C1-C7 alkyl group, and R₁₂ represents R₄ defined above or a protecting group for a hydroxyl group.

In the method shown by reaction scheme 2, the compound having an enol ether represented by formula (2a) is reacted with the compound having a halogen atom represented by formula (2b) in the presence of a metal to induce an enone derivative (2c).

Specifically, L₂ in compound (2b) can be any halogen atom. Examples include a chlorine atom, a bromine atom, and an iodine atom.

R₁₁ is a C1-C7 alkyl group. Examples include a methyl group, an ethyl group, a propyl group, an isopropyl group, a butyl group, an isobutyl group, a sec-butyl group, and a tert-butyl group.

R₁₂ represents R₄ defined above or a protecting group for a hydroxyl group. Examples of protecting groups for a hydroxyl group include a methoxymethyl group, a tetrahydropyranyl group, a benzyl group, a tert-butyldimethylsilyl group, and a tert-butyldiphenylsilyl group.

In an appropriate solvent, the reaction is allowed to proceed with 0.5 to 50 mol, preferably 0.8 to 10 mol of the compound represented by formula (2b), per mol of the enol ether compound represented by formula (2a), in the presence of 0.5 to 100 mol, preferably 0.8 to 30 mol, of a metal per mol of the compound represented by formula (2a), at 0°C to 150°C, preferably 20°C to 120°C, to obtain an enone compound represented by formula (2c) .

The appropriate solvent is not particularly limited as long as the solvent does not affect the reaction. Examples include benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane. These can be used singly or in a combination.

The metal is, for example, lithium, sodium, strontium, magnesium, or zinc, and preferably lithium or magnesium.

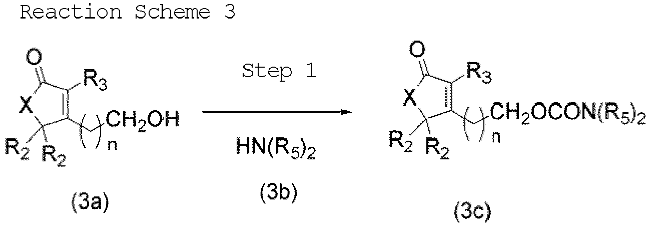

In reaction scheme 3, X, R₂, R₃, R₅, and n are as defined above. In the method shown by reaction scheme 3, the compound having a hydroxyl group represented by formula (3a) or an active species thereof is condensed with the amine compound represented by formula (3b) or a salt thereof according to an ordinary method to induce a carbamate derivative (3c).

Specifically, in condensation, it is preferable to use an active species having a leaving group prepared by allowing, for example, triphosgene, 1,1'-carbonyldiimidazole (CDI), phenyl chloroformate, 4-nitrophenyl chloroformate, or ethyl chloroformate to act on the compound having a hydroxyl group represented by formula (3a) in the presence or absence of an organic base, such as triethylamine, diisopropylethylamine, or pyridine, in a solvent inert to reaction, such as dichloromethane, tetrahydrofuran, acetonitrile, ethyl acetate, or N,N-dimethylacetamide at -20°C to 150°C, preferably 0°C to 100°C.

The active species of formula (3a) can be, for example, an active species having a leaving group. Such an active species having a leaving group can be isolated for use in reaction, or prepared in a reaction system and used in reaction without isolation. Examples of leaving groups include a chlorine atom, an imidazolyl group, a phenoxy group, a 4-nitrophenoxy group, and an ethoxy group.

Examples of salts of the amine compound represented by formula (3b) include acid addition salts formed with an inorganic acid, such as hydrochloric acid, hydrobromic acid, or sulfuric acid, or with an organic acid, such as carbonic acid or methanesulfonic acid.

When 0.5 to 100 mol, preferably 0.8 to 50 mol of the amine compound represented by formula (3b) or a salt thereof is used per mol of the compound having a hydroxyl group represented by formula (3a) or an active species thereof, with the use of the condensation agent, the amount of the condensation agent for use is 0.5 to 20 mol, preferably 0.8 to 3 mol, per mol of the compound having a hydroxyl group represented by formula (3a) or a salt thereof.

The reaction can be performed typically in a solvent inert to reaction, for example, a halogenated hydrocarbon such as dichloromethane or chloroform, an aromatic hydrocarbon, such as toluene, an ether, such as tetrahydrofuran, an ester, such as ethyl acetate, an alcohol, such as methanol or ethanol, water, acetonitrile, N,N-dimethylformamide, N,N-dimethylacetamide, dimethylsulfoxide, or pyridine at -20°C to 150°C, preferably 0°C to 100°C, although the reaction varies according to the active species or condensation agent for use.

The reaction may proceed smoothly when the reaction is performed in the presence of 0.5 to 20 mol, preferably 0.8 to 5 mol of a base, such as triethylamine, diisopropylethylamine, N-methylmorpholine, N,N-diethylaniline, 4-(N,N-dimethylamino)pyridine, or pyridine, per mol of the compound having a hydroxyl group represented by formula (3a) or an active species thereof.

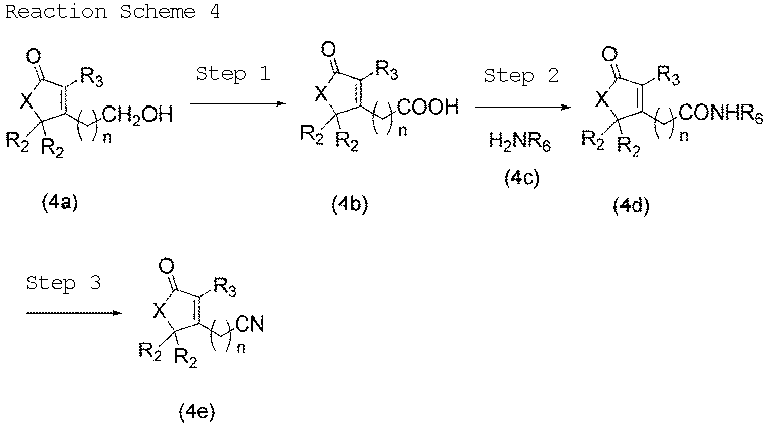

In reaction scheme 4, X, R₂, R₃, R₆, and n are as defined above. In the method shown by reaction scheme 4, of the compound having a hydroxyl group represented by formula (4a), the hydroxyl group is oxidized into carboxylic acid to form the carboxylic acid derivative represented by formula (4b) in step 1. Then, the carboxylic acid derivative (4b) is condensed with the amine compound represented by formula (4c) to induce the amide derivative (4d) in step 2. In step 3, the amide derivative (4d) is further dehydrated to induce the nitrile derivative (4e).

### Step 1

Specifically, the method for oxidizing the hydroxyl group of the compound represented by formula (4a) to carboxylic acid may be reacting 0.5 to 30 mol, preferably 0.8 to 20 mol of a commonly used appropriate oxidant, per mol of the compound having a hydroxyl group represented by formula (4a) in an appropriate solvent at a temperature of about -80 to 150°C, preferably about -80 to 120°C, although an aldehyde is isolated for use or oxidation into carboxylic acid is directly performed in some cases.

Examples of oxidants include manganese reagents, such as activated manganese dioxide and potassium permanganate, chromium reagents, such as pyridinium chlorochromate (PCC), pyridinium dichromate (PDC), and chromium trioxide, sodium hypochlorite- or calcium hypochlorite-2,2,6,6-tetramethyl-1-piperidinyloxy free radical (TEMPO), dimethylsulfoxide (DMSO) oxidation (DMSO-acetic anhydride, DMSO-trifluoroacetic anhydride, DMSO-oxalyl chloride, DMSO-dicyclohexylcarbodiimide (DCC), DMSO-1-ethyl-3-(3-dimethylaminopropyl)carbodiimide, and a DMSO-sulfur trioxide pyridine complex).

The appropriate solvent is not particularly limited as long as the solvent does not affect the reaction. Examples include water, dichloromethane, chloroform, carbon tetrachloride, 1,2-dichloroethane, diethyl ether, tetrahydrofuran, dioxane, benzene, toluene, xylene, chlorobenzene, dimethylsulfoxide, tert-butyl alcohol, pyridine, and triethylamine. These can be used singly or in a combination.

### Step 2

This step is a method for obtaining the amide compound represented by formula (4d) by amidation between the carboxyl group of the compound represented by formula (4b) with the amine compound (4c), which can be a commercially available product or a compound produced according to a known method.

Amidation can be performed according a known method. Examples include a method of performing a reaction in the presence of a condensation agent, and a method of activating a carboxylic acid moiety according to a known method to form a reactive derivative and then performing amidation of the derivative with an amine (see peptide gosei no kiso to jikken for these methods, Nobuo Izumiya et al., Maruzen Publishing Co., Ltd., 1983).

Examples of condensation agents include N,N'-dicyclohexylcarbodiimide (DCC), N,N'-diisopropylcarbodiimide (DIC), 1-ethyl-3-(3-dimethylaminopropyl)carbodiimide hydrochloride (WSC), diphenylphosphoryl azide (DPPA), benzotriazol-1-yl-oxytrisdimethylaminophosphonium hexafluorophosphate (BOP), benzotriazol-1-yl-oxytripyrrolidinophosphonium hexafluorophosphate (PyBOP), 7-azabenzotriazol-1-yl oxytrispyrrolidinophosphonium phosphate (PyAOP), bromotrispyrrolidinophosphonium hexafluorophosphate (BroP), chlorotris(pyrrolidin-1-yl)phosphonium hexafluorophosphate (PyCroP), 3-(diethoxyphosphoryloxy)-1,2,3-benzotriazine-4(3H)-one (DEPBT), O-(benzotriazol-1-yl)-N,N,N',N'-tetramethyluronium hexafluorophosphate (HATU), and 4-(5,6-dimethoxy-1,3,5-triazin-2-yl)-4-methylmorpholine hydrochloride (DMTMM). Examples of additives for this include 1-hydroxybenzotriazole (HOBt), 1-hydroxy-7-azabenzotriazol (HOAt), and N-hydroxy succinimide (HOSu). The amount of these for use is typically about 1 to 100 mol, preferably about 1 to 10 mol, per mol of the compound represented by formula (4b).

A base can also optionally be added. Examples of bases include organic amines (e.g., trimethylamine, triethylamine, diisopropylethylamine, N-methylmorpholine, 1,8-diazabicyclo[5,4,0]undec-7-ene, pyridine, and N,N-dimethylaniline), alkali metal salts (e.g., sodium hydrogen carbonate, potassium hydrogen carbonate, sodium carbonate, potassium carbonate, cesium carbonate, sodium phosphate, potassium phosphate, sodium hydroxide, and potassium hydroxide), metal hydrides (e.g., potassium hydride and sodium hydride), and alkali metal alkoxides (e.g., sodium methoxide, sodium ethoxide, sodium tert-butoxide, and potassium tert-butoxide). The amount of the base for use is typically about 1 to 100 mol, preferably about 1 to 10 mol, per mol of the compound represented by formula (4b) .

The solvent for use in the reaction can be any solvent that does not adversely affect the reaction. Examples of solvents for use include alcohols (e.g., methanol), hydrocarbons (e.g., benzene, toluene, and xylene), halogenated hydrocarbons (e.g., dichloromethane, chloroform, and 1,2-dichloroethane), nitriles (e.g., acetonitrile), ethers (e.g., dimethoxyethane and tetrahydrofuran), aprotic polar solvents (e.g., N,N-dimethylformamide, dimethylsulfoxide, and hexamethylphosphoramide), and mixtures thereof. The reaction time is 0.1 to 100 hours, and preferably 0.5 to 24 hours. The reaction temperature is in a range of 0°C to a temperature at which the solvent boils, preferably in a range of 0°C to 100°C.

### Step 3

This step is a method for obtaining the nitrile compound represented by formula (4e) by dehydration reaction of the amide compound represented by formula (4d).

Specifically, an example of the method for performing dehydration is allowing, for example, trifluoroacetic anhydride, acetic anhydride, triphosgene, a Burgess reagent, p-toluenesulfonyl chloride, benzenesulfonyl chloride, thionyl chloride, or phosphorus oxychloride to act on the compound with an amide bond represented by formula (4d) in the presence or absence of an organic base, such as triethylamine, diisopropylethylamine, or pyridine, in a solvent inert to reaction, such as dichloromethane, tetrahydrofuran, acetonitrile, ethyl acetate, or N,N-dimethylacetamide, at a temperature of -20°C to 150°C, preferably 0°C to 100°C.

When the dehydrating agent is used, the amount of the dehydrating agent for use is 0.5 to 20 mol, preferably 0.8 to 5 mol, per mol of the compound with an amide bond represented by formula (4d).

The reaction can be performed typically in a solvent inert to reaction, for example, a halogenated hydrocarbon, such as dichloromethane or chloroform, an aromatic hydrocarbon, such as toluene, an ether, such as tetrahydrofuran, an ester, such as ethyl acetate, an alcohol, such as methanol or ethanol, water, acetonitrile, N,N-dimethylformamide, N,N-dimethylacetamide, dimethylsulfoxide, or pyridine, at -20°C to 150°C, preferably 0°C to 100°C, although the reaction varies according to the base or dehydrating agent for use.

The reaction may proceed smoothly when the reaction is performed in the presence of 0.5 to 20 mol, preferably 0.8 to 5 mol of a base, such as triethylamine, diisopropylethylamine, N-methylmorpholine, N,N-diethylaniline, 4-(N,N-dimethylamino)pyridine, or pyridine, per mol of the compound with an amide bond represented by formula (4d).

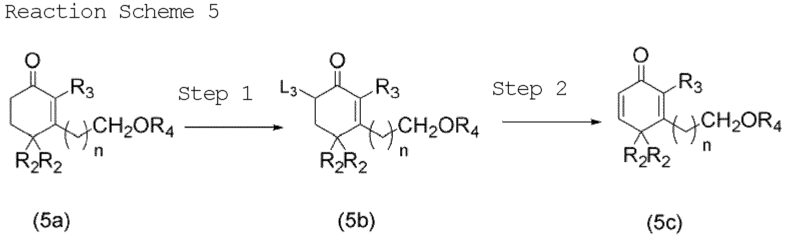

In reaction scheme 5, R₂, R₃, R₄, and n are as defined above, and L₃ represents a leaving functional group, such as a phenylselenyl group.

In the method shown by reaction scheme 5, a functional group is introduced into the α-position of the ketone group of the compound with a ketone group represented by formula (5a) to form the compound represented by formula (5b) in step 1. Then, the compound (5b) is oxidized to induce the dienone compound (5c) in step 2.

### Step 1

Specifically, the method for introducing a functional group into the α-position of the ketone group of the compound represented by formula (5a) may be reacting 0.5 to 30 mol, preferably 0.8 to 10 mol, of a commonly used appropriate base, per mol of the compound with a ketone group represented by formula (5a) in an appropriate solvent at a temperature of about -100 to 120°C, preferably about -80 to 100°C, although an enol ether is isolated for use or prepared in a reaction system and used in reaction without isolation in some cases.

Examples of bases include organic amines (e.g., trimethylamine, triethylamine, diisopropylethylamine, N-methylmorpholine, 1,8-diazabicyclo[5,4,0]undec-7-ene, pyridine, and N,N-dimethylaniline), alkali metal salts (e.g., sodium hydrogen carbonate, potassium hydrogen carbonate, sodium carbonate, potassium carbonate, cesium carbonate, sodium phosphate, potassium phosphate, sodium hydroxide, and potassium hydroxide), metal hydrides (e.g., potassium hydride and sodium hydride), alkali metal alkoxides (e.g., sodium methoxide, sodium ethoxide, sodium tert-butoxide, and potassium tert-butoxide), and metal reagents (e.g., methyllithium, n-butyllithium, lithium diisopropylamide, and lithium hexamethyldisilazide).

The appropriate solvent is not particularly limited as long as the solvent does not affect the reaction. Examples include dichloromethane, chloroform, carbon tetrachloride, 1,2-dichloroethane, diethyl ether, tetrahydrofuran, 2-methyltetrahydrofuran, 1,2-dimethoxyethane, dioxane, benzene, toluene, xylene, chlorobenzene, dimethylsulfoxide, pyridine, and triethylamine. These can be used singly or in a combination.

Examples of functional groups to be introduced include a phenylselenyl group, and examples of reagents for use include phenylselenyl chloride and phenylselenyl bromide.

### Step 2

This step is a method for obtaining the dienone compound represented by formula (5c) by subjecting the compound represented by formula (5b) to extraction reaction. This step may be performed by reacting 0.5 to 30 mol, preferably 0.8 to 10 mol, of a commonly used appropriate oxidant, per mol of the compound with a leaving functional group represented by formula (5b) in an appropriate solvent at a temperature of about -50 to 120°C, preferably about -20 to 100°C.

Examples of oxidants include a hydrogen peroxide solution, m-chloroperbenzoic acid, and peracetic acid.

The appropriate solvent is not particularly limited as long as the solvent does not affect the reaction. Examples include water, dichloromethane, chloroform, carbon tetrachloride, 1,2-dichloroethane, tetrahydrofuran, ethyl acetate, benzene, toluene, xylene, chlorobenzene, dimethylsulfoxide, pyridine, and triethylamine. These can be used singly or in a combination.

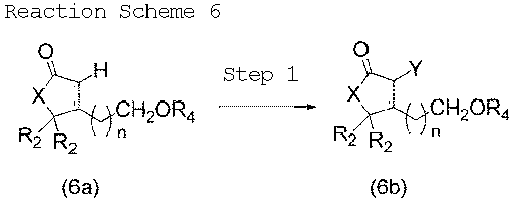

In reaction scheme 6, X, R₂, R₃, R₄, and n are as defined above, and Y represents a halogen atom.

In the method shown by reaction scheme 6, a halogen atom is introduced into the compound with an enone group represented by formula (6a) to induce the compound represented by formula (6b). The method for using a halogenated reagent can be performed, for example, by a method using fluorine, chlorine, bromine, iodine, or the like, or a method using N-chlorosuccinimide, N-bromosuccinimide, or N-iodosuccinimide.

The halogenated reagent is used in an amount of 0.5 to 30 mol, preferably about 0.8 to 10 mol, per mol of the compound with an enone group represented by formula (6a).

The solvent can be any solvent that does not adversely affect the reaction. Examples include hydrocarbons (e.g., benzene, toluene, and xylene), halogenated hydrocarbons (e.g., chloroform and 1,2-dichloroethane), nitriles (e.g., acetonitrile), ethers (e.g., dimethoxyethane and tetrahydrofuran), alcohols (e.g., methanol and ethanol), aprotic polar solvents (e.g., N,N-dimethylformamide, dimethylsulfoxide, and hexamethylphosphoramide), pyridine, triethylamine, and water. These can be used singly or in a combination.

The reaction temperature is in a range of -20°C to a temperature at which the solvent boils, and preferably in a range of 0°C to 100°C. The reaction may proceed smoothly when the reaction is performed in the presence of 0.1 to 10 mol, preferably 0.2 to 5 mol of a reagent reaction, such as potassium carbonate, 4-(N,N-dimethylamino)pyridine, or trimethylsilyl azido, per mol of the compound with an enone group represented by formula (6a).

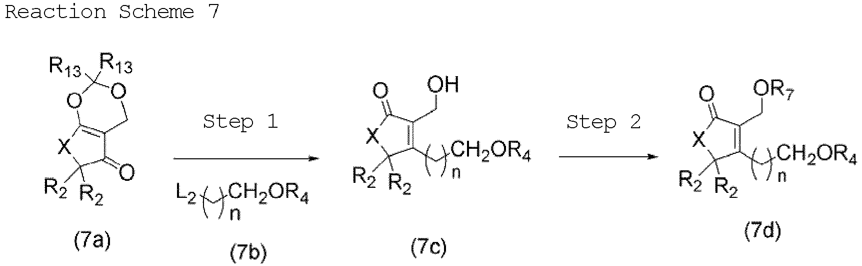

In reaction scheme 7, X, R₂, R₄, R₇, and n are as defined above, L₂ represents a halogen atom, and R₁₃ represents a hydrogen atom or a C1-C4 alkyl group.

In the method shown by reaction scheme 7, the compound with an enol ether represented by formula (7a) is reacted with the compound with a halogen atom represented by formula (7b) in the presence of a metal to induce the enone derivative (7c) in step 1. In step 2, the hydroxyl group of the compound represented by formula (7c) is further etherified to induce the ether derivative (7d).

### Step 1

L₂ of compound (7b) can be any halogen atom. Examples include a chlorine atom, a bromine atom, and an iodine atom.

R₁₃ represents a hydrogen atom or a C1-C4 alkyl group. Examples include a hydrogen atom, a methyl group, an ethyl group, a propyl group, an isopropyl group, and a butyl group.

In an appropriate solvent, the reaction is performed by using 0.5 to 50 mol, preferably 0.8 to 10 mol of the compound represented by formula (7b), per mol of the enol ether compound represented by formula (7a) in the presence of 0.5 to 100 mol, preferably 0.8 to 30 mol of a metal, per mol of the compound represented by formula (7a) at 0°C to 150°C, preferably 20°C to 120°C to obtain the enone compound represented by formula (7c).

The appropriate solvent is not particularly limited as long as the solvent does not affect the reaction. Examples include benzene, toluene, chlorobenzene, xylene, tetrahydrofuran, methyltetrahydrofuran, dioxane, methyl tert-butyl ether, n-hexane, n-heptane, cyclohexane, acetonitrile, hexamethylphosphoric triamide, and sulfolane. These can be used singly or in a combination.

The metal is, for example, lithium, sodium, strontium, magnesium, or zinc, and preferably lithium or magnesium.

### Step 2

Examples of reagents for etherifying the compound having a hydroxyl group represented by formula (7c) include trimethyloxonium tetrafluoroborate and triethyloxonium hexafluoroborate.

In an appropriate solvent, the reaction is performed by using 0.5 to 50 mol, preferably 0.8 to 20 mol, of an etherification reagent, per mol of the compound having a hydroxyl group represented by formula (7c) at -50°C to 120°C, preferably - 20°C to 100°C to obtain the ether compound represented by formula (7d) .

The appropriate solvent is not particularly limited as long as the solvent does not affect the reaction. Examples include dichloromethane, chloroform, ethyl acetate, acetonitrile, tetrahydrofuran, dioxane, diethyl ether, diisopropyl ether, benzene, toluene, N,N-dimethylformamide, N,N-dimethylacetamide, N-methylpyrrolidone, and dimethylsulfoxide. These can be used singly or in a combination.

The reaction may proceed smoothly when the reaction is performed in the presence of 0.5 to 20 mol, preferably 0.8 to 10 mol, of a reagent, such as 1,8-bis(dimethylamino)naphthalene, per mol of the compound having a hydroxyl group represented by formula (7c).

The compound of the present disclosure can be easily isolated and purified according to a typical separation means. Examples of such means include solvent extraction, recrystallization, preparative reverse-phase high-performance liquid chromatography, column chromatography, and preparative thin-layer chromatography.

When the compound of the present disclosure has an isomer such as an optical isomer, a stereoisomer, a rotamer, or a tautomer, the compound of the present disclosure encompasses any isomers and mixtures thereof unless otherwise specified. For example, when the compound of the present disclosure has an optical isomer, the scope of the compound of the present disclosure encompasses the optical isomer separated from the racemate unless otherwise specified. These isomers can be individually obtained as a single compound according to a known synthesis technique or separation technique (e.g., condensation, solvent extraction, column chromatography, or recrystallization).

The compound of the present disclosure or a salt thereof may be amorphous and/or crystalline. The scope of the compound of the present disclosure or a salt thereof includes single crystalline form, mixtures of multiple polymorphic forms, and mixtures of these forms with amorphous forms. Crystals can be produced by performing crystallization using a known crystallization method. The compound of the present disclosure or a salt thereof may be a solvate (e.g., a hydrate) or a non-solvate, and both are included in the compound of the present disclosure or a salt thereof. Compounds labeled with isotopes (e.g., 3H, 14C, 35S, and 125I) are also included in the compound of the present disclosure or a salt thereof.

The salt of the compound of the present disclosure or the salt of a production intermediate thereof means those commonly used in the field of organic chemistry. The salt of the compound of the present disclosure is preferably a pharmaceutically acceptable salt.

The salt of the compound of the present disclosure or the salt of a production intermediate thereof is, for example, a salt such as a base addition salt at a carboxyl group when the carboxyl group is present in the compound or an acid addition salt at an amino group or at a basic heterocyclic group when the amino group or basic heterocyclic group is present in the compound.

Examples of base addition salts include alkali metal salts, such as sodium salts and potassium salts; alkaline-earth metal salts, such as calcium salts and magnesium salts; ammonium salts; and organic amine salts, such as trimethylamine salts, triethylamine salts, dicyclohexylamine salts, ethanolamine salts, diethanolamine salts, triethanolamine salts, procaine salts, and N,N'-dibenzylethylenediamine salts.

Examples of acid addition salts include inorganic acid salts, such as hydrochloride, sulfate, nitrate, phosphate, and perchlorate; organic acid salts, such as acetate, formate, maleate, fumarate, tartrate, citrate, ascorbate, and trifluoroacetate; and sulfonate, such as methanesulfonate, isethionate, benzenesulfonate, and p-toluenesulfonate.

The scope of the present disclosure encompasses any pharmaceutically acceptable modified product (prodrug) that converts into the compound of the present disclosure in vivo.

Due to its excellent nerve growth stimulation effect, such as neurite outgrowth action and/or action of increasing the proportion of neurite-bearing cells, the compound of the present disclosure or a salt thereof is useful as a medical drug for the prevention or treatment of diseases that are ameliorated by the promotion of nerve growth (including neurodegenerative diseases), pain, and/or lower urinary tract dysfunction.

The pathogenesis of pathological conditions varies in diseases that are ameliorated by the promotion of nerve growth (including neurodegenerative diseases), pain, and/or lower urinary tract dysfunction. One of the causes is degeneration or atrophy of neurites or neuronal cell death. Thus, nerve growth promotion can prevent or treat these diseases.

In the present specification, the diseases that are ameliorated by the promotion of nerve growth include neurodegenerative diseases, and traumatic neurological disorders caused by accidents etc. (e.g., cerebral contusion and spinal cord injury), or mental illness (e.g., schizophrenia, depression, and bipolar disorder).

In the present specification, neurodegenerative diseases are, for example, amyotrophic lateral sclerosis (ALS), Parkinson's disease, Alzheimer's dementia, Lewy body dementia, Guillain-Barre syndrome, chronic inflammatory demyelinating polyneuropathy, multiple sclerosis, and neuromyelitis optica; preferably amyotrophic lateral sclerosis, Parkinson's disease, Alzheimer's dementia, or Lewy body dementia; and more preferably amyotrophic lateral sclerosis.

In the present specification, pain is, for example, peripheral neuropathic pain (e.g., diabetic pain, postherpetic neuralgia, fibromyalgia, and chemotherapy-induced peripheral neuropathy), or central neuropathic pain (e.g., post-stroke pain and post-spinal-cord-injury pain); preferably peripheral neuropathic pain (e.g., diabetic pain, postherpetic neuralgia, fibromyalgia, and chemotherapy-induced peripheral neuropathy); and more preferably diabetic pain or chemotherapy-induced peripheral neuropathy.

In the present specification, lower urinary tract dysfunction are, for example, stress urinary incontinence, urge urinary incontinence, mixed urinary incontinence, overactive bladder, underactive bladder, neurogenic bladder, unstable bladder, detrusor sphincter dyssynergia (DSD), detrusor hyperactivity with impaired contractility (DHIC), or urinary frequency caused by cystitis or prostatitis; preferably stress urinary incontinence, urge urinary incontinence, mixed urinary incontinence, overactive bladder, underactive bladder, or neurogenic bladder; and more preferably overactive bladder or underactive bladder.

The compound of the present disclosure or a salt thereof for use as a medical drug may optionally contain a pharmaceutical carrier, and can be in various dosage forms according to the prevention or treatment purposes. The dosage form can be, for example, any of the following: oral drugs, injectable drugs, suppositories, ointments, and patches, and preferably oral drugs. These dosage forms can be produced according to a formulation method that would be known and commonly used by a person skilled in the art.

The pharmaceutical carrier for use can be various organic or inorganic carrier substances commonly used as a formulation material. The pharmaceutical carrier is added, for example, as an excipient, a binder, a disintegrant, a lubricant, or a colorant in a solid formulation, or as a solvent, a solubilization agent, a suspending agent, a tonicity agent, a buffer, or a soothing agent in a liquid formulation. Additionally, formulation additives, such as a preservative, an antioxidant, a colorant, a sweetener, and a stabilizer, may optionally be used.

In the preparation of a solid formulation for oral use, for example, an excipient, a binder, a disintegrant, a lubricant, a colorant, and/or a flavor-smell-masking agent is added to the compound of the present disclosure as necessary, and then tablets, coated tablets, granules, a powdered drug, capsules, or the like can be produced according to an ordinary method.

Examples of excipients include lactose, sucrose, D-mannitol, glucose, starch, calcium carbonate, kaolin, microcrystalline cellulose, and silicic anhydride. Examples of binders include water, ethanol, 1-propanol, 2-propanol, simple syrup, a dextrose solution, an α-starch solution, a gelatin solution, D-mannitol, carboxymethyl cellulose, hydroxypropyl cellulose, hydroxypropyl starch, methylcellulose, ethylcellulose, shellac, calcium phosphate, and polyvinyl pyrrolidone. Examples of disintegrants include dry starch, sodium alginate, powdered agar, sodium hydrogen carbonate, calcium carbonate, lauryl sodium sulfate, stearic acid monoglyceride, and lactose. Examples of lubricants include purified talc, sodium stearate, magnesium stearate, borax, and polyethylene glycol. Examples of colorants include titanium oxide and iron oxide. Examples of flavor-smell-masking agents include sucrose, orange peel, citric acid, and tartaric acid.

In the preparation of a liquid formulation for oral use, for example, a flavoring agent, a buffer, a stabilizer, and/or a smell-masking agent is added to the compound of the present disclosure to produce an internal fluid medicine, a syrup medicine, an elixir, or the like according to an ordinary method.

The flavor-smell-masking agent can be those listed above. The buffer can be, for example, sodium citrate. The stabilizer can be, for example, tragacanth, gum arabic, or gelatin. Optionally, enteric coating or coating intended for persistence of the efficacy may be added to an oral formulation according to a known method. Examples of such coating agents include hydroxypropyl methylcellulose, ethylcellulose, hydroxymethy cellulose, hydroxypropyl cellulose, polyoxyethylene glycol, and Tween 80 (registered trademark).

In the preparation of an injectable drug, for example, a pH adjuster, a buffer, a stabilizer, a tonicity agent, and/or a local anesthetic is added to the compound of the present disclosure to produce a subcutaneously, intramuscularly, or intravenously injectable drug according to an ordinary method.

Examples of pH adjusters and buffers include sodium citrate, sodium acetate, and sodium phosphate. Examples of stabilizers include sodium pyrosulfite, EDTA, thioglycolic acid, and thiolactic acid. Examples of local anesthetics include procaine hydrochloride and lidocaine hydrochloride. Examples of tonicity agents include sodium chloride, glucose, D-mannitol, and glycerin.

The amount of the compound of the present disclosure to be contained in each unit dosage form described above is preferably 0.05 to 1000 mg for an oral drug, 0.01 to 500 mg for an injectable drug, and 1 to 1000 mg for a suppository, typically per unit dosage form, although the amount is not constant, for example, due to the symptoms of the patient to be treated with the compound or its dosage form.

Although the daily dose of the drug in the dosage forms described above cannot be generalized due to the variation of the patient's symptoms, body weight, age, gender, etc., the daily dose based on the compound of the present disclosure may be typically 0.05 to 5000 mg, preferably 0.1 to 1000 mg, for an adult (body weight: 50 kg). The drug in this dosage can be administered once daily or in about two to three divided doses daily.

The present disclosure also relates to the following embodiments.
[1] A compound represented by the following formula (I) or a salt thereof: 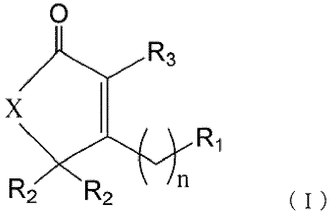 wherein
   X represents -CH₂ -, -CH(CH₃)-, -CH₂-CH₂-, or -CH=CH-,
   R₁ represents -CH₂OR₄, -CH₂OCON(R₅)₂, -CONHR₆, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OR₇, or a halogen atom,
   R₄ represents CₘH₂ₘ₊₁,
   R₅s are identical or different and each represent a hydrogen atom or a methyl group,
   R₆ represents a hydrogen atom or a methyl group,
   R₇ represents a C1-C3 alkyl group,
   m represents an integer of 1 to 14, and
   n represents an integer of 1 to 15,
   with the proviso that when R₄ is a linear alkyl group, the sum of m and n is an integer of 12 to 17, and when R₄ is a branched alkyl group, the sum of m_{b} and n is an integer of 12 to 17, wherein m_{b} is the number of carbon atoms of the longest linear chain of the branched alkyl group.
[2] A compound represented by the following formula (I) or a salt thereof: 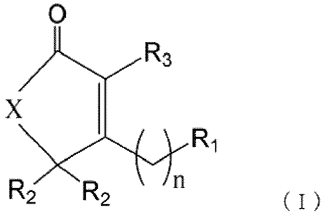 wherein
   X represents -CH₂ -, -CH(CH₃)-, -CH₂-CH₂-, or -CH=CH-,
   R₁ represents -CH₂OR₄, -CH₂OCON(R₅)₂, -CONHR₆, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OR₇, or a halogen atom,
   R₄ represents CₘH₂ₘ₊₁,
   R₅s are identical or different and each represent a hydrogen atom or a methyl group,
   R₆ represents a hydrogen atom or a methyl group,
   R₇ represents a C1-C3 alkyl group,
   m represents an integer of 1 to 14, and
   n represents an integer of 1 to 14,
   with the proviso that when R₄ is a linear alkyl group, the sum of m and n is an integer of 12 to 15, and when R₄ is a branched alkyl group, the sum of m_{b} and n is an integer of 12 to 15, wherein m_{b} is the number of carbon atoms of the longest linear chain of the branched alkyl group.
[3] A compound represented by the following formula (I) or a salt thereof: 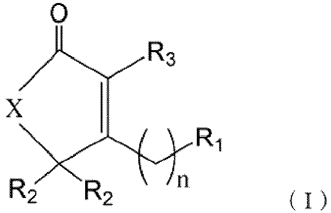 wherein
   X represents -CH₂-, -CH(CH₃)-, -CH₂-CH₂-, or -CH=CH-,
   R₁ represents -CH₂OR₄, -CH₂OCON(CH₃)₂, -CONHR₆, or a cyano group,
   R₂s are identical or different and each represent a hydrogen atom or a methyl group, or two R₂s may form a cyclopropyl group together with the adjacent carbon atom, and
   R₃ represents a hydrogen atom, a methyl group, -CH₂OCH₃, or an iodine atom,
   with the proviso that when R₂s are identical or different and each represent a hydrogen atom or a methyl group, R₃ represents a hydrogen atom, a methyl group, -CH₂OCH₃, or an iodine atom, and when two R₂s form a cyclopropyl group together with the adjacent carbon atom, R₃ represents a methyl group, -CH₂OCH₃, or an iodine atom,
   R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12, and R₆ represents a hydrogen atom or a methyl group.
[4] The compound or a salt thereof according to any one of [1] to [3], wherein X represents -CH₂-CH₂-, -CH₂-CH₂-, or -CH=CHin formula (I).
[5] The compound or a salt thereof according to any one of [1] to [4],
   wherein in formula (I),
   R₁ represents -CH₂OR₄, -CONH₂, or a cyano group,
   R₂s are identical or different and each represent a hydrogen atom or a methyl group, or two R₂s may form a cyclopropyl group together with the adjacent carbon atom,
   R₃ represents a hydrogen atom, a methyl group, -CH₂OCH₃, or an iodine atom, with the proviso that when R₃ is a hydrogen atom, each R₂ is a hydrogen atom, when R₃ is a methyl group, -CH₂OCH₃, or an iodine atom, R₂s are identical or different and each represent a hydrogen atom or a methyl group, or two R₂s may form a cyclopropyl group together with the adjacent carbon atom, and R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12.
[6] The compound or a salt thereof according to any one of [1] to [5], wherein in formula (I),
   X represents -CH₂- or -CH₂-CH₂-,
   R₁ represents -CH₂OR₄ or -CONH₂,
   each R₂ represents a methyl group,
   R₃ represents a methyl group or -CH₂OCH₃, and
   R₄ represents a methyl group, an ethyl group, or an isopropyl group, with the proviso that when R₄ is a methyl group, n represents an integer of 11 to 14, and when R₄ is an ethyl group or an isopropyl group, n represents an integer of 11 to 13.
[7] The compound or a salt thereof according to any one of [1] to [6], wherein X represents -CH₂-CH₂- in formula (I) .
[8] The compound or a salt thereof according to any one of [1] to [7], which is any one of the following compounds (1) to (10) :
   (1) 3-(12-methoxydodecyl)-2,4,4-trimethylcyclohex-2-en-1-one,
   (2) 3-(12-ethoxydodecyl)-2,4,4-trimethylcyclohex-2-en-1-one,
   (3) 3-(12-isopropoxydodecyl)-2,4,4-trimethylcyclohex-2-en-1-one,
   (4) 3-(13-methoxytridecyl)-2,4,4-trimethylcyclohex-2-en-1-one,
   (5) 3-(13-ethoxytridecyl)-2,4,4-trimethylcyclohex-2-en-1-one,
   (6) 3-(13-isopropoxytridecyl)-2,4,4-trimethylcyclohex-2-en-1-one,
   (7) 3-(14-methoxytetradecyl)-2,4,4-trimethylcyclohex-2-en-1-one,
   (8) 3-(14-ethoxytetradecyl)-2,4,4-trimethylcyclohex-2-en-1-one,
   (9) 3-(14-isopropoxytetradecyl)-2,4,4-trimethylcyclohex-2-en-1-one, and
   (10) 3-(15-methoxypentadecyl)-2,4,4-trimethylcyclohex-2-en-1-one.
[9] The compound or a salt thereof according to any one of [1] to [8], wherein the compound is 3-(15-methoxypentadecyl)-2,4,4-trimethylcyclohex-2-en-1-one.
[10] A nerve growth promoter comprising the compound or a salt thereof of any one of [1] to [9] as an active ingredient.
[11] A therapeutic agent for a neurodegenerative disease, comprising the compound or a salt thereof of any one of [1] to [9] as an active ingredient.
[12] A therapeutic agent for amyotrophic lateral sclerosis, comprising the compound or a salt thereof of any one of [1] to [9] as an active ingredient.
[13] A therapeutic agent for pain, comprising the compound or a salt thereof of any one of [1] to [9] as an active ingredient.
[14] A therapeutic agent for a lower urinary tract disfunction, comprising the compound or a salt thereof of any one of [1] to [9] as an active ingredient.
[15] A pharmaceutical composition comprising
   the compound or a salt thereof of any one of [1] to [9], and
   a pharmaceutical carrier.
[16] The compound or a salt thereof according to any one of [1] to [9], which is for use in the treatment of a disease that is ameliorated by nerve growth promotion.
[17] Use of the compound or a salt thereof of any one of [1] to [9] in the production of a nerve growth promoter.
[18] A method for treating a disease that is ameliorated by nerve growth promotion, comprising administering an effective dose of the compound or a salt thereof of any one of [1] to [9] to a subject in need thereof.
[19] The pharmaceutical composition according to [15], which is for use in the treatment of a disease that is ameliorated by nerve growth promotion.
[20] The compound or a salt thereof according to any one of [1] to [9], which is for use in the treatment of a neurodegenerative disease.
[21] The compound or a salt thereof according to any one of [1] to [9], which is for use in the production of a therapeutic agent for a neurodegenerative disease.
[22] A method for treating a neurodegenerative disease, comprising administering an effective dose of the compound or a salt thereof of any one of [1] to [9] to a subject in need thereof.
[23] The pharmaceutical composition according to [15], which is for use in the treatment of a neurodegenerative disease.
[24] The compound or a salt thereof according to any one of [1] to [9], which is for use in the treatment of amyotrophic lateral sclerosis.
[25] Use of the compound or a salt thereof of any one of [1] to [9] in the production of a therapeutic agent for amyotrophic lateral sclerosis.
[26] A method for treating an amyotrophic lateral sclerosis, comprising administering an effective dose of the compound or a salt thereof of any one of [1] to [9] to a subject in need thereof.
[27] The pharmaceutical composition according to [15], which is for use in the treatment of amyotrophic lateral sclerosis.
[28] The compound or a salt thereof according to any one of [1] to [9], which is for use in the treatment of pain.
[29] Use of the compound or a salt thereof of any one of [1] to [9] in the production of a therapeutic agent for pain.
[30] A method for treating pain, comprising administering an effective dose of the compound or a salt thereof of any one of [1] to [9] to a subject in need thereof.
[31] The pharmaceutical composition according to [15], which is for use in the treatment of pain.
[32] The compound or a salt thereof according to any one of [1] to [9], which is for use in the treatment of a lower urinary tract dysfunction.
[33] Use of the compound or a salt thereof of any one of [1] to [9] in the production of a therapeutic agent for a lower urinary tract dysfunction.
[34] A method for treating a lower urinary tract dysfunction, comprising administering an effective dose of the compound or a salt thereof of any one of [1] to [9] to a subject in need thereof.
[35] The pharmaceutical composition according to [15], which is for use in the treatment of a lower urinary tract dysfunction.

### Examples

The embodiments of the present invention are described in more detail below with reference to Examples. However, the present disclosure is not limited to the Examples. Although the embodiments of the present invention are sufficiently described by the Examples, a person skilled in the art would understand that various changes and modifications are available. Such changes and modifications are thus included in the present disclosure unless they depart from the spirit and principal concept of the present disclosure.

The reagents used in the Examples are commercially available products unless indicated otherwise. Silica gel column chromatography was performed using Wakosil C-300 (registered trademark) produced by Wako Pure Chemical Industries, Ltd., Wakogel C-300 (registered trademark) produced by Wako Pure Chemical Industries, Ltd., a SNAP-Ultra (registered trademark) silica prepacked column produced by Biotage, or a KP-NH (registered trademark) prepacked column produced by Biotage. NMR spectra were obtained using an AL400 NMR Spectrometer (400 MHz; produced by JEOL), a Mercury 400 NMR Spectrometer (400 MHz; produced by Agilent Technologies, Inc.), a 400-MHz Bruker Avance NEO 400 NMR Spectrometer (400 MHz; produced by Bruker), or a 500-MHz Bruker Avance III HD NMR Spectrometer (500 MHz; produced by Bruker). Tetramethylsilane was used as the internal reference when the deuterated solvent contained tetramethylsilane. Otherwise, an NMR solvent was used for the measurement as the internal reference. All of the δ values are shown in ppm. The microwave reaction was performed using an Initiator produced by Biotage.

LCMS spectra were obtained using an Acquity SQD (quadrupole) produced by Waters Corporation under the following conditions.
Column: Acquity UPLC (registered trademark) BEH C18, 2.1 × 50 mm, 1.7 um (produced by Waters Corporation)
MS detection: ESI positive
UV detection: 254 and 280 nm
Column flow rate: 0.5 mL/minute
Mobile phase: Water/acetonitrile (0.1% formic acid) Injection volume: 1 µL

**Gradient (Table 1)**

| Time (min) | Water | Acetonitrile |
|---|---|---|
| 0 | 50 | 50 |
| 0.1 | 50 | 50 |
| 1.1 | 2 | 98 |
| 3.0 | STOP | |

Preparative reversed-phase HPLC purification was performed under the following conditions using a preparative separation system available from Gilson, Inc.
Column: Xselect CSH Prep C18 5 µm OBD (19 × 50 mm) + (19 × 100 mm), produced by Waters Corporation
UV detection: 254 nm
Column flow rate: 18 mL/min
Mobile phase: Water/acetonitrile (0.1% formic acid)
Injection volume: 0.1 to 0.5 mL

The symbols stand for the following.
- s:: Singlet
- d:: Doublet
- t:: Triplet
- q:: Quartet
- m:: Multiplet
- br:: Broad
- DMSO:: Dimethyl sulfoxide

In terms of compounds 1 to 8, for example, compounds 1 to 5 can be synthesized according to the production method disclosed in WO99/08987, compound 6 can be synthesized according to the production method disclosed in WO2017/125087, and compounds 7 and 8 can be synthesized according to the production method disclosed in Tetrahedron (1998), 54(27), pp. 7735-7748. Alternatively, they can also be synthesized according to the methods shown below. Compound 4 is 3-(15-hydroxypentadecyl)-2,4,4-trimethylcyclohex-2-en-1-one, which differs from the compound of formula (I) of the present disclosure, in that R₁ represents -CH₂OH.

### Synthesis of 3-(12-hydroxydodecyl)-2,4,4-trimethylcyclohex-2-en-1-one (compound 1)

A solution of 5.5 mL of 3,4-dihydro-2H-pyran in 10 mL of chloroform and 1.01 g of pyridinium p-toluenesulfonate was added to a solution of 10.6 g of 12-bromododecan-1-ol in 100 mL of chloroform, and the mixture was stirred at room temperature for 1 hour. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue.

Tetrahydrofuran (40 mL) was added to 694 mg of lithium, and the mixture was heated to 37°C. Then, 30 mL of a tetrahydrofuran solution of the residue obtained above and 4.20 g of 3-isobutoxy-2,6,6-trimethylcyclohex-2-en-1-one was added dropwise thereto, and the mixture was stirred at 50°C for 2 hours. After the reaction mixture was cooled on ice, 20 mL of water was added thereto, and the mixture was stirred at room temperature for 2 hours. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 40 mL of methanol and 20 mL of tetrahydrofuran. Then, 380 mg of p-toluenesulfonic acid monohydrate was added thereto, and the mixture was stirred at room temperature for 2 hours. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 4.76 g of the title compound.

Physical properties: m/z [M+H]⁺ 324.8 [M+Na]⁺ 345.2
NMR(CDCl₃) δ ppm 1.15 (s, 6H), 1.20-1.65 (m, 21H), 1.76 (s, 3H), 1.80 (t, J = 6.9 Hz, 2H), 2.12-2.25 (m, 2H), 2.37-2.52 (m, 2H), 3.58-3.70 (m, 2H).

### Synthesis of 3-(13-hydroxytridecyl)-2,4,4-trimethylcyclohex-2-en-1-one (compound 2)

A solution of 2.7 mL of 3,4-dihydro-2H-pyran in 5 mL of chloroform and 502 mg of pyridinium p-toluenesulfonate, and the mixture was stirred at room temperature for 1 hour. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue.

Tetrahydrofuran (20 mL) was added to 347 mg of lithium, and the mixture was heated to 32°C. Then, 15 mL of a tetrahydrofuran solution of the residue obtained above and 2.10 g of 3-isobutoxy-2,6,6-trimethylcyclohex-2-en-1-one was added dropwise thereto, and the mixture was stirred at 70°C for 2 hours. After the reaction mixture was cooled on ice, 10 mL of water was added thereto, and the mixture was stirred at room temperature for 1 hour. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 20 mL of methanol and 10 mL of tetrahydrofuran. Then, 190 mg of p-toluenesulfonic acid monohydrate was added thereto, and the mixture was stirred at room temperature for 2 hours. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 2.57 g of the title compound.

Physical properties: m/z[M+H]⁺ 338.8 [M+Na]⁺ 359.3
NMR(CDCl₃) δppm 1.15 (s, 6H), 1.20-1.65 (m, 23H), 1.76 (s, 3H), 1.80 (t, J = 6.9 Hz, 2H), 2.10-2.23 (m, 2H), 2.40-2.52 (m, 2H), 3.55-3.73 (m, 2H).

### Synthesis of 3-(14-hydroxytetradecyl)-2,4,4-trimethylcyclohex-2-en-1-one (compound 3)

A solution of 2.7 mL of 3,4-dihydro-2H-pyran in 5 mL of chloroform and 502 mg of pyridinium p-toluenesulfonate, and the mixture was stirred at room temperature for 2 hours. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue.

Tetrahydrofuran (20 mL) was added to 347 mg of lithium, and the mixture was heated to 32°C. Then, 15 mL of a tetrahydrofuran solution of the residue obtained above and 2.10 g of 3-isobutoxy-2,6,6-trimethylcyclohex-2-en-1-one was added dropwise thereto, and the mixture was stirred at 80°C for 2 hours. After the reaction mixture was cooled on ice, 10 mL of water was added thereto, and the mixture was stirred at room temperature for 1 hour. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 20 mL of methanol and 10 mL of tetrahydrofuran. Then, 190 mg of p-toluenesulfonic acid monohydrate was added thereto, and the mixture was stirred at room temperature for 2 hours. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 2.98 g of the title compound.

Physical properties: m/z[M+H]⁺ 352.7 [M+Na]⁺ 373.3
NMR(CDCl₃) δppm 1.15 (s, 6H), 1.20-1.65 (m, 25H), 1.76 (s, 3H), 1.80 (t, J = 6.8 Hz, 2H), 2.10-2.23 (m, 2H), 2.38-2.52 (m, 2H), 3.58-3.72 (m, 2H).

### Synthesis of 3-(16-hydroxyhexadecyl)-2,4,4-trimethylcyclohex-2-en-1-one (compound 5)

A solution of 2.7 mL of 3,4-dihydro-2H-pyran in 5 mL of chloroform and 502 mg of pyridinium p-toluenesulfonate, and the mixture was stirred at room temperature for 3 hours. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue.

Tetrahydrofuran (20 mL) was added to 347 mg of lithium, and the mixture was heated to 32°C. Then, 15 mL of a tetrahydrofuran solution of the residue obtained above and 2.10 g of 3-isobutoxy-2,6,6-trimethylcyclohex-2-en-1-one was added dropwise thereto, and the mixture was stirred at 50°C for 3 hours. After the reaction mixture was cooled on ice, 10 mL of water was added thereto, and the mixture was stirred at room temperature for 1 hour. After the insoluble matter was filtered off with Celite, the resulting filtrate was extracted with ethyl acetate, washed with water and brine, and dried over anhydrous sodium sulfate. After the desiccant was filtered off, the residue obtained after evaporation under reduced pressure was dissolved in 20 mL of methanol and 10 mL of tetrahydrofuran. Then, 190 mg of p-toluenesulfonic acid monohydrate was added thereto, and the mixture was stirred at room temperature for 5 hours. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 3.02 g of the title compound.

Physical properties: m/z[M+H]⁺ 379.6 [M+Na]⁺ 401.4
NMR(CDCl₃) δppm 1.15 (s, 6H), 1.20-1.65 (m, 29H), 1.75 (s, 3H), 1.73-1.85 (m, 2H), 2.10-2.23 (m, 2H), 2.40-2.53 (m, 2H), 3.55-3.70 (m, 2H).

### Synthesis of 2,4,4-trimethyl-3-(15-((tetrahydro-2H-pyran-2-yl)oxy)pentadecyl)cyclohex-2-en-1-one (compound 6)

Pyridinium p-toluenesulfonate (50 mg) and 0.27 mL of 3,4-dihydro-2H-pyran were added to a solution of 729 mg of 3-(15-hydroxypentadecyl)-2,4,4-trimethylcyclohex-2-en-1-one (compound 4, Taiho Pharmaceutical Co., Ltd.) in 7 mL of dichloromethane, and the mixture was stirred for 3 hours. At ice cooling temperature, a saturated aqueous sodium hydrogen carbonate solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 811 mg of the title compound.

Physical properties: m/z[M+H]⁺ 449.5
NMR(CDCl₃) δppm 1.17 (s, 6H), 1.20-1.90 (m, 37H), 2.10-2.25 (m, 2H), 2.45-2.52 (m, 2H), 3.35-3.45 (m, 1H), 3.48-3.60 (m, 1H), 3.70-3.82 (m, 1H), 3.85-3.98 (m, 1H), 4.57-4.63 (m, 1H).

### Synthesis of 3-(15-hydroxypentadecyl)-2-methylcyclohex-2-en-1-one (compound 7)

A solution of 1.4 mL of 3,4-dihydro-2H-pyran in 5 mL of chloroform and 251 mg of pyridinium p-toluenesulfonate, and the mixture was stirred at room temperature for 3 hours. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue (4.1 g). Then, 95 mg of lithium was added to 8 mL of a tetrahydrofuran solution of 1.17 g of the obtained residue and 273 mg of 3-isobutoxy-2-methylcyclohex-2-en-1-one, and the mixture was stirred at 70°C for 4 hours. After the reaction mixture was cooled on ice, water was added thereto, and the mixture was stirred at room temperature for 0.5 hours, extracted with ethyl acetate, washed with water and brine, and dried over anhydrous sodium sulfate. After the desiccant was filtered off, the residue obtained after evaporation under reduced pressure was dissolved in 2 mL of methanol and 4 mL of tetrahydrofuran. Then, 29 mg of p-toluenesulfonic acid monohydrate was added thereto, and the mixture was stirred at room temperature for 4 hours. A saturated aqueous sodium hydrogen carbonate solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 244 mg of the title compound.

Physical properties: m/z[M+H]⁺ 337.2
NMR(CDCl₃) δppm 1.20-1.83 (m, 32H), 1.88-2.02 (m, 2H), 2.23-2.50 (m, 2H), 3.57-3.72 (m, 2H).

### Synthesis of 3-(15-hydroxypentadecyl)-4,4-dimethylcyclohex-2-en-1-one (compound 8)

A solution of 1.4 mL of 3,4-dihydro-2H-pyran in 5 mL of chloroform and 251 mg of pyridinium p-toluenesulfonate, and the mixture was stirred at room temperature for 3 hours. At ice cooling temperature, an aqueous sodium hydrogen carbonate solution was added to the reaction mixture, and the mixture was extracted with chloroform, washed with water and brine, and dried over anhydrous sodium sulfate. After the desiccant was filtered off, evaporation was performed under reduced pressure to obtain a residue (4.1 g). Then, 95 mg of lithium was added to 8 mL of a tetrahydrofuran solution of 1.17 g of the obtained residue and 294 mg of 3-isobutoxy-6,6-dimethylcyclohex-2-en-1-one, and the mixture was stirred at 70°C for 2 hours. After the reaction mixture was cooled on ice, water was added thereto, and the mixture was stirred at room temperature for 0.5 hours, extracted with ethyl acetate, washed with water and brine, and dried over anhydrous sodium sulfate. After the desiccant was filtered off, the residue obtained after evaporation under reduced pressure was dissolved in 2 mL of methanol and 4 mL of tetrahydrofuran. Then, 29 mg of p-toluenesulfonic acid monohydrate was added thereto, and the mixture was stirred at room temperature for 4 hours. A saturated aqueous sodium hydrogen carbonate solution was added to the reaction mixture and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 366 mg of the title compound.

Physical properties: m/z[M+H]⁺ 351.5
NMR(CDCl₃) δppm 1.18 (s, 6H), 1.20-1.63 (m, 27H), 1.86 (t, J = 6.8 Hz, 2H), 2.15-2.28 (m, 2H), 2.43-2.50 (m, 2H), 3.57-3.72 (m, 2H), 5.81 (s, 1H).

### Intermediate 1: Synthesis of 15-(2,6,6-trimethyl-3-oxocyclohex-1-en-1-yl)pentadecanal

Dimethyl sulfoxide (2.0 mL) was added at -70°C to a solution of 1.1 mL of oxalyl chloride in 30 mL of dichloromethane, and the mixture was stirred for 15 minutes. Subsequently, a solution of 3.79 g of 3-(15-hydroxypentadecyl)-2,4,4-trimethylcyclohex-2-en-1-one (compound 4, Taiho Pharmaceutical Co., Ltd.) in 10 mL of dichloromethane was added dropwise thereto over 10 minutes. The internal temperature was raised to -30°C over 20 minutes by removing the dry ice-acetone bath, and then cooled again to -70°C, and 7.0 mL of triethylamine was added thereto, followed by stirring at room temperature for 2 hours. At ice cooling temperature, brine was added to the reaction mixture, and the resulting mixture was extracted with a mixed solvent of ethyl acetate and n-hexane, washed with 1 mol/L hydrochloric acid, saturated sodium bicarbonate, water, brine, and dried over anhydrous sodium sulfate. After the desiccant was filtered off, the residue obtained after evaporation under reduced pressure was purified by silica gel column chromatography (ethyl acetate/hexane) to give 3.04 g of the title compound.

Physical properties: m/z[M+H]⁺ 363.9
NMR(CDCl₃) δppm 1.15 (s, 6H), 1.24-1.51 (m, 22H), 1.53-1.65 (m, 2H), 1.75 (s, 3H), 1.73-1.88 (m, 2H), 2.15-2.24 (m, 2H), 2.38-2.53 (m, 4H), 9.76 (t, J = 2.0 Hz, 1H).

### Intermediate 2: Synthesis of 15-(2,6,6-trimethyl-3-oxocyclohex-1-en-1-yl)pentadecanoic acid

At ice cooling temperature, 4 mL of 2-methyl-2-butene and 1.02 g of potassium dihydrogen phosphate were added to a mixed solution of 1.81 g of 15-(2,6,6-trimethyl-3-oxocyclohex-1-en-1-yl)pentadecanal (i.e., Intermediate 1) in 6 mL of tert-butyl alcohol and 2 mL of water. A solution of 1.71 g of 79% sodium chlorite in 5 mL water was added dropwise to the reaction mixture, and the mixture was stirred at room temperature for 1 hour. Then, at ice cooling temperature, 1 mol/L hydrochloric acid was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (methanol/chloroform) to give 1.69 g of the title compound.

Physical properties: m/z[M+H]⁺ 379.8
NMR(CDCl₃) δppm 1.15 (s, 6H), 1.20-1.45 (m, 22H), 1.55-1.65 (m, 2H), 1.76 (s, 3H), 1.75-1.85 (m, 2H), 2.13-2.26 (m, 2H), 2.35 (t, J = 7.4 Hz, 2H), 2.46 (t, J = 6.8 Hz, 2H).

### Intermediate 3: Synthesis of 4-nitrophenyl(15-(2,6,6-trimethyl-3-oxocyclohex-1-en-1-yl)pentadecyl)carbonate

At ice cooling temperature, 0.43 mL of triethylamine and 443 mg of 4-nitrophenyl chloroformate were added to a solution of 729 mg of 3-(15-hydroxypentadecyl)-2,4,4-trimethylcyclohex-2-en-1-one (compound 4, Taiho Pharmaceutical Co., Ltd.) in 10 mL of dichloromethane, and the mixture was stirred at ice cooling temperature for 1 hour and at room temperature for 5 hours. Then, at ice cooling temperature, water was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 1.03 g of the title compound.

Physical properties: m/z[M+H]⁺ 530.6
NMR(CDCl₃) δppm 1.15 (s, 6H), 1.25-1.56 (m, 24H), 1.75 (s, 3H), 1.70-1.90 (m, 4H), 2.10-2.25 (m, 2H), 2.45 (t, J = 6.8 Hz, 2H), 4.29 (t, J = 6.7 Hz, 2H), 7.39 (d, J = 9.3 Hz, 2H), 8.29 (d, J = 9.3 Hz, 2H).

### Intermediate 4: Synthesis of 15-methoxypentadecan-1-ol

Pyridinium p-toluenesulfonate (251 mg) and 1.35 mL of 3,4-dihydro-2H-pyran were added to a solution of 3.07 g of 15-bromopentadecan-1-ol in 30 mL of chloroform, and the mixture was stirred at room temperature for 2 hours. At ice cooling temperature, an aqueous sodium hydrogen carbonate solution was added to the reaction mixture, and the mixture was extracted with chloroform, washed with water and brine and dried over anhydrous sodium sulfate. After the desiccant was filtered off, the residue obtained after evaporation under reduced pressure was dissolved in 40 mL of tetrahydrofuran. Then, 10 mL of sodium methoxide (5 M methanol solution) and 250 mg of sodium iodide were added thereto, and the mixture was stirred at 70°C for 2 hours. A saturated ammonium chloride solution was added at ice cooling temperature to the residue obtained after evaporation under reduced pressure, and the resulting mixture was extracted with ethyl acetate, washed with water and brine, and dried over anhydrous sodium sulfate. After the desiccant was filtered off, the residue obtained after evaporation under reduced pressure was dissolved in 10 mL of methanol and 5 mL of tetrahydrofuran. Then, 95 mg of p-toluenesulfonic acid monohydrate was added thereto, and the mixture was stirred at room temperature for 3 hours. At ice cooling temperature, an aqueous sodium hydrogen carbonate solution was added to the reaction mixture, and the residue obtained after evaporation under reduced pressure was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 2.15 g of the title compound.

### Intermediate 5: Synthesis of 1-bromo-15-methoxypentadecane

At ice cooling temperature, 2.92 g of carbon tetrabromide and 2.52 g of triphenylphosphine were added to a solution of 2.07 g of 15-methoxypentadecan-1-ol (i.e., Intermediate 4) in 25 mL of dichloromethane, and the mixture was stirred for 1 hour. An aqueous sodium hydrogen carbonate solution was added to the reaction mixture, and the resulting product was extracted with ethyl acetate, washed with water and brine, and dried over anhydrous sodium sulfate. After the desiccant was filtered off, the residue obtained after evaporation under reduced pressure was purified by silica gel column chromatography (chloroform/hexane) to give 2.28 g of the title compound.

Physical properties:
NMR(CDCl₃) δ ppm 1.18-1.58 (m, 24H), 1.78-1.95 (m, 2H), 3.33 (s, 3H), 3.36 (t, J = 6.8 Hz, 2H), 3.41 (t, J = 6.8 Hz, 2H).

### Intermediate 6: Synthesis of 6,6-dimethyl-4,6,7,8-tetrahydro-5H-benzo[d][1,3]dioxy-5-none

Lithium hexamethyldisilazide (1.3 M tetrahydrofuran solution) (14.37 mL) was added at -78°C to a solution of 1.25 g of 4,6,7,8-tetrahydro-5H-benzo[d][1,3]dioxy-5-none in 40 mL of tetrahydrofuran, and the mixture was stirred for 20 minutes. Then, 1.45 mL of iodomethane was added to the reaction mixture, and the mixture was stirred at the same temperature for 80 minutes and at -45°C for 80 minutes. The reaction mixture was cooled to -78°C, and 3.80 mL of lithium hexamethyldisilazide (1.3 M tetrahydrofuran solution) and 0.28 mL of iodomethane were added thereto, followed by stirring at -45°C for 1 hour. A saturated aqueous ammonium chloride solution was added to the reaction mixture, and the resulting mixture was extracted with ethyl acetate, washed with brine, and dried over anhydrous sodium sulfate. After the desiccant was filtered off, the residue obtained after evaporation under reduced pressure was purified by silica gel column chromatography (ethyl acetate/heptane) to give 173.1 mg of the title compound.

### Intermediate 7: Synthesis of 2-(hydroxymethyl)-3-(15-methoxypentadecyl)-4,4-dimethylcyclohex-2-en-1-one

Lithium (0.11 g) was added to a solution of 0.20 g of 6,6-dimethyl-4,6,7,8-tetrahydro-5H-benzo[d][1,3]dioxy-5-none (i.e., Intermediate 6) and 0.42 g of 1-bromo-15-methoxypentadecane (i.e., Intermediate 5) in 8.0 mL of tetrahydrofuran, and the mixture was stirred at 70°C for 85 minutes. After the reaction mixture was cooled on ice, 32 mL of 0.5 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 276 mg of the title compound.

Physical properties: m/z[M+H]⁺ 395.4
NMR(CDCl₃) δppm 1.21 (s, 6H), 1.22-1.67 (m, 26H), 1.80-1.93 (m, 2H), 2.20-2.34 (m, 2H), 2.45-2.55 (m, 2H), 3.03 (t, J = 6.8 Hz, 1H), 3.35 (s, 3H), 3.39 (t, J = 6.6 Hz, 2H), 4.32 (d, J = 7.0 Hz, 2H) .

### Intermediate 8: Synthesis of 6-isobutoxyspiro[2,5]oct-5-en-4-one

Isobutanol (0.54 g) and 31 mg of p-toluenesulfonic acid monohydrate were added to a solution of 0.50 g of spiro[2,5]octane-4,6-dione in 6.5 mL of cyclohexane, and the mixture was stirred at 70°C for 6 hours. Then, 1.0 g of molecular sieves 4A was added to the reaction mixture, and the mixture was heated and stirred for 16 hours. After cooling to room temperature, the insoluble matter was filtered off with ethyl acetate, and a saturated aqueous sodium hydrogen carbonate solution was added. The organic layer was washed with water and brine and dried over anhydrous sodium sulfate. After the desiccant was filtered off, the residue obtained after evaporation under reduced pressure was purified by silica gel column chromatography (ethyl acetate/heptane) to give 0.51 g of the title compound.

### Intermediate 9: Synthesis of 5-methylspiro[2,5]octane-4,6-dione

Spiro[2,5]octane-4,6-dione (1.0 g) and 2.0 g of potassium hydroxide were dissolved in 10 mL of methanol and 20 mL of water, followed by heating to 70°C. Then, 2 mL of iodomethane was added four times at 40-minute intervals, followed by stirring. After cooling to room temperature, a saturated aqueous ammonium chloride solution was added to the residue obtained after evaporation under reduced pressure, and the mixture was extracted with dichloromethane, washed with brine, and dried over anhydrous sodium sulfate. After the desiccant was filtered off, the residue obtained after evaporation under reduced pressure was purified by silica gel column chromatography (ethyl acetate/heptane) to give 0.3 g of the title compound.

### Intermediate 10: Synthesis of 6-isobutoxy-5-methylspiro[2,5]oct-5-en-4-one

5-Methylspiro[2,5]octane-4,6-dione (200 mg) (i.e., Intermediate 9) was dissolved in toluene, and 2.0 equivalents of isobutanol and 0.043 equivalents of p-toluenesulfonic acid monohydrate were added thereto, followed by stirring at 70°C for 1 day. Then, 400 mg of molecular sieves 4A was added to the reaction mixture, and the mixture was stirred at 120°C for 1 day. After cooling to room temperature, the insoluble matter was filtered off with ethyl acetate, and a saturated aqueous sodium hydrogen carbonate solution was added. The organic layer was washed with water and brine and dried over anhydrous sodium sulfate. After the desiccant was filtered off, the residue obtained after evaporation under reduced pressure was purified by silica gel column chromatography (ethyl acetate/heptane) to give 73.8 mg of the title compound.

### Intermediate 11: Synthesis of 3-isobutoxy-2,4,5-trimethylcyclopent-2-en-1-one

Lithium hexamethyldisilazide (1.3 M tetrahydrofuran solution) (9.60 mL) was added at -78°C to a solution of 1.00 g of 3-isobutoxy-2-methylcyclopent-2-en-1-one in 50 mL of tetrahydrofuran, and the mixture was stirred for 10 minutes. Then, 0.78 mL of iodomethane was added to the reaction mixture, and the mixture was stirred at the same temperature for 10 minutes and at -45°C for 3 hours. After cooling the reaction mixture to -78°C, 1.20 mL of lithium hexamethyldisilazide (1.3 M tetrahydrofuran solution) and 0.10 mL of iodomethane were added thereto, and the mixture was stirred at the same temperature for 40 minutes and at -45°C for 1 hour. At ice cooling temperature, a saturated aqueous ammonium chloride solution was added to the reaction mixture, and the resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 419 mg of the title compound.

### Intermediate 12: Synthesis of methyl 15-(2,6,6-trimethyl-3-oxocyclohex-1-en-1-yl)pentadecanoate

1-Hydroxybenzotriazole monohydrate (168 mg), 230 mg of 1-(3-dimethylaminopropyl)-3-ethylcarbodiimide hydrochloride, and 0.4 mL of methanol were added to a solution of 379 mg of 15-(2,6,6-trimethyl-3-oxocyclohex-1-en-1-yl)pentadecanoic acid (i.e., Intermediate 2) in 5 mL of acetonitrile and 1 mL of N,N-dimethylformamide, and the mixture was stirred at room temperature for 1 hour. Then, a solution of 0.21 mL of triethylamine was added to the reaction mixture, and the mixture was stirred at room temperature for 16 hours. At ice cooling temperature, a sodium hydrogen carbonate solution was added to the reaction mixture, and the resulting mixture was extracted with ethyl acetate, washed with water and brine, and dried over anhydrous sodium sulfate. After the desiccant was filtered off, evaporation under reduced pressure was performed to give 386 mg of the title compound.

### Intermediate 13: Synthesis of methyl 15-3-((tertbutyldimethylsilyl)oxy)-2,6,6-trimethylcyclohex-1-en-1-yl)pentadecanoate

At ice cooling temperature, 34 mg of sodium borohydride was added to a solution of 118 mg of methyl 15-(2,6,6-trimethyl-3-oxocyclohex-1-en-1-yl)pentadecanoate (i.e., Intermediate 12) in 2 mL of tetrahydrofuran and 1 mL of methanol, and the mixture was stirred for 2 hours. A saturated aqueous ammonium chloride solution was added to the reaction mixture, and the resulting mixture was extracted with ethyl acetate, washed with water and brine, and dried over anhydrous sodium sulfate. After the desiccant was filtered off, the remaining product obtained after evaporation under reduced pressure was dissolved in 3 mL of dichloromethane. Then, 126 µL of triethylamine, 90 mg of tert-butyldimethylchlorosilane, and 7 mg of 4-dimethylaminopyridine were added thereto, and the mixture was stirred at room temperature for 2 hours. Water was added to the reaction mixture, and the resulting mixture was extracted with ethyl acetate, washed with water and saturated saline, and dried over anhydrous sodium sulfate. After the desiccant was filtered off, the residue obtained after evaporation under reduced pressure was purified by silica gel column chromatography (ethyl acetate/hexane) to give 116 mg of the title compound.

### Intermediate 14: Synthesis of 16-3-((tertbutyldimethylsilyl)oxy)-2,6,6-trimethylcyclohex-1-en-1-yl)-2-methylmethylhexadeca-2-nol

At ice cooling temperature, 2.2 mL of a tetrahydrofuran solution of 0.96 mol/L methylmagnesium bromide was added to a solution of 108 mg of methyl 15-3-((tert-butyldimethylsilyl)oxy)-2,6,6-trimethylcyclohex-1-en-1-yl)pentadecanoate (i.e., Intermediate 13) in 2 mL of tetrahydrofuran, and the mixture was stirred for 1 hour. A saturated aqueous ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 89 mg of the title compound.

### Intermediate 15: Synthesis of tert-butyl((3-(15-methoxy-15-methylhexadecyl)-2,4,4-trimethylcyclohex-2-en-1-yl)oxy)dimethylsilane

At ice cooling temperature, 130 mg of 55% sodium hydride was added to a solution of 509 mg of 16-3-((tertbutyldimethylsilyl)oxy)-2,6,6-trimethylcyclohex-1-en-1-yl)-2-methylmethylhexadeca-2-nol (i.e., Intermediate 14) in 10 mL of tetrahydrofuran, and the mixture was stirred at room temperature for 1 hour. Then, 125 µL of iodomethane was added to the reaction mixture, and the mixture was stirred at room temperature for 2 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (chloroform/hexane) to give 435 mg of the title compound.

### Intermediate 16: Synthesis of 3-(15-bromopentadecyl)-2,4,4-trimethylcyclohex-2-en-1-one

At ice cooling temperature, 3.65 g of carbon tetrabromide and 3.15 g of triphenylphosphine were added to a solution of 3.64 g of 3-(15-hydroxypentadecyl)-2,4,4-trimethylcyclohex-2-en-1-one (compound 4, Taiho Pharmaceutical Co., Ltd.) in 40 mL of dichloromethane, and the mixture was stirred for 3 hours. An aqueous sodium hydrogen carbonate solution was added to the reaction mixture, and the resulting mixture was extracted with ethyl acetate, washed with water and brine, and dried over anhydrous sodium sulfate. After the desiccant was filtered off, a mixed solution of diethyl ether:hexane (1:1) was added to the residue obtained after evaporation under reduced pressure, and the insoluble matter was filtered off. Thereafter, the residue obtained after evaporation under reduced pressure was purified by silica gel column chromatography (ethyl acetate/chloroform) to give 4.18 g of the title compound.

Physical properties: m/z[M+H]⁺ 427.1, 429.2
NMR(CDCl₃) δppm 1.15 (s, 6H), 1.20-1.52 (m, 24H), 1.76 (s, 3H), 1.70-1.85 (m, 4H), 2.10-2.23 (m, 2H), 2.38-2.51 (m, 2H), 3.41 (t, J = 6.9 Hz, 2H).

### Intermediate 17: Synthesis of 3-isobutoxy-2,5,5-trimethylcyclopent-2-en-1-one

Lithium diisopropylamide (1 M hexane-tetrahydrofuran solution) (120 mL) was added to 400 mL of tetrahydrofuran. Then, in a dry ice-acetone bath, a solution of 20.0 g of 3-isobutoxy-2-methylcyclopent-2-en-1-one in 100 mL of tetrahydrofuran was added. Then, 7.36 mL of iodomethane was added to the reaction mixture, and the temperature was raised to 20°C, followed by stirring for 10 minutes. The reaction mixture was cooled in a dry ice-acetone bath, and 120 mL of lithium diisopropylamide (1 M hexane-tetrahydrofuran solution) and 7.36 mL of iodomethane were added thereto. The temperature was then raised to 15°C, followed by stirring for 15 minutes. Water was added at ice cooling temperature to the reaction mixture, and the resulting mixture was extracted with diisopropyl ether, washed with 10% sodium chloride solution and brine, and dried over anhydrous sodium sulfate. After the desiccant was filtered off, the residue obtained after evaporation under reduced pressure was purified by silica gel column chromatography (ethyl acetate/heptane) to give 14.6 g of the title compound.

Physical properties: m/z[M+H]⁺ 197.2
NMR(CDCl₃) δppm 1.01 (d, J = 6.8 Hz, 6H), 1.15 (s, 6H), 1.64 (t, J = 1.8 Hz, 3H), 1.95-2.15 (m, 1H), 2.48 (q, J = 1.8 Hz, 2H), 3.89 (d, J = 6.8 Hz, 2H).

### Intermediate 18: Synthesis of 3-(12-hydroxydodecyl)-2,4,4-trimethylcyclopent-2-en-1-one

Pyridinium p-toluenesulfonate (361 mg) and 1.97 mL of 3,4-dihydro-2H-pyran were added to a solution of 3.81 g of 12-bromododecan-1-ol in 39.6 mL of chloroform, and the mixture was stirred at room temperature for 1 hour. Thereafter, 180 mg of pyridinium p-toluenesulfonate and 985 µL of 3,4-dihydro-2H-pyran were added thereto, and the mixture was stirred at room temperature for 30 minutes. At ice cooling temperature, a saturated aqueous sodium hydrogen carbonate solution was added to the reaction mixture, and the resulting mixture was extracted with chloroform, washed with water and brine, and dried over anhydrous sodium sulfate. After the desiccant was filtered off, evaporation was performed under reduced pressure to obtain a residue.

Tetrahydrofuran (10.8 mL) was added to 249 mg of lithium, and the mixture was heated to 40°C. Then, 10.8 mL of a tetrahydrofuran solution of the residue obtained above and 1.41 g of 3-isobutoxy-2,5,5-trimethylcyclopent-2-en-1-one (i.e., Intermediate 17) was added dropwise thereto, and the mixture was stirred at 50°C for 2 hours. After the reaction mixture was cooled on ice, 7 mL of water was added thereto, and the mixture was stirred at room temperature for 30 minutes. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 14.4 mL of methanol and 10.8 mL of tetrahydrofuran. Then, 137 mg of p-toluenesulfonic acid monohydrate was added thereto, and the mixture was stirred at room temperature for 49 minutes. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 1.88 g of the title compound.

Physical properties: m/z[M+H]⁺ 309.4
NMR(CDCl₃) δppm 1.20 (s, 6H), 1.23-1.75 (m, 21H), 1.70 (s, 3H), 2.28 (s, 2H), 2.20-2.40 (m, 2H), 3.66 (t, J = 6.6 Hz, 2H).

### Intermediate 19: Synthesis of 3-(14-hydroxytetradecyl)-2,4,4-trimethylcyclopent-2-en-1-one

Pyridinium p-toluenesulfonate (171 mg) and 931 µL of 3,4-dihydro-2H-pyran were added to a solution of 1.99 g of 14-bromotetradecan-1-ol in 20.8 mL of chloroform, and the mixture was stirred at room temperature for 45 minutes. Thereafter, 85 mg of pyridinium p-toluenesulfonate and 466 µL of 3,4-dihydro-2H-pyran were added thereto, and the mixture was stirred at room temperature for 30 minutes. At ice cooling temperature, a saturated aqueous sodium hydrogen carbonate solution was added to the reaction mixture, and the resulting mixture was extracted with chloroform, washed with water and brine, and dried over anhydrous sodium sulfate. After the desiccant was filtered off, evaporation was performed under reduced pressure to obtain a residue.

Tetrahydrofuran (5.66 mL) was added to 131 mg of lithium, and the mixture was heated to 40°C. Then, 5.66 mL of a tetrahydrofuran solution of the residue obtained above and 740 mg of 3-isobutoxy-2,5,5-trimethylcyclopent-2-en-1-one (i.e., Intermediate 17) was added dropwise thereto, and the mixture was stirred at 50°C for 2 hours. After the reaction mixture was cooled on ice, 3.7 mL of water was added thereto, and the mixture was stirred at room temperature. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 7.55 mL of methanol and 3.77 mL of tetrahydrofuran. Then, 72 mg of p-toluenesulfonic acid monohydrate was added thereto, and the mixture was stirred at room temperature for 1 hour. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 832 mg of the title compound.

Physical properties: m/z[M+H]⁺ 337.5
NMR(CDCl₃) δppm 1.21 (s, 6H), 1.23-1.65 (m, 25H), 1.70 (s, 3H), 2.28 (s, 2H), 2.20-2.40 (m, 2H), 3.60-3.70 (m, 2H).

### Intermediate 20: Synthesis of 3-(15-hydroxypentadecyl)-2,4,4-trimethylcyclopent-2-en-1-one

Pyridinium p-toluenesulfonate (361 mg) and 1.97 mL of 3,4-dihydro-2H-pyran were added to a solution of 4.42 g of 15-bromopentadecan-1-ol in 39.6 mL of chloroform, and the mixture was stirred at room temperature for 45 minutes. Thereafter, 180 mg of pyridinium p-toluenesulfonate and 985 µL of 3,4-dihydro-2H-pyran were added thereto, and the mixture was stirred at room temperature for 1 hour. At ice cooling temperature, a saturated aqueous sodium hydrogen carbonate solution was added to the reaction mixture, and the resulting mixture was extracted with chloroform, washed with water and brine, and dried over anhydrous sodium sulfate. After the desiccant was filtered off, evaporation was performed under reduced pressure to obtain a residue.

Tetrahydrofuran (10.8 mL) was added to 249 mg of lithium, and the mixture was heated to 40°C. Then, 10.8 mL of a tetrahydrofuran solution of the residue obtained above and 1.41 g of 3-isobutoxy-2,5,5-trimethylcyclopent-2-en-1-one (i.e., Intermediate 17) was added dropwise thereto, and the mixture was stirred at 50°C for 2 hours. After the reaction mixture was cooled on ice, 7.19 mL of water was added thereto, and the mixture was stirred at room temperature. After the insoluble matter was filtered off with Celite, the filtrate was extracted with ethyl acetate, washed with water and brine, and dried over anhydrous sodium sulfate. After the desiccant was filtered off, the residue obtained after evaporation under reduced pressure was dissolved in 14.4 mL of methanol and 7.19 mL of tetrahydrofuran. Then, 137 mg of p-toluenesulfonic acid monohydrate was added thereto, and the mixture was stirred at room temperature for 1 hour. At ice cooling temperature, a saturated aqueous sodium hydrogen carbonate solution was added thereto, and the solvent was removed by evaporation under reduced pressure. Then, the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 2.08 g of the title compound.

Physical properties: m/z[M+H]⁺ 351.5
NMR(CDCl₃) δppm 1.20 (s, 6H), 1.23-1.68 (m, 27H), 1.70 (s, 3H), 2.28 (s, 2H), 2.20-2.40 (m, 2H), 3.60-3.70 (m, 2H).

### Intermediate 21: Synthesis of 15-(2,5,5-trimethyl-3-oxocyclopent-1-en-1-yl)pentadecanal

Dimethyl sulfoxide (3.18 mL) was added to a solution of 1.00 g of 3-(15-hydroxypentadecyl)-2,4,4-trimethylcyclopent-2-en-1-one (i.e., Intermediate 20) in 7.98 mL of dichloromethane. Then, at ice cooling temperature, 3.18 mL of triethylamine and 1.82 g of sulfur trioxide-pyridine complex were added thereto, and the mixture was stirred at room temperature for 30 minutes. At ice cooling temperature, water was added to the reaction mixture, the resulting mixture was extracted with a mixed solvent of ethyl acetate and n-heptane, washed with 0.1 mol/L hydrochloric acid, a saturated aqueous ammonium chloride solution, water, and brine, and dried over anhydrous sodium sulfate. After the desiccant was filtered off, the residue obtained after evaporation under reduced pressure was purified by silica gel column chromatography (ethyl acetate/heptane) to give 530 mg of the title compound.

### Intermediate 22: Synthesis of 15-(2,5,5-trimethyl-3-oxocyclopent-1-en-1-yl)pentadecanoic acid

At ice cooling temperature, 1.22 mL of 2-methyl-2-butene and 310 mg of potassium dihydrogen phosphate were added to a mixed solution of 530 mg of 15-(2,5,5-trimethyl-3-oxocyclopent-1-en-1-yl)pentadecanal (i.e., Intermediate 21) in 1.83 mL of tert-butyl alcohol and 0.609 mL of water. A solution of 515 mg of 80% sodium chlorite in 1.52 mL of water was added dropwise to the reaction mixture, and the mixture was stirred at room temperature for 1 hour. Then, at ice cooling temperature, 1 mol/L hydrochloric acid was added to the reaction mixture, and the resulting mixture was extracted with ethyl acetate, washed with a mixture of a saturated aqueous sodium thiosulfate solution and brine, and brine, and dried over anhydrous sodium sulfate. After the desiccant was filtered off, the residue obtained after evaporation under reduced pressure was purified by silica gel column chromatography (methanol/chloroform) to give 505 mg of the title compound.

Physical properties: m/z[M+H]⁺ 365.5
NMR(CDCl₃) δppm 1.21 (s, 6H), 1.23-1.55 (m, 22H),1.58-1.73 (m, 2H), 1.70 (s, 3H), 2.29 (s, 2H), 2.25-2.45 (m, 4H).

### Example 1: Synthesis of 3-(12-methoxydodecyl)-2,4,4-trimethylcyclohex-2-en-1-one (compound 11)

At ice cooling temperature, 72 mg of sodium tert-butoxide was added to a solution of 161 mg of 3-(12-hydroxydodecyl)-2,4,4-trimethylcyclohex-2-en-1-one (compound 1) in 2.0 mL of tetrahydrofuran, and the mixture was stirred for 30 minutes. Then, 62 µL of iodomethane was added to the reaction solution, and the mixture was stirred at ice cooling temperature for 0.5 hours and at room temperature for 2 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 114 mg of the title compound.

### Example 2: Synthesis of 3-(12-ethoxydodecyl)-2,4,4-trimethylcyclohex-2-en-1-one (compound 12)

At ice cooling temperature, 43 mg of 55% sodium hydride was added to a solution of 161 mg of 3-(12-hydroxydodecyl)-2,4,4-trimethylcyclohex-2-en-1-one (compound 1) in 2.0 mL of tetrahydrofuran, and the mixture was stirred for 30 minutes. Then, 80 µL of iodoethane was added to the reaction mixture, and the mixture was stirred at ice cooling temperature for 1 hour and at room temperature for 24 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 57 mg of the title compound.

### Example 3: Synthesis of 3-(12-isopropoxydodecyl)-2,4,4-trimethylcyclohex-2-en-1-one (compound 13)

Silver oxide (232 mg) was added to a solution of 161 mg of 3-(12-hydroxydodecyl)-2,4,4-trimethylcyclohex-2-en-1-one (compound 1) in 0.57 mL of 2-iodopropane, and the mixture was stirred at room temperature for 7 days. The reaction mixture was diluted in tert-butyl methyl ether, and the insoluble matter was filtered off with Celite. Thereafter, the residue obtained after evaporation under reduced pressure was purified by silica gel column chromatography (ethyl acetate/hexane) to give 106 mg of the title compound.

### Example 4: Synthesis of 2,4,4-trimethyl-3-(12-propoxydodecyl)cyclohex-2-en-1-one (compound 14)

At ice cooling temperature, 86 mg of 55% sodium hydride was added to a solution of 323 mg of 3-(12-hydroxydodecyl)-2,4,4-trimethylcyclohex-2-en-1-one (compound 1) in 5.0 mL of tetrahydrofuran, and the mixture was stirred for 30 minutes. Then, 0.20 mL of 1-iodopropane was added to the reaction mixture, and the mixture was stirred at ice cooling temperature for 1 hour and at room temperature for 2 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 102 mg of the title compound.

### Example 5: Synthesis of 3-(12-butoxydodecyl)-2,4,4-trimethylcyclohex-2-en-1-one (compound 15)

At ice cooling temperature, 86 mg of 55% sodium hydride was added to a solution of 323 mg of 3-(12-hydroxydodecyl)-2,4,4-trimethylcyclohex-2-en-1-one (compound 1) in 5.0 mL of tetrahydrofuran, and the mixture was stirred for 30 minutes. Then, 0.23 mL of 1-iodobutane was added to the reaction mixture, and the mixture was stirred at ice cooling temperature for 1 hour and at room temperature for 2 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 101 mg of the title compound.

### Example 6: Synthesis of 3-(13-methoxytridecyl)-2,4,4-trimethylcyclohex-2-en-1-one (compound 16)

At ice cooling temperature, 72 mg of sodium tert-butoxide was added to a solution of 168 mg of 3-(13-hydroxytridecyl)-2,4,4-trimethylcyclohex-2-en-1-one (compound 2) in 3.0 mL of tetrahydrofuran, and the mixture was stirred for 30 minutes. Then, 62 µL of iodomethane was added to the reaction mixture, and the mixture was stirred at ice cooling temperature for 0.5 hours and at room temperature for 30 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 119 mg of the title compound.

### Example 7: Synthesis of 3-(13-ethoxytridecyl)-2,4,4-trimethylcyclohex-2-en-1-one (compound 17)

At ice cooling temperature, 43 mg of 55% sodium hydride was added to a solution of 168 mg of 3-(13-hydroxytridecyl)-2,4,4-trimethylcyclohex-2-en-1-one (compound 2) in 3.0 mL of tetrahydrofuran, and the mixture was stirred for 30 minutes. Then, 80 µL of iodoethane was added to the reaction mixture, and the mixture was stirred at ice cooling temperature for 1 hour and at room temperature for 4 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 72 mg of the title compound.

### Example 8: Synthesis of 3-(13-isopropoxytridecyl)-2,4,4-trimethylcyclohex-2-en-1-one (compound 18)

Silver oxide (232 mg) was added to a solution of 161 mg of 3-(13-hydroxytridecyl)-2,4,4-trimethylcyclohex-2-en-1-one (compound 2) in 0.57 mL of 2-iodopropane, and the mixture was stirred at room temperature for 5 days. The reaction mixture was diluted in tert-butyl methyl ether, and the insoluble matter was filtered off with Celite. Thereafter, the residue obtained after evaporation under reduced pressure was purified by silica gel column chromatography (ethyl acetate/hexane) to give 71 mg of the title compound.

### Example 9: Synthesis of 2,4,4-trimethyl-3-(13-propoxytridecyl)cyclohex-2-en-1-one (compound 19)

At ice cooling temperature, 86 mg of 55% sodium hydride was added to a solution of 337 mg of 3-(13-hydroxytridecyl)-2,4,4-trimethylcyclohex-2-en-1-one (compound 2) in 5.0 mL of tetrahydrofuran, and the mixture was stirred for 30 minutes. 0.20 mL of 1-iodopropane was added to the reaction mixture, and the mixture was stirred at ice cooling temperature for 1 hour and at room temperature for 5 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 76 mg of the title compound.

### Example 10: Synthesis of 3-(13-butoxytridecyl)-2,4,4-trimethylcyclohex-2-en-1-one (compound 20)

At ice cooling temperature, 86 mg of 55% sodium hydride was added to a solution of 337 mg of 3-(13-hydroxytridecyl)-2,4,4-trimethylcyclohex-2-en-1-one (compound 2) in 5.0 mL of tetrahydrofuran, and the mixture was stirred for 30 minutes. Then, 0.23 mL of 1-iodobutane was added to the reaction mixture, and the mixture was stirred at ice cooling temperature for 1 hour and at room temperature for 5 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 71 mg of the title compound.

### Example 11: Synthesis of 3-(14-methoxytetradecyl)-2,4,4-trimethylcyclohex-2-en-1-one (compound 21)

At ice cooling temperature, 72 mg of sodium tert-butoxide was added to a solution of 175 mg of 3-(14-hydroxytetradecyl)-2,4,4-trimethylcyclohex-2-en-1-one (compound 3) in 3.0 mL of tetrahydrofuran, and the mixture was stirred for 30 minutes. Then, 62 µL of iodomethane was added to the reaction mixture, and the mixture was stirred at ice cooling temperature for 0.5 hours and at room temperature for 24 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 133 mg of the title compound.

### Example 12: Synthesis of 3-(14-ethoxytetradecyl)-2,4,4-trimethylcyclohex-2-en-1-one (compound 22)

At ice cooling temperature, 43 mg of 55% sodium hydride was added to a solution of 175 mg of 3-(14-hydroxytetradecyl)-2,4,4-trimethylcyclohex-2-en-1-one (compound 3) in 3.0 mL of tetrahydrofuran, and the mixture was stirred for 30 minutes. Then, 80 µL of iodoethane was added to the reaction mixture, and the mixture was stirred at ice cooling temperature for 1 hour and at room temperature for 3 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 66 mg of the title compound.

### Example 13: Synthesis of 3-(14-isopropoxytetradecyl)-2,4,4-trimethylcyclohex-2-en-1-one (compound 23)

Silver oxide (232 mg) was added to a solution of 175 mg of 3-(14-hydroxytetradecyl)-2,4,4-trimethylcyclohex-2-en-1-one (compound 3) in 0.57 mL of 2-iodopropane, and the mixture was stirred at room temperature for 6 days. The reaction mixture was diluted in tert-butyl methyl ether, and the insoluble matter was filtered off with Celite. Thereafter, the residue obtained after evaporation under reduced pressure was purified by silica gel column chromatography (ethyl acetate/hexane) to give 43 mg of the title compound.

### Example 14: Synthesis of 3-(15-methoxypentadecyl)-2,4,4-trimethylcyclohex-2-en-1-one (compound 24)

At ice cooling temperature, 30 mg of 55% sodium hydride was added to a solution of 95 mg of 3-(15-hydroxypentadecyl)-2,4,4-trimethylcyclohex-2-en-1-one (compound 4, Taiho Pharmaceutical Co., Ltd.) in 0.8 mL of tetrahydrofuran, and the mixture was stirred for 30 minutes. Then, 50 µL of iodomethane was added to the reaction mixture, and the mixture was stirred at ice cooling temperature for 1 hour and at room temperature for 15 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 47 mg of the title compound.

### Example 15: Synthesis of 3-(15-ethoxypentadecyl)-2,4,4-trimethylcyclohex-2-en-1-one (compound 25)

At ice cooling temperature, 33 mg of 55% sodium hydride was added to a solution of 109 mg of 3-(15-hydroxypentadecyl)-2,4,4-trimethylcyclohex-2-en-1-one (compound 4, Taiho Pharmaceutical Co., Ltd.) in 1.5 mL of tetrahydrofuran, and the mixture was stirred for 30 minutes. Then, 72 µL of iodoethane was added to the reaction mixture, and the mixture was stirred at ice cooling temperature for 1 hour and at room temperature for 2 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 55 mg of the title compound.

### Example 16: Synthesis of 3-(15-isopropoxypentadecyl)-2,4,4-trimethylcyclohex-2-en-1-one (compound 26)

Silver oxide (139 mg) was added to a solution of 109 mg of 3-(15-hydroxypentadecyl)-2,4,4-trimethylcyclohex-2-en-1-one (compound 4, Taiho Pharmaceutical Co., Ltd.) in 0.30 mL of 2-iodopropane, and the mixture was stirred at room temperature for 6 days. The reaction mixture was diluted in diethyl ether, and the insoluble matter was filtered off with Celite. Thereafter, the residue obtained after evaporation under reduced pressure was purified by silica gel column chromatography (ethyl acetate/hexane) to give 65 mg of the title compound.

### Example 17: Synthesis of 3-(15-(methoxy-d₃)pentadecyl)-2,4,4-trimethylcyclohex-2-en-1-one (compound 27)

At ice cooling temperature, 55 mg of 55% sodium hydride was added to a solution of 182 mg of 3-(15-hydroxypentadecyl)-2,4,4-trimethylcyclohex-2-en-1-one (compound 4, Taiho Pharmaceutical Co., Ltd.) in 3.0 mL of tetrahydrofuran, and the mixture was stirred for 30 minutes. Then, 93 µL of iodomethane-d₃ was added to the reaction mixture, and the mixture was stirred at ice cooling temperature for 1 hour and at room temperature for 5 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 151 mg of the title compound.

### Example 18: Synthesis of 3-(15-methoxypentadecyl-15,15-d₂)-2,4,4-trimethylcyclohex-2-en-1-one (compound 28)

At ice cooling temperature, 0.98 mL of triethylamine and 0.59 mL of ethyl chloroformate were added to a solution of 717 mg of 15-(2,6,6-trimethyl-3-oxocyclohex-1-en-1-yl)pentadecanoic acid (i.e., Intermediate 2) in 15 mL of tetrahydrofuran, and the mixture was stirred for 30 minutes. A solution of 544 mg of sodium borodeuteride in 3.0 mL of deuterium oxide was added to the reaction mixture, and the mixture was stirred at the same temperature for 0.5 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 548 mg of 3-(15-hydroxypentadecyl-15,15-d₂)-2,4,4-trimethylcyclohex-2-en-1-one. At ice cooling temperature, 43 mg of 55% sodium hydride was added to a solution of 183 mg of the obtained 3-(15-hydroxypentadecyl-15,15-d₂)-2,4,4-trimethylcyclohex-2-en-1-one in 3.0 mL of tetrahydrofuran, and the mixture was stirred for 30 minutes. Then, 93 µL of iodomethane was added to the reaction mixture, and the mixture was stirred at ice cooling temperature for 1 hour and at room temperature for 3 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 100 mg of the title compound.

### Example 19: Synthesis of 3-(16-methoxyhexadecyl)-2,4,4-trimethylcyclohex-2-en-1-one (compound 29)

At ice cooling temperature, 72 mg of sodium tert-butoxide was added to a solution of 189 mg of 3-(16-hydroxyhexadecyl)-2,4,4-trimethylcyclohex-2-en-1-one (compound 5) in 3.0 mL of tetrahydrofuran, and the mixture was stirred for 30 minutes. Then, 62 µL of iodomethane was added to the reaction mixture, and the mixture was stirred at ice cooling temperature for 0.5 hours and at room temperature for 9 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 127 mg of the title compound.

### Example 20: Synthesis of 3-(16-ethoxyhexadecyl)-2,4,4-trimethylcyclohex-2-en-1-one (compound 30)

At ice cooling temperature, 44 mg of 55% sodium hydride was added to a solution of 189 mg of 3-(16-hydroxyhexadecyl)-2,4,4-trimethylcyclohex-2-en-1-one (compound 5) in 3.0 mL of tetrahydrofuran, and the mixture was stirred for 30 minutes. Then, 80 µL of iodoethane was added to the reaction mixture, and the mixture was stirred at ice cooling temperature for 1 hour and at room temperature for 4 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 46 mg of the title compound.

### Example 21: Synthesis of 3-(16-isopropoxyhexadecyl)-2,4,4-trimethylcyclohex-2-en-1-one (compound 31)

Silver oxide (232 mg) was added to a solution of 189 mg of 3-(16-hydroxyhexadecyl)-2,4,4-trimethylcyclohex-2-en-1-one (compound 5) in 0.57 mL of 2-iodopropane, and the mixture was stirred at room temperature for 5 days. The reaction mixture was diluted in tert-butyl methyl ether, and the insoluble matter was filtered off with Celite. Thereafter, the residue obtained after evaporation under reduced pressure was purified by silica gel column chromatography (ethyl acetate/hexane) to give 106 mg of the title compound.

### Example 22: Synthesis of 15-(2,6,6-trimethyl-3-oxocyclohex-1-en-1-yl)pentadecanamide (compound 32)

1-Hydroxybenzotriazole monohydrate (168 mg) and 230 mg of 1-(3-dimethylaminopropyl)-3-ethylcarbodiimide hydrochloride were added to a solution of 379 mg of 15-(2,6,6-trimethyl-3-oxocyclohex-1-en-1-yl)pentadecanoic acid (i.e., Intermediate 2) in 5 mL of acetonitrile, and the mixture was stirred at room temperature for 15 minutes. Then, 0.5 mL of 28% aqueous ammonia was added to the reaction mixture, and the mixture was stirred at room temperature for 18 hours. At ice cooling temperature, a sodium hydrogen carbonate solution was added to the reaction mixture, and the precipitate was collected by filtration, followed by drying under reduced pressure to give 299 mg of the title compound.

### Example 23: Synthesis of N-methyl-15-(2,6,6-trimethyl-3-oxocyclohex-1-en-1-yl)pentadecanamide (compound 33)

1-Hydroxybenzotriazole monohydrate (168 mg) and 230 mg of 1-(3-dimethylaminopropyl)-3-ethylcarbodiimide hydrochloride were added to a solution of 379 mg of 15-(2,6,6-trimethyl-3-oxocyclohex-1-en-1-yl)pentadecanoic acid (i.e., Intermediate 2) in 5 mL of acetonitrile, and the mixture was stirred at room temperature for 15 minutes. Then, 1 mL of a 2 mol/L methylamine-tetrahydrofuran solution was added to the reaction mixture, and the mixture was stirred at room temperature for 14 hours. At ice cooling temperature, a sodium hydrogen carbonate solution was added to the reaction mixture, and the precipitate was collected by filtration, followed by drying under reduced pressure to give 340 mg of the title compound.

### Example 24: Synthesis of 15-(2,6,6-trimethyl-3-oxocyclohex-1-en-1-yl)pentadecanitrile (compound 34)

The Burgess reagent (108 mg) was added to a solution of 113 mg of 15-(2,6,6-trimethyl-3-oxocyclohex-1-en-1-yl)pentadecanamide (Example 22) in 2 mL of dichloromethane, and the mixture was stirred for 2 hours. The residue obtained after subjecting the reaction mixture to evaporation under reduced pressure was purified by silica gel column chromatography (ethyl acetate/hexane) to give 104 mg of the title compound.

### Example 25: Synthesis of 15-(2,6,6-trimethyl-3-oxocyclohex-1-en-1-yl)pentadecylcarbamate (compound 35)

Twenty-eight percent aqueous ammonia (0.5 mL) was added to a solution of 265 mg of 4-nitrophenyl(15-(2,6,6-trimethyl-3-oxocyclohex-1-en-1-yl)pentadecyl)carbonate (i.e., Intermediate 3) in 5 mL of tetrahydrofuran, and the mixture was stirred at room temperature for 1 hour. A saturated aqueous sodium hydrogen carbonate solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 169 mg of the title compound.

### Example 26: Synthesis of 15-(2,6,6-trimethyl-3-oxocyclohex-1-en-1-yl)pentadecyl dimethylcarbamate (compound 36)

At ice cooling temperature, 0.2 mL of a 2 mol/L dimethylamine-tetrahydrofuran solution was added to a solution of 159 mg of 4-nitrophenyl(15-(2,6,6-trimethyl-3-oxocyclohex-1-en-1-yl)pentadecyl)carbonate (i.e., Intermediate 3) in 3 mL of tetrahydrofuran, and the mixture was stirred at room temperature for 3 hours. At ice cooling temperature, a saturated aqueous ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 113 mg of the title compound.

### Example 27: Synthesis of 3-(15-methoxypentadecyl)cyclohex-2-en-1-one (compound 37)

Lithium (694 mg) was added to a solution of 1.40 g of 3-ethoxycyclohex-2-en-1-one and 3.86 g of 1-bromo-15-methoxypentadecane (i.e., Intermediate 5) in 50 mL of tetrahydrofuran, and the mixture was stirred at 70°C for 22 hours. After the reaction mixture was cooled on ice, 0.5 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 2.10 g of the title compound.

### Example 28: Synthesis of 3-(15-methoxypentadecyl)-2-methylcyclohex-2-en-1-one (compound 38)

Sodium tert-butoxide (43 mg) and 34 µL of iodomethane were added to a solution of 101 mg of 3-(15-hydroxypentadecyl)-2-methylcyclohex-2-en-1-one (compound 7) in 2.0 mL of tetrahydrofuran, and the mixture was stirred at room temperature for 44 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 38 mg of the title compound.

### Example 29: Synthesis of 3-(15-methoxypentadecyl)-4,4-dimethylcyclohex-2-en-1-one (compound 39)

Lithium (80 mg) was added to a solution of 154 mg of 3-methoxy-6,6-dimethylcyclohex-2-en-1-one and 642 mg of 1-bromo-15-methoxypentadecane (i.e., intermediate 5) in 6 mL of tetrahydrofuran, and the mixture was stirred at 70°C for 8 hours. After the reaction mixture was cooled on ice, 0.5 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 224 mg of the title compound.

### Example 30: Synthesis of 2-iodo-3-(15-methoxypentadecyl)-4,4-dimethylcyclohex-2-en-1-one (compound 40)

At ice cooling temperature, 0.63 mL of trimethylsilyl azide was added to a solution of 729 mg of 3-(15-methoxypentadecyl)-4,4-dimethylcyclohex-2-en-1-one (Example 29) in 5 mL of chloroform, and the mixture was stirred for 3 hours. At ice cooling temperature, a solution of 2.03 g of iodine and 2 mL of pyridine in 2 mL chloroform was added to the reaction mixture, and the mixture was stirred at room temperature for 3 days. Then, a 10% aqueous sodium sulfite solution was added thereto, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 693 mg of the title compound.

### Example 31: Synthesis of 3-(15-methoxypentadecyl)-2,4,4-trimethylcyclohex-2,5-dien-1-one (compound 41)

At ice cooling temperature, 609 µL of lithium hexamethyldisilazide (1.3 M tetrahydrofuran solution) was added to a solution of 200 mg of 3-(15-methoxypentadecyl)-2,4,4-trimethylcyclohex-2-en-1-one (Example 14) in 4.0 mL of tetrahydrofuran, and the mixture was stirred for 30 minutes. Then, 100 µL of chlorotrimethylsilane was added to the reaction mixture, and the mixture was stirred at room temperature for 1 hour. An aqueous sodium hydrogen carbonate solution was added to the reaction solution, and the resulting mixture was extracted with ethyl acetate, washed with brine, and dried over anhydrous sodium sulfate. After the desiccant was filtered off, the residue obtained after evaporation under reduced pressure was dissolved in 5.0 mL of tetrahydrofuran, and 609 µL of lithium hexamethyldisilazide (1.3 M tetrahydrofuran solution) was added thereto at -78°C, followed by stirring for 30 minutes. A solution of 102 mg of phenylselenyl chloride in 5.0 mL of tetrahydrofuran was added dropwise to the reaction mixture, and the mixture was stirred at the same temperature for 30 minutes, followed by heating to room temperature. Water was added to the reaction mixture, and the resulting mixture was extracted with ethyl acetate, washed with brine, and dried over anhydrous sodium sulfate. After the desiccant was filtered off, the residue obtained after evaporation under reduced pressure was purified by silica gel column chromatography (ethyl acetate/hexane).
To a solution of 155 mg of the thus-obtained 3-(15-methoxypentadecyl)-2,4,4-trimethyl-6-(phenylselenyl)cyclohex-2-en-1-one in 16 mL of dichloromethane was added 71.5 mg of m-chloroperbenzoic acid, and the mixture was stirred at room temperature for 2 hours. An aqueous sodium thiosulfate solution was added to the reaction mixture, and the resulting mixture was extracted with dichloromethane, washed with brine, and dried over anhydrous sodium sulfate. After the desiccant was filtered off, the residue obtained after evaporation under reduced pressure was purified by silica gel column chromatography (ethyl acetate/hexane) to give 65.2 mg of the title compound.

### Example 32: Synthesis of 2-(methoxymethyl)-3-(15-methoxypentadecyl)-4,4-dimethylcyclohex-2-en-1-one (compound 42)

At ice cooling temperature, 212 mg of 1,8-bis(dimethylamino)naphthalene and 146 mg of trimethyloxonium tetrafluoroborate were added to a solution of 60 mg of 2-(hydroxymethyl)-3-(15-methoxypentadecyl)-4,4-dimethylcyclohex-2-en-1-one (i.e., Intermediate 7) in 1.0 mL of dichloromethane, and the mixture was stirred at room temperature for 1 hour. Then, the reaction mixture was neutralized with 1 M hydrochloric acid, extracted with ethyl acetate, washed with brine, and dried over anhydrous sodium sulfate. After the desiccant was filtered off, the residue obtained after evaporation under reduced pressure was purified by silica gel column chromatography (ethyl acetate/heptane) to give 50 mg of the title compound.

### Example 33: Synthesis of 4-(15-methoxypentadecyl)spiro[2,5]oct-4-en-6-one (compound 43)

Lithium (0.1 g) was added to a solution of 0.2 g of 6-isobutoxyspiro[2,5]oct-5-en-4-one (i.e., Intermediate 8) and 0.5 g of 1-bromo-15-methoxypentadecane (i.e., Intermediate 5) in 8 mL of tetrahydrofuran, and the mixture was stirred at 70°C for 3 hours. After the reaction mixture was cooled on ice, 0.5 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 0.36 g of the title compound.

### Example 34: Synthesis of 4-(15-methoxypentadecyl)-5-methylspiro[2,5]oct-4-en-6-one (compound 44)

Lithium (14 equivalents) was added to a tetrahydrofuran solution of 73.8 mg of 6-isobutoxy-5-methylspiro[2,5]oct-5-en-4-one (i.e., Intermediate 10) and 1.2 equivalents of 1-bromo-15-methoxypentadecane (i.e., Intermediate 5), and the mixture was stirred at 70°C for 4 hours. After the reaction mixture was cooled on ice, 0.5 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 77.9 mg of the title compound.

### Example 35: Synthesis of 3-(15-methoxypentadecyl)-2-methylcyclopent-2-en-1-one (compound 45)

Lithium (38.3 mg) was added to a solution of 63.6 mg of 3-isobutoxy-2-methylcyclopent-2-en-1-one and 232.1 mg of 1-bromo-15-methoxypentadecane (i.e., Intermediate 5) in 3.0 mL of tetrahydrofuran, and the mixture was stirred at 70°C for 4 hours. After the reaction mixture was cooled on ice, 0.5 M hydrochloric acid was added thereto, and the mixture was stirred at room temperature for 30 minutes. The resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 89.0 mg of the title compound.

### Example 36: Synthesis of 3-(15-methoxypentadecyl)-2,4,5-trimethylcyclopent-2-en-1-one (compound 46)

Lithium (35.5 mg) was added to a solution of 75.6 mg of 3-isobutoxy-2,4,5-trimethylcyclopent-2-en-1-one (i.e., Intermediate 11) and 230 mg of 1-bromo-15-methoxypentadecane (i.e., Intermediate 5) in 3.0 mL of tetrahydrofuran, and the mixture was stirred at 70°C for 3.5 hours. After the reaction mixture was cooled on ice, 0.5 M hydrochloric acid was added thereto, and the mixture was stirred at room temperature for 20 minutes. The resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 112.5 mg of the title compound.

### Example 37: Synthesis of 3-(15-methoxypentadecyl)-2,4,4-trimethylcyclopent-2-en-1-one (compound 69)

Lithium (156 mg) was added to a solution of 981 mg of 3-isobutoxy-2,5,5-trimethylcyclopent-2-en-1-one (i.e., Intermediate 17) and 2.89 g of 1-bromo-15-methoxypentadecane (i.e., Intermediate 5) in 15 mL of tetrahydrofuran, and the mixture was stirred at 70°C for 5 hours. After the reaction s mixture was cooled on ice, 1 M hydrochloric acid was added thereto, and the mixture was stirred at room temperature for 30 minutes. The resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 1.68 g of the title compound.

### Example 38: Synthesis of 3-(12-methoxydodecyl)-2,4,4-trimethylcyclopent-2-en-1-one (compound 70)

At ice cooling temperature, 140 mg of sodium tert-butoxide was added to a solution of 300 mg of 3-(12-hydroxydodecyl)-2,4,4-trimethylcyclopent-2-en-1-one (Intermediate 18) in 3.9 mL of tetrahydrofuran, and the mixture was stirred for 30 minutes. Then, 122 µL of iodomethane was added to the reaction mixture, and the mixture was stirred at ice cooling temperature for 0.5 hours and at room temperature for 1 hour. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 199 mg of the title compound.

### Example 39: Synthesis of 2,4,4-trimethyl-3-(12-propoxydodecyl)cyclopent-2-en-1-one (compound 71)

At ice cooling temperature, 141 mg of 55% sodium hydride was added to a solution of 500 mg of 3-(12-hydroxydodecyl)-2,4,4-trimethylcyclopent-2-en-1-one (Intermediate 18) in 8.09 mL of tetrahydrofuran, and the mixture was stirred for 30 minutes. Then, 323 µL of 1-iodopropane was added to the reaction mixture, and the mixture was stirred at ice cooling temperature for 1 hour and at room temperature for 44 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 119 mg of the title compound.

### Example 40: Synthesis of 3-(14-methoxytetradecyl)-2,4,4-trimethylcyclopent-2-en-1-one (compound 72)

At ice cooling temperature, 107 mg of sodium tert-butoxide was added to a solution of 250 mg of 3-(14-hydroxytetradecyl)-2,4,4-trimethylcyclopent-2-en-1-one (Intermediate 19) in 2.98 mL of tetrahydrofuran, and the mixture was stirred for 30 minutes. Then, 122 µL of iodomethane was added to the reaction mixture, and the mixture was stirred at ice cooling temperature for 30 minutes and at room temperature for 3 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 174 mg of the title compound.

### Example 41: Synthesis of 2,4,4-trimethyl-3-(14-ethoxytetradecyl)cyclopent-2-en-1-one (compound 73)

At ice cooling temperature, 130 mg of 55% sodium hydride was added to a solution of 500 mg of 3-(14-hydroxytetradecyl)-2,4,4-trimethylcyclopent-2-en-1-one (Intermediate 19) in 5.95 mL of tetrahydrofuran, and the mixture was stirred for 30 minutes. Then, 246 µL of iodoethane was added to the reaction mixture, and the mixture was stirred at ice cooling temperature for 1 hour and at room temperature for 21 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/heptane) to give 274 mg of the title compound.

### Example 42: Synthesis of 15-(2,5,5-trimethyl-3-oxocyclopent-1-en-1-yl)pentadecanamide (compound 74)

1-Hydroxybenzotriazole monohydrate (115 mg) and 158 mg of 1-(3-dimethylaminopropyl)-3-ethylcarbodiimide hydrochloride were added to a solution of 250 mg of 15-(2,5,5-trimethyl-3-oxocyclopent-1-en-1-yl)pentadecanoic acid (i.e., Intermediate 22) in 3.44 mL of acetonitrile, and the mixture was stirred at room temperature for 16 minutes. Then, 343 µL of 28% aqueous ammonia was added to the reaction mixture, followed by stirring at room temperature for 17 hours. Then, 170 µL of 28% aqueous ammonia was added thereto, followed by stirring at room temperature for 4 hours. Further, 343 µL of 28% aqueous ammonia was added, followed by stirring at room temperature for 20 hours. At ice cooling temperature, a saturated sodium hydrogen carbonate solution was added to the reaction mixture, and the solvent was evaporated under reduced pressure. Thereafter, ethyl acetate and water were added thereto, and the mixture was extracted Heptane was added to the organic layer, and the mixture was washed with 10% sodium chloride solution and dried over anhydrous sodium sulfate. After the desiccant was filtered off, the residue obtained after evaporation under reduced pressure was purified by silica gel column chromatography (methanol/chloroform) to give 146 mg of the title compound.

### Example 43: Synthesis of 15-(2,5,5-trimethyl-3-oxocyclopent-1-en-1-yl)pentadecanitrile (compound 75)

The Burgess reagent (71 mg) was added to a solution of 73 mg of 15-(2,5,5-trimethyl-3-oxocyclopent-1-en-1-yl)pentadecanamide (Example 42) in 2 mL dichloromethane, and the mixture was stirred for 2 hours. The residue obtained after subjecting the reaction mixture to evaporation under reduced pressure was purified by silica gel column chromatography (ethyl acetate/hexane) to give 51 mg of the title compound.

### Reference Example 1: Synthesis of 3-(15-methoxy-15-methylhexadecyl)-2,4,4-trimethylcyclohex-2-en-1-ol (compound 47)

A tetrahydrofuran solution (1.4 mL) of 1 mol/L tetrabutylammonium fluoride was added to a solution of 366 mg of tert-butyl((3-(15-methoxy-15-methylhexadecyl)-2,4,4-trimethylcyclohex-2-en-1-yl)oxy)dimethylsilane (i.e., Intermediate 14) in 5 mL of tetrahydrofuran, and the mixture was stirred at room temperature for 2 days. The residue obtained after subjecting the reaction mixture to evaporation under reduced pressure was purified by silica gel column chromatography (ethyl acetate/hexane) to give 272 mg of the title compound.

### Reference Example 2: Synthesis of 3-(15-methoxy-15-methylhexadecyl)-2,4,4-trimethylcyclohex-2-en-1-one (compound 48)

Activated manganese dioxide (435 mg) was added to a solution of 204 mg of 3-(15-methoxy-15-methylhexadecyl)-2,4,4-trimethylcyclohex-2-en-1-ol (Reference Example 1) in 10 mL of chloroform, and the mixture was stirred for 4 days. After the insoluble matter was filtered off with Celite, the residue obtained after evaporation under reduced pressure was purified by silica gel column chromatography (ethyl acetate/hexane) to give 199 mg of the title compound.

### Reference Example 3: Synthesis of 2,4,4-trimethyl-3-(15-(trifluoromethoxy)pentadecyl)-cyclohex-2-en-1-one (compound 49)

Ethyl acetate (5 mL) was added to 365 mg of 3-(15-hydroxypentadecyl)-2,4,4-trimethylcyclohex-2-en-1-one (compound 4, Taiho Pharmaceutical Co., Ltd.), 514 mg of silver trifluoromethanesulfonate, 531 mg of N-fluoro-N'-(chloromethyl)triethylenediamine bis(tetrafluoroborate), and 174 mg of potassium fluoride. Then, at room temperature, 172 µL of 2-fluoropyridine and 295 µL of (trifluoromethyl)trimethylsilane were added thereto, followed by stirring for 25 hours. The reaction mixture was diluted in ethyl acetate, and the insoluble matter was filtered off with Celite. Thereafter, the residue obtained after evaporation under reduced pressure was purified by silica gel column chromatography (ethyl acetate/hexane) to give 212 mg of the title compound.

### Reference Example 4: Synthesis of 3-(15-(tertbutyldimethylsilyl)oxy)pentadecyl)-2,4,4-trimethylcyclohex-2-en-1-one (compound 50)

Imidazole (102 mg) and 151 mg of tert-butyldimethylchlorosilane were added to a solution of 182 mg of 3-(15-hydroxypentadecyl)-2,4,4-trimethylcyclohex-2-en-1-one (compound 4, Taiho Pharmaceutical Co., Ltd.) in 1 mL of N,N-dimethylformamide, and the mixture was stirred at room temperature for 2 hours. Water was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 226 mg of the title compound.

### Reference Example 5: Synthesis of 3-(15-phenoxypentadecyl)-2,4,4-trimethylcyclohex-2-en-1-one (compound 51)

At ice cooling temperature, 144 mg of triphenylphosphine and 0.12 mL of diisopropyl azodicarboxylate were added to a solution of 182 mg of 3-(15-hydroxypentadecyl)-2,4,4-trimethylcyclohex-2-en-1-one (compound 4, Taiho Pharmaceutical Co., Ltd.) and 52 mg of phenol in 4 mL of tetrahydrofuran, and the mixture was stirred for 1 hour. Then, the resulting mixture was stirred at room temperature for 22 hours. The reaction mixture was diluted in ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 125 mg of the title compound.

### Reference Example 6: Synthesis of 3-(15-(benzyloxy)pentadecyl)-2,4,4-trimethylcyclohex-2-en-1-one (compound 52)

At ice cooling temperature, 55 mg of 55% sodium hydride was added to a solution of 182 mg of 3-(15-hydroxypentadecyl)-2,4,4-trimethylcyclohex-2-en-1-one (compound 4, Taiho Pharmaceutical Co., Ltd.) in 3 mL of tetrahydrofuran, and the mixture was stirred for 30 minutes. Then, 178 µL of benzyl bromide was added to the reaction mixture, and the mixture was stirred at ice cooling temperature for 1 hour and at room temperature for 3 days. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 107 mg of the title compound.

### Reference Example 7: Synthesis of 15-(2,6,6-trimethyl-3-oxocyclohex-1-en-1-yl)pentadecyl benzoate (compound 53)

At ice cooling temperature, 1.05 mL of triethylamine and 0.64 mL of benzoyl chloride were added to a solution of 1.82 g of 3-(15-hydroxypentadecyl)-2,4,4-trimethylcyclohex-2-en-1-one (compound 4, Taiho Pharmaceutical Co., Ltd.) in 20 mL of dichloromethane, and the mixture was stirred at room temperature for 2 days. At ice cooling temperature, water was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 2.34 g of the title compound.

### Reference Example 8: Synthesis of N,N-dimethyl-15-(2,6,6-trimethyl-3-oxocyclohex-1-en-1-yl)pentadecanamide (compound 54)

1-Hydroxybenzotriazole monohydrate (168 mg) and 230 mg of 1-(3-dimethylaminopropyl)-3-ethylcarbodiimide hydrochloride were added to a solution of 379 mg of 15-(2,6,6-trimethyl-3-oxocyclohex-1-en-1-yl)pentadecanoic acid (i.e., Intermediate 2) in 5 mL of acetonitrile, and the mixture was stirred at room temperature for 15 minutes. Then, 1 mL of a 2 mol/L dimethylamine-tetrahydrofuran solution was added to the reaction mixture, and the mixture was stirred at room temperature for 14 hours. At ice cooling temperature, a sodium hydrogen carbonate solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/chloroform) to give 338 mg of the title compound.

### Reference Example 9: Synthesis of 15-(2,6,6-trimethyl-3-oxocyclohex-1-en-1-yl)pentadecylmethylcarbamate (compound 55)

At ice cooling temperature, 0.3 mL of a 2 mol/L methylamine-tetrahydrofuran solution was added to a solution of 265 mg of 4-nitrophenyl(15-(2,6,6-trimethyl-3-oxocyclohex-1-en-1-yl)pentadecyl)carbonate (i.e., Intermediate 3) in 5 mL of tetrahydrofuran, and the mixture was stirred at room temperature for 1 hour. At ice cooling temperature, a saturated aqueous ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 177 mg of the title compound.

### Reference Example 10: Synthesis of 15-(2,6,6-trimethyl-3-oxocyclohex-1-en-1-yl)pentadecylethylcarbamate (compound 56)

At ice cooling temperature, 0.2 mL of a 2 mol/L ethylamine-tetrahydrofuran solution was added to a solution of 159 mg of 4-nitrophenyl(15-(2,6,6-trimethyl-3-oxocyclohex-1-en-1-yl)pentadecyl)carbonate (i.e., Intermediate 3) in 3 mL of tetrahydrofuran, and the mixture was stirred at room temperature for 3 hours. At ice cooling temperature, a saturated aqueous ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 101 mg of the title compound.

### Reference Example 11: Synthesis of ethyl (15-(2,6,6-trimethyl-3-oxocyclohex-1-en-1-yl)pentadecyl)carbonate (compound 57)

Ethanol (175 µL) and 67 µL of 1,8-diazabicyclo[5.4.0]undec-7-ene were added to a solution of 159 mg of 4-nitrophenyl(15-(2,6,6-trimethyl-3-oxocyclohex-1-en-1-yl)pentadecyl)carbonate (i.e., Intermediate 3) in 3 mL of tetrahydrofuran, and the mixture was stirred at room temperature for 22 hours. At ice cooling temperature, a saturated aqueous ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 36 mg of the title compound.

### Reference Example 12: Synthesis of 2,4,4-trimethyl-3-(15-(methylamino)pentadecyl)cyclohex-2-en-1-one (compound 58)

A 2 mol/L methylamine-tetrahydrofuran solution (1 mL) was added to a solution of 214 mg of 3-(15-bromopentadecyl)-2,4,4-trimethylcyclohex-2-en-1-one (i.e., Intermediate 16) in 5 mL of tetrahydrofuran, and the mixture was stirred at room temperature for 4 days. Water was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by NH silica gel column chromatography (ethyl acetate/chloroform) to give 98 mg of the title compound.

### Reference Example 13: Synthesis of 3-(15-(dimethylamino)pentadecyl)-2,4,4-trimethylcyclohex-2-en-1-one (compound 59)

A 2 mol/L dimethylamine-tetrahydrofuran solution (1 mL) was added to a solution of 214 mg of 3-(15-bromopentadecyl)-2,4,4-trimethylcyclohex-2-en-1-one (i.e., Intermediate 16) in 5 mL of tetrahydrofuran, and the mixture was stirred at room temperature for 3 days. Water was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (methanol/chloroform) to give 162 mg of the title compound.

### Reference Example 14: Synthesis of 2-ethyl-3-(15-methoxypentadecyl)-4,4-dimethylcyclohex-2-en-1-one (compound 60)

Potassium hydroxide (1 equivalent), 8 mL of water, and 1.5 equivalents of iodoethane were added to a solution of 1.0 g of 4,4-dimethylcyclohexane-1,3-dione in 20 mL of ethanol, and the mixture was stirred at 70°C for 1 day. The reaction mixture was dried under reduced pressure and filtered with ethyl acetate. Thereafter, the residue obtained after evaporation under reduced pressure was purified by silica gel column chromatography (acetone/heptane) to give 221 mg of 2-ethyl-4,4-dimethylcyclohexane-1,3-dione. The obtained 2-ethyl-4,4-dimethylcyclohexane-1,3-dione (221 mg) was dissolved in toluene, and 2.0 equivalents of isobutanol and 0.043 equivalents of p-toluenesulfonic acid monohydrate were added thereto, followed by stirring at 70°C for 1 day. Then, molecular sieves 4A (2.0 equivalents) was added to the reaction mixture, and the mixture was stirred at 120°C for 1 day. After cooling to room temperature, the insoluble matter was filtered off with ethyl acetate, and a 0.5 mol/L aqueous sodium hydroxide solution was added. The organic layer was washed with water and brine, and dried over anhydrous sodium sulfate. After the desiccant was filtered off, the residue obtained after evaporation under reduced pressure was purified by silica gel column chromatography (ethyl acetate/heptane) to give 143.8 mg of 2-ethyl-3-isobutoxy-6,6-dimethylcyclohex-2-en-1-one. Lithium (14 equivalents) was added to a tetrahydrofuran solution of 143.8 mg of the obtained 2-ethyl-3-isobutoxy-6,6-dimethylcyclohex-2-en-1-one and 1.2 equivalents of 1-bromo-15-methoxypentadecane (Intermediate 5), and the mixture was stirred at 70°C for 4 hours. After the reaction mixture was cooled on ice, 0.5 mol/L hydrochloric acid was added thereto, and the mixture was stirred at room temperature for 30 minutes. The resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 84.6 mg of the title compound.

### Reference Example 15: Synthesis of 3-(15-methoxypentadecyl)-4,4-dimethyl-2-propylcyclohex-2-en-1-one (compound 61)

Potassium hydroxide (1 equivalent), 24 mL of water, and 1.5 equivalents of 1-iodopropane were added to a solution of 3.0 g of 4,4-dimethylcyclohexane-1,3-dione in 24 mL of ethanol, and the mixture was stirred at 70°C for 1 day. The reaction mixture was dried under reduced pressure and filtered with ethyl acetate. Thereafter, the residue obtained after evaporation under reduced pressure was purified by silica gel column chromatography (acetone/heptane) to give 868.5 mg of 4,4-dimethyl-2-propylcyclohexane-1,3-dione. Then, 868.5 mg of the obtained 4,4-dimethyl-2-propylcyclohexane-1,3-dione was dissolved in toluene, and 2.0 equivalents of isobutanol and 0.043 equivalents of p-toluenesulfonic acid monohydrate were added thereto, followed by stirring at 70°C for 1 day. Then, molecular sieves 4A (2.0 equivalents) was added to the reaction mixture, and the mixture was stirred at 120°C for 1 day. After cooling to room temperature, the insoluble matter was filtered off with ethyl acetate, and a 0.5 mol/L aqueous sodium hydroxide solution was added. The organic layer was washed with water and brine, and dried over anhydrous sodium sulfate. After the desiccant was filtered off, the residue obtained after evaporation under reduced pressure was purified by silica gel column chromatography (ethyl acetate/heptane) to give 723 mg of 3-isobutoxy-6,6-dimethyl-2-propylcyclohex-2-en-1-one. Lithium (14 equivalents) were added to a tetrahydrofuran solution of 723 mg of the obtained 3-isobutoxy-6,6-dimethyl-2-propylcyclohex-2-en-1-one and 1.2 equivalents of 1-bromo-15-methoxypentadecane (Intermediate 5), and the mixture was stirred at 70°C for 4 hours. After the reaction mixture was cooled on ice, 0.5 mol/L hydrochloric acid was added thereto, and the mixture was stirred at room temperature for 30 minutes. The resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 78.1 mg of the title compound.

### Reference Example 16: Synthesis of 2-isopropyl-3-(15-methoxypentadecyl)-4,4-dimethylcyclohex-2-en-1-one (compound 62)

Potassium hydroxide (1 equivalent), 24 mL of water, and 1.5 equivalents of 2-iodopropane were added to a solution of 3.0 g of 4,4-dimethylcyclohexane-1,3-dione in 44 mL of ethanol, and the mixture was stirred at 70°C for 1 day. The reaction mixture was dried under reduced pressure and filtered with ethyl acetate. Thereafter, the residue obtained after evaporation under reduced pressure was purified by silica gel column chromatography (acetone/heptane) to give 168.5 mg of 2-isopropyl-4,4-dimethylcyclohexane-1,3-dione. The obtained 2-isopropyl-4,4-dimethylcyclohexane-1,3-dione (168.5 mg) was dissolved in toluene, and 2.0 equivalents of isobutanol and 0.043 equivalents of p-toluenesulfonic acid monohydrate were added thereto, followed by stirring at 70°C for 1 day. Then, molecular sieves 4A (2.0 equivalents) was added to the reaction mixture, and the mixture was stirred at 120°C for 1 day. After cooling to room temperature, the insoluble matter was filtered off with ethyl acetate, and a 0.5 mol/L aqueous sodium hydroxide solution was added. The organic layer was washed with water and brine, and dried over anhydrous sodium sulfate. After the desiccant was filtered off, the residue obtained after evaporation under reduced pressure was purified by silica gel column chromatography (ethyl acetate/heptane) to give 70.2 mg of 3-isobutoxy-2-isopropyl-6,6-dimethylcyclohex-2-en-1-one. Lithium metal (14 equivalents) was added to a tetrahydrofuran solution of 70.2 mg of the obtained 3-isobutoxy-2-isopropyl-6,6-dimethylcyclohex-2-en-1-one and 1.2 equivalents of 1-bromo-15-methoxypentadecane (Intermediate 5), and the mixture was stirred at 70°C for 4 hours. After the reaction mixture was cooled on ice, 0.5 mol/L hydrochloric acid was added thereto, and the mixture was stirred at room temperature for 30 minutes. The resulting mixture was extracted with ethyl acetate and dried over anhydrous sodium sulfate. After the desiccant was filtered off, the residue obtained after evaporation under reduced pressure was purified by silica gel column chromatography (ethyl acetate/heptane) to give 28.5 mg of the title compound.

### Reference Example 17: Synthesis of 3-(15-methoxypentadecyl)-2,4,4-trimethylcyclohexan-1-one (compound 63)

Palladium on carbon (10 mg) was added to a solution of 100 mg of 3-(15-methoxypentadecyl)-2,4,4-trimethylcyclohex-2-en-1-one (Example 14) in 5 mL of ethanol, and the mixture was stirred for 4 hours under a hydrogen atmosphere. The reaction mixture was filtered with Celite, and evaporation was performed under reduced pressure to give 68.9 mg of the title compound.

### Reference Example 18: Synthesis of 2-(15-methoxypentadecyl)-1,3,3-trimethylcyclohex-1-ene (compound 64)

At ice cooling temperature, 240 mg of sodium borohydride was added to a mixed solution of 1 mL of acetonitrile, 1 mL of trifluoroacetic acid, and 1 mL of acetic acid. Then, a solution of 200 mg of 3-(15-methoxypentadecyl)-2,4,4-trimethylcyclohex-2-en-1-one (Example 14) in 0.4 mL of dichloromethane was added, and the mixture was stirred at ice cooling temperature for 2 hours. A saturated aqueous sodium hydrogen carbonate solution was added to the reaction mixture, and the mixture was extracted with dichloromethane, followed by drying over anhydrous sodium sulfate. After the desiccant was filtered off, the residue obtained after evaporation under reduced pressure was purified by silica gel column chromatography (chloroform/hexane) to give 138 mg of the title compound.

### Reference Example 19: Synthesis of 3-(15-methoxypentadecyl)-2,4,4-trimethylcyclohex-2-en-1-ol (compound 65)

At ice cooling temperature, 148 mg of cerium chloride heptahydrate and 15 mg of sodium borohydride were added to a solution of 150 mg of 3-(15-methoxypentadecyl)-2,4,4-trimethylcyclohex-2-en-1-one (Example 14) in 3 mL of methanol, and the mixture was stirred for 30 minutes, followed by stirring at room temperature. A saturated aqueous ammonium chloride solution was added to the reaction mixture, and the resulting mixture was extracted with ethyl acetate and dried over anhydrous sodium sulfate. After the desiccant was filtered off, the residue obtained after evaporation under reduced pressure was purified by silica gel column chromatography (ethyl acetate/hexane) to give 130 mg of the title compound.

### Reference Example 20: Synthesis of 3-(15-methoxypentadecyl)-2,4,4-trimethylcyclohex-2-en-1-amine (compound 66)

Phthalimide (154 mg) and 275 mg of triphenylphosphine were added to a solution of 200 mg of 3-(15-methoxypentadecyl)-2,4,4-trimethylcyclohex-2-en-1-ol (Reference Example 19) in 4 mL of tetrahydrofuran. Then, at ice cooling temperature, 204 µL of diisopropyl azodicarboxylate was added thereto, and the mixture was stirred at room temperature overnight. The residue obtained after subjecting the reaction mixture to evaporation under reduced pressure was purified by silica gel column chromatography (ethyl acetate/hexane). Then, 4 mL of ethanol and 107 µL of hydrazine monohydrate were added thereto, and the mixture was stirred and heated overnight. After the reaction mixture was cooled to room temperature, the precipitate was filtered off, and the residue obtained by subjecting the filtrate to evaporation under reduced pressure was purified by silica gel column chromatography (ethyl acetate/hexane) to give 69.1 mg of the title compound.

### Reference Example 21: Synthesis of 3-(15-methoxypentadecyl)-2,4,4-trimethylcyclohex-2-en-1-one oxime (compound 67)

Water (4 mL), 550 mg of hydroxylamine hydrochloride, and 1.09 g of potassium carbonate were added to a solution of 500 mg of 3-(15-methoxypentadecyl)-2,4,4-trimethylcyclohex-2-en-1-one (Example 14) in 8 mL of ethanol, and the mixture was stirred in a microwave apparatus at 70°C for 2 hours and 30 minutes. Water was added to the reaction mixture, and the resulting mixture was extracted with ethyl acetate, washed with brine, and dried over anhydrous sodium sulfate. After the desiccant was filtered off, the residue obtained after evaporation under reduced pressure was purified by NH silica gel column chromatography (ethyl acetate/dichloromethane) to give 361 mg of the title compound.

### Reference Example 22: Synthesis of 3-(15-methoxypentadecyl)-2,4,4-trimethylcyclohex-2-en-1-one O-methyloxime (compound 68)

O-Methylhydroxylamine hydrochloride (198 mg) and 329 mg of potassium carbonate were added to a solution of 300 mg of 3-(15-methoxypentadecyl)-2,4,4-trimethylcyclohex-2-en-1-one (Example 14) in 3 mL of ethanol, and the mixture was stirred in a microwave apparatus at 70°C for 2 hours and 30 minutes. Water was added to the reaction mixture, and the resulting mixture was extracted with ethyl acetate, washed with brine, and dried over anhydrous sodium sulfate. After the desiccant was filtered off, the residue obtained after evaporation under reduced pressure was purified by silica gel column chromatography (dichloromethane/hexane) to give 151 mg of the title compound.

**Table 1**

| Compound number | Structural formula | Compound number | Structural formula |
|---|---|---|---|
| 1 | 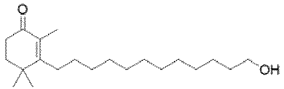 | 5 | 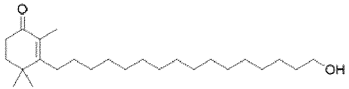 |
| 2 | 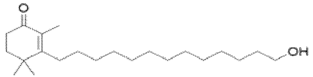 | 6 | 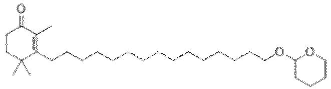 |
| 3 | 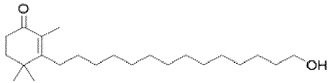 | 7 | 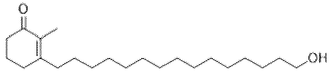 |
| 4 | 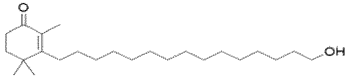 | 8 | 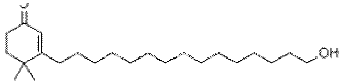 |

**Table 2**

| Compound number | Structural formula | MS m/z (M+1) | NMR |
|---|---|---|---|
| 11 | 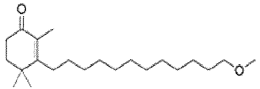 | 337.4 | NMR(CDCl3) *δ*ppm 1.15 (s. 6H). 1.24-1, 45 (m. 18H). 1.50-1.65 (m. 2H). 1.15 (s. 3H). 1.70-1.85 (m. 2H). 2.13-2.22 (m. 2H). 2.45 (t. J = 6.8 Hz. 2H). 3.33 (s. 3H). 3.36 (t. J = 6.6 Hz. 2H). |
| 12 | 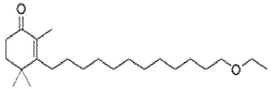 | 351.3 | NMR(CDCl3) *δ*ppm 1.15 (s. 6H). 1.20 (t, J = 7.1 Hz. 3H). 1.24-1.48 (m. 18H). 1.50-1.65 (m. 2H). 1.76 (s. 3H). 1.73-1.88 (m. 2H), 2.15-2.23 (m. 2H), 2.45 (t. J = 7.0 Hz. 2H). 3.40 (t. J = 6.8 Hz. 2H). 3.46 (q. J = 7. 1 Hz. 2H). |
| 13 | 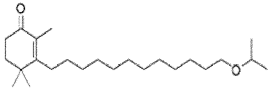 | 365.3 | NMR (CDCl3) *δ*ppm 1.08-1.63 (m. 32H). 1.76 (s. 3H). 1.73-1.85 (m. 2H). 2.15-2.25 (m, 2H). 2.43 (t. J = 6.8 Hz. 2H). 3.39 (t. J = 6.7 Hz. 2H). 3.48-3.62 (m. 1H). |
| 14 | 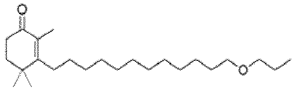 | 365. 3 | NMR(CDCl3) δ ppm 0.94 (t. J = 7.5 Hz. 3H). 1.17 (s. 6H). 1.22-1.65 (m. 22H). 1.78 (s. 3H). 1.73-1.92 (m. 2H). 2.12-2.25 (m. 2H), 2.44-2.49 (m. 2H). 3.39 (t, J = 6.8 Hz. 2H). 3.42 (t, J = 6.8 Hz. 2H). |
| 15 | 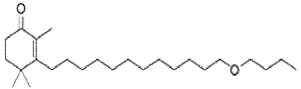 | 379.2 | NMR(CDCI3) *δ*ppm 0.94 (t. J = 7.3 Hz. 3H). 1.17 (s. 6H). 1.20-1.65 (m. 24H), 1.78 (s. 3H). 1.75-1.88 (m. 2M). 2. 17-2. 25 (m. 2H). 2.42-2.50 (m. 2H). 3. 35-3. 48 (m. 4H). |
| 16 | 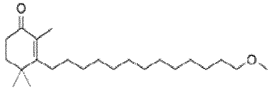 | 351.2 | NMR(CDCl3) *δ*ppm 1.15 (s. 6H) . 1.22-1.46 (m. 200). 1.50-1.65 (m. 2H), 1.76 (s. 3H). 1.70-1.85 (m. 2H). 2.14-2.25 (m. 2H). 2.45 (t. J = 6.8 Hz. 2H). 3.33 (s. 3H). 3.36 (t. J = 6.6 Hz, 2H). |
| 17 | 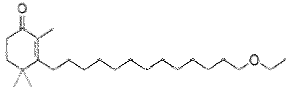 | 365 3 | NMR(CDCl3) *δ*ppm 1.15 (s. 6H). 1.20 (t, J = 7.0 Hz. 3H). 1.20-1.48 (m. 20H). 1.50-1.63 (m. 2H), 1. 76 (s. 3H). 1.73-1.85 (m. 2H). 2.15-2.23 (m. 2H). 2.45 (t. J = 7.0 Hz. 2H). 3.40 (t. J = 6.8 Hz. 2H). 3.46 (q. J = 7.1 Hz. 2H). |
| 18 | 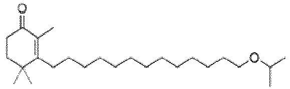 | 379.3 | NMR (CDCl3) *δ*ppm 1.10-1.63 (m. 34H). 1.76 (s. 3H). 1.13-1.85 (m. 2H). 2.15-2.25 (m, 2H). 2.45 (t. J = 6.8 Hz. 2H). 3.39 (t, J = 6.8 Hz. 2H), 3.50-3.62 (m. 1H). |
| 19 | 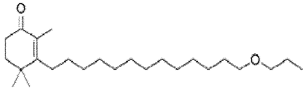 | 379.3 | NMR(CDCl3) *δ*ppm 0.94 (t. J = 7.5 Hz. 3H). 1.17 (s. 6H). 1.22-1.61 (m. 24H). 1.78 (s. 3H). 1.71-1.90 (m. 2H), 2.14-2.24 (m. 2H). 2.40-2.52 (m. 2H), 3, 39 (t. J = 6.8 Hz. 2H). 3.42 (t. J = 6.8 Hz. 2H). |
| 20 | 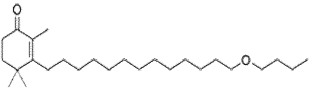 | 393.3 | NMR(CDCl3) *δ*ppm 0.94 (t. J = 7.3 Hz. 3H). 1.17 (s. 6H). 1.25-1.68 (m, 24H). 1.78 (s. 3H). 1.75-1.88 (m, 2H). 2. 15-2. 25 (m. 2H), 2.43-2. 54 (m. 2H), 3. 37-3. 50 (m. 4H). |
| 21 | 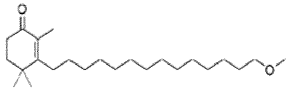 | 365.3 | NMR(CDCl3) *δ*ppm 1.15 (s. 6H). 1.24-1.48 (m. 22H). 1.50-1.64 (m, 2H), 1.76 (s, 3H), 1.70-1.88 (m. 2H). 2.13-2.24 (m, 2H), 2.45 (t. J = 6.8 Hz. 2H). 3, 33 (s. 3H). 3.36 (t, J = 6.7 Hz. 2H). |
| 22 | 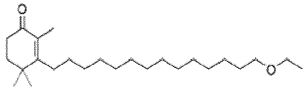 | 379.3 | NMR (CDC 13) *δ*ppm 1.15 (s. 6H), 1.20 (t. J = 7.1 Hz. 3H), 1.20-1.48 (m. 22H). 1.50-1.65 (m. 2H), 1. 76 (s. 3H). 1.73-1.85 (m, 2H), 2. 13-2.25 (m. 2H). 2.45 (t. J = 6.8 Hz. 2H). 3.40 (t. J = 6.7 Hz. 2H). 3.47 (q. J = 7.0 Hz. 2H). |

**Table 3**

| | | | |
|---|---|---|---|
| 23 | 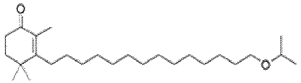 | 393.3 | NMR(CDCl3) *δ*ppm 1.00-1.65 (m. 36H). 1.76 (s. 3H). 1. 70-1.90 (m. 2H). 2. 15-2, 25 (m. 2H). 2, 45 (t. J = 6.9 Hz, 2H). 3.39 (t. J = 6.7 Hz. 2H). 3.50-3.59 (m. 1H). |
| 24 | 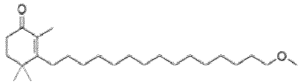 | 379.2 | NMR(CDCl3) *δ*ppm 1.15 (s. 6H). 1.20-1.48 (m. 24H). 1.50-1.63 (m. 2H). 1.75 (s. 3H). 1.75-1.85 (m. 2H). 2.13-2.24 (m. 2H). 2.45 (t. J = 6.8 Hz, 2H). 3.33 (s, 3H). 3.36 (t, J = 6.8 Hz. 2H). |
| 25 | 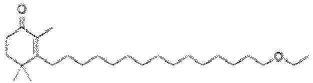 | 393.4 | NMR(CDCl3) *δ*ppm 1.15 (s. 6H). 1.10-1.90 (m. 34H) . 2. 14-2.32 (m. 2H). 2.38-2.60 (m. 2H). 3.40 (t. J = 6.8 Hz. 2H). 3.46 (q. J - 7.1 Hz. 2H). |
| 26 | 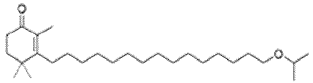 | 407 4 | NMR(CDCl3) *δ*ppm 1.03-1.65 (m. 38H). 1.76 (s. 3H). 1.75-1.88 (m. 2H). 2. 10-2.23 (m. 2H). 2.45 (t, J = 6.9 Hz. 2H). 3.39 (t. J = 6.8 Hz. 2H). 3.45-3.63 (m. 1H). |
| 27 | 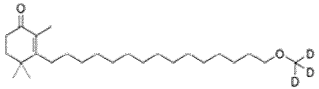 | 382.5 | NMR(CDCl3) *δ*ppm 1.12 (s. 6H). 1.10-1.65 (m. 26H). 1.76 (s. 3H). 1.75-1.88 (m. 2H). 2.13-2.24 (m. 2H). 2.45 (t. J = 6.8 Hz. 2H). 3.36 (t. J = 6.8 Hz. 2H). |
| 28 | 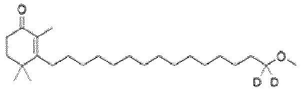 | 381.5 | NMR(CDCl3) *δ*ppm 1.15 (s. 6H), 1.10-1.68 (m, 26H). 1.76 (s. 3H), 1.77-1.82 (m. 2H). 2.10-2.33 (m. 2H), 2.38-2.51 (m. 2H). 3.33 (s. 3H). |
| 29 | 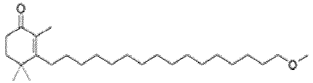 | 393.3 | NMR(CDCl3) *δ*ppm 1.15 (s. 6H). 1.19-1.65 (m. 28H). 1.76 (s. 3H). 1.75-1.85 (m. 2H). 2.10-2.25 (m. 2H). 2.45 (t. J = 6.8 Hz. 2H). 3.33 (s. 3H). 3.36 (t. J = 6.6 Hz. 2H) . |
| 30 | 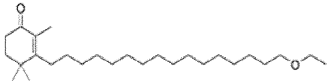 | 407.4 | NMR(CDCl3) *δ*ppm 1.10-1.65 (m. 37H). 1. 76 (s, 3H). 1.70-1.83 (m. 2H). 2.10-2.24 (m. 2H). 2. 38-2. 52 (m. 2H). 3.40 (t, J = 6.7 Hz. 2H). 3.47 (q. J = 7. 1 Hz. 2H). |
| 31 | 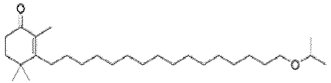 | 421.4 | NMR(CDCl3) *δ*ppm 1.05-1.63 (m. 40H). 1.75 (s. 3H). 1.73-1.85 (m. 2H). 2.10-2.23 (m. 2H). 2.40-2.50 (m. 2H). 3.39 (t. J = 6.8 Hz. 2H). 3.45-3.62 (m. 1H). |
| 32 | 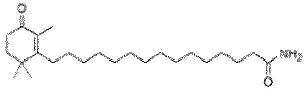 | 378.3 | NMR(C0CI3) *δ*ppm 1.12 (s. 6H). 1.20-1.45 (m. 22H). 1.53-1.70 (m. 211). 1.76 (s. 311), 1.75-1.88 (m. 2H). 2.13-2.32 (m. 4H). 2.46 (t. J = 6.8 Hz. 2H). 5.20-3.58 (br, 2H). |
| 33 | 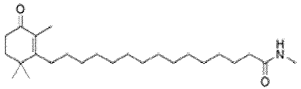 | 392.4 | NMR(CDCl3) *δ*ppm 1.15 (s. 6H). 1.20-1.45 (m. 22H). 1.55-1.70 (m. 2H). 1.75 (s, 3H). 1 73-1.85 (m. 211), 2. 13-2.32 (m. 4H). 2.45 (t, J = 6.8 Hz. 2H). 2 80 (d. J = 4.9 Hz. 3H). 5.32-5.58 (br. !H). |
| 34 | 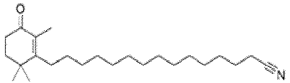 | 360.5 | NMR(CDCl3) *δ*ppm 1.15 (s. 6H). 1.22-1.52 (m. 22H), 1.58-1.70 (m. 2H). 1.76 (s. 3H). 1. 73-1.85 (m. 2H). 2.15-2.24 to. 2H). 2.33 (t. J = 7.1 Hz, 2H). 2.45 (t, J = 6 8 Hz. 2H). |

**Table 4**

| | | | |
|---|---|---|---|
| 35 | 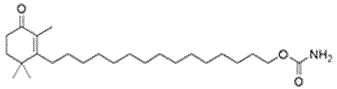 | 409.5 | NMR(CDCl3) *δ*ppm 1.15 (s. 6H). 1, 18-1. 50 (m. 24H), 1.55-1.60 (m. 2H), 1. 75 (s. 3H). 1. 77-1. 82 (m. 2H), 2.15-2.23 (m. 2H), 2. 45 (t. J = 6.8 Hz. 2H). 4.05 (t. J = 6.7 Hz. 2H). 4. 40-4.80 (brs. 2H). |
| 36 | 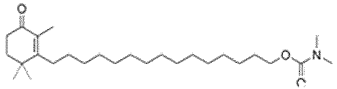 | 436.1 | NMR(CDCl₃) δ ppm 1.15 (s. 6H). 1.22-1.50 (m. 24H). 1.55-1.70 (m. 2H). 1.76 (s. 3H). 1. 12-1.83 (m. 2H). 2. 12-2. 25 (m. 2H). 2.45 (t. J = 6.8 Hz. 2H). 2.90 (s. 6H). 4.05 (t, J = 6. 7 Hz, 2H). |
| 37 | 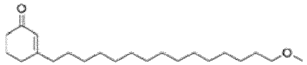 | 337.3 | NMR(CDCl3) *δ*ppm 1.20-1.70 (m. 2611), 1.91-2, 10 (m. 2H). 2.18-2.45 (m. 6H). 3.35 (s. 3H). 3.38 (t, J = 6.1 Hz. 2H). 5.81 (s. 1H). |
| 38 | 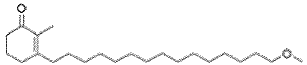 | 351. 3 | NMR(CDCl3) *δ*ppm 1.18-1.72 (m. 26H). 1.61 (s. 3H). 1. 91-1. 99 (m. 2H). 2, 20-2. 45 (m. 611) . 3. 35 (s. 3H). 3.38 (t. J = 6.6 Hz. 2H), |
| 39 | 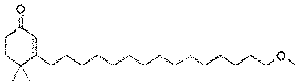 | 365.4 | NMR(CDCl3) *δ*ppm 1.18 (s. 6H). 1.22-1,65 (m. 26H). 1. 85-1. 92 (m. 2H). 2.18-2.30 (m. 2H). 2.43-2.53 (m. 2H). 3.35 (s, 3H). 3.38 (t. J = 6.5 Hz. 2H). 5.81 (s. 1H). |
| 40 | 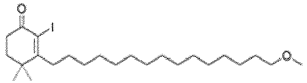 | 491. 3 | NMR(CDCl3) *δ*ppm 1.20-1.70 (m. 32H), 1.83-1.95 (m. 2H). 2.40-2.53 (m. 2H), 2.70-2.83 (m. 2H). 3.35 (s. 3H). 3.39 (t. J = G.6 Hz. 2H). |
| 41 | 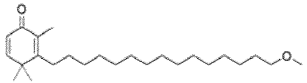 | 377,4 | NMR(CDCl3) *δ*ppm 1.25 (s, 6H). 1.20-1.65 (m. 26H). 1.91 (s. 3H). 2.25-2.40 (m. 2H), 3.35 (s. 3H), 3.39 (t. J = 6.8 Hz. 2H). 6.20 (d. J = 9.8 Hz. 1H). 6, 74 (d. J = 10.0 Hz. 1H). |
| 42 | 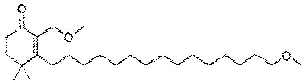 | 409.5 | NMR(CDCl3) *δ*ppm 1.22 (s. 6H). 1.20-1.68 (m. 26H). 1.83-1.90 (m. 2H). 2.27-2.36 (m. 2H). 2.48-2. 60 (m. 2H). 3. 35 (s. 3H). 3.37 (s. 3H). 3.38 (t. J = 6.6 Hz. 2H). 4,14 (s. 2H). |
| 43 | 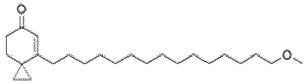 | 363. 4 | NMR(CDCl3) *δ*ppm 0.75-0.83 (m. 2H). 0.95-1. 03 (m. 2H). 1.20-1.90 (m. 30H). 2.45-2.55 (m. 2H). 3.35 (s. 3H). 3.30-3.45 (m. 2H). 5.94 (s. 1H). |
| 44 | 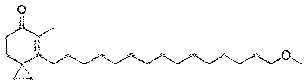 | 377.3 | NMR(CDCl3) *δ*ppm 0.70-0.80 (m. 2H). 0.84-0.95 (m. 2H). 1.20-1.80 (m. 30H). 1.82 (s. 3H). 2.45-2.58 (m. 2H). 3.35 (s. 3H). 3.30-3.50 (m. 2H). |
| 45 | 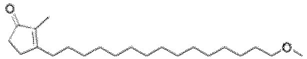 | 337. 3 | NMR(CDCl3) *δ*ppm 1.22-1 45 (m. 22H), 1.50-1.80 (m. 7H). 2.30-2.53 (m. 6H). 3.35 (s. 3H). 3.38 (t. J = G. δ Hz. 2H). |
| 46 | 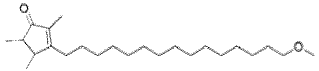 | 365.3 | NMR(CDCl3) *δ*ppm 1.20-1.78 (m. 35H). 1.90-2.56 (m. 4H). 3.36 (s. 3H). 3.39 (t. J = 6.6 Hz. 2H). |

**Table 5**

| Compound number | Structural formula | MS m/z (M+1) | NMR |
|---|---|---|---|
| 47 | 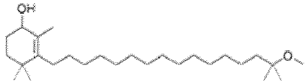 | | NMR(CDCL₃) *δ*ppm 0.96 (s. 3H). 1.03 (s. 3H). 1.13 (s. 6H). 1.28-2.05 (m. 36H). 3.17 (s. 3H). 3.83-3.96 (br. 1H). |
| 48 | 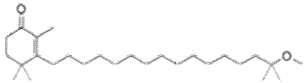 | | NMR(CDCl3) *δ*ppm 1.13 (s. 6H). 1.15 (s. 6H). 1.20-1.55 (m. 26H). 1.65 (s. 3H). 1.68-1.83 (m. 2H). 2.08-2.24 (m. 2H). 2.45 (t. J = 6.8 Hz. 2H). 3.17 (s. 3H). |
| 49 | 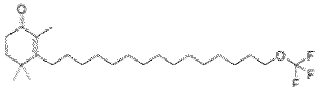 | 433.5 | NMR(CDCl3) *δ*ppm 1.15 (s. 6H). 1.20-1.85 (m. 31H). 2.12-2.24 (m. 2H). 2.40-2.52 (m. 2H). 3.95 (t. J = 6.6 Hz. 2H). |
| 50 | 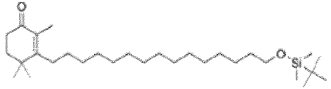 | | NMR(CDCl3) *δ*ppm 0.04 (s. 6H). 0.89 (s. 9H). 1.15 (s. 6H). 1.18-1.58 (m. 26H). 1.15 (s. 3H). 1.70-1.83 (m. 2H). 2, 10-2.22 (m. 2H). 2.45 (t. J = 6.8 Hz. 2H). 3.59 (t. J = 6.6 Hz. 2H). |
| 51 | 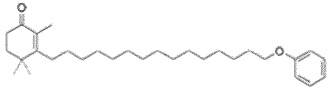 | 441.6 | NMR(CDCl3) *δ*ppm 1.15 (s. 6H). 1.20-1.50 (m. 24H), 1.15 (s. 3H). 1.70-1.85 (m. 4H). 2. 12-2.25 (m. 2H). 2.37-2.50 (m. 2H). 3.95 (t, J = 6. 6 Hz. 2H). 6.83-7.00 (m. 3H). 7.20-7.35 (m. 2H). |
| 52 | 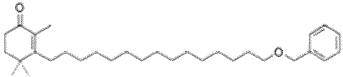 | 456 6 | NMR(CDCl3) *δ*ppm 1.15 (s. 6H). 1.18-1.48 (m. 24H). (1.52-1.70 (m. 2H). 1.75 (s. 3H). 1.72-1.85 (m. 2H). 2.12-2.24 (m. 2H). 2.38-2.52 (m. 2H). 3.46 (t. J = 6, 6 Hz. 2H). 4.50 (s. 2H). 7.20-7.45 (m. 5H). |
| 53 | 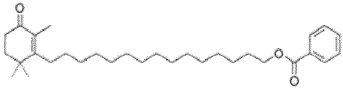 | 469.6 | NMR(CDCl3) *δ*ppm 1.15 (s. 6H). 1.20-1.70 (m. 24H). 1.75 (s, 3H), 1.72-1.85 (m. 4H). 2.10-2.23 (m. 2H). 2.38-2.52 (m. 211). 4.31 (t, J = 6.6 Hz. 2H). 7.38-7.62 (m. 3H). 8.00-8.12 (m. 2H). |
| 54 | 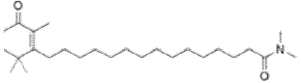 | 401, 7 | NMR(CDCl3) *δ*ppm 1.15 (s. 6H). 1.20-1.45 (m. 22H). 1.55-1.70 (m. 2H). 1.76 (s. 3H). 1.73-1.88 (m. 2H). 2.13-2.23 (m. 2H), 2.25-2.40 (m. 2H). 2.42-2.53 (m. 2H). 2.94 (s. 3H). 3.00 (s. 3H). |
| 55 | 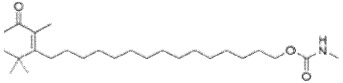 | 422.6 | NMR(CDCl3) *δ*ppm 1.12 (s. 6H). 1.20-1.70 (m. 26H). 1.76 (s. 3H). 1.75-1.85 (m. 2H). 2. 12-2.25 (m. 2H). 2.40-2.53 (m. 2H). 2.70-2.93 (m. 3H). 4.04 (t. J = 6.7 Hz. 2H). 4.40-4.80 (br. 1H). |
| 56 | 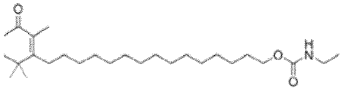 | 436.2 | NMR(CDCl3) *δ*ppm 1.13 (t. J = 7.1 Hz. 3H). 1.15 (s. 6H). 1.20-1.45 (m. 24H). 1.55-1.70 (m. 2H). 1.76 (s, 3H), 1.73-1.85 (m. 2H). 2.10-2.22 (m. 2H). 2.45 (t. J = 6.9 Hz, 2H). 3.15-3.30 (m. 2H). 3.98-4.15 (m. 2H). 4.40-4.65 (br. 1H). |
| 57 | 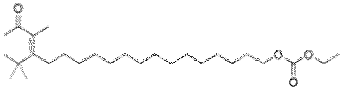 | 437.6 | NMR(CDCl3) δppm 1.15 (s. 6H). 1.20-1.50 (m. 27H). 1.55-1.68 (m. 2H), 1.75 (s. 3H). 1.75-1.85 (m. 2H). 2.10-2.25 (m. 2H). 2.38-2.50 (m. 2H). 4.12 (t. J = 6.6 Hz. 2H). 4, 19 (q, J = 7.1 Hz. 2H) |

**Table 6**

| | | | |
|---|---|---|---|
| 58 | 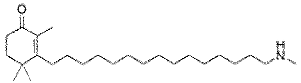 | 379.9 | NMR (CDCI 3) δ ppm 1.14 (s. 6H). 1.21-1.56 (m, 27H), 1.75 (s. 3H), 1. 70-1. 84 (m. 2H). 2.11-2.23 (m. 2H). 2.42 (s. 3H). 2, 40-2. 63 (m. 4H) . |
| 59 | 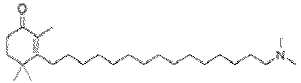 | 393.9 | NMR(CDCl3) *δ* ppm 1.15 (s. 6H). 1.20-1.55 (m, 24H). 1.75 (s. 3H). 1. 10-1.90 (m. 4H). 2. 10-2.30 (m. 4H). 2.21 (s. 6H), 2. 40-2. 52 (m. 2H). |
| 60 | 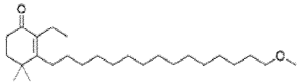 | 393.4 | NMR(CDCl3) *δ* ppm 0.96 (t, J = 7.4 Hz, 3H). 1.17 (s. 6H). 1.20-1.70 (m. 26H), 1. 78-1. 87 (m. 2H). 2. 13-2. 32 (m. 4H), 2. 40-2. 53 (m. 2H) . 3. 35 (s. 3H) . 3.38 (t, J = 6. 6 Hz. 2H), |
| 61 | 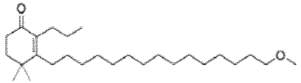 | 407. 4 | NMR (CDCl3) *δ* ppm 0.94 (t. J = 7.3 Hz. 3H). 1.18 (s. 6H). 1.20-2.00 (m. 30H). 2.10-2.25 (m. 4H) . 2. 43-2. 52 (m. 2H), 3.36 (s. 3H). 3 39 (t. J = δ. 6 Hz. 2H). |
| 62 | 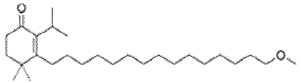 | 407.4 | NMR(CDCl3) *δ* ppm 1.16 (s. 6H). 1.20-1.68 (m, 32H), 1.70-1.82 (m. 2H). 2.08-2. 20 (m. 2H). 2.33-2. 42 (m. 2H). 2.60-2.75 (m. 1H), 3, 36 (s. 3H). 3.39 (t. J = 6. 6 Hz, 2H). |
| 63 | 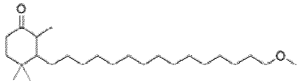 | 381.3 | NMR(CDCl3) δ ppm 0.95-1.90 (m, 40H), 2.24-2.55 (m. 2H), 2. 78-2. 90 (m, 1H) . 3.35 (s. 3H). 3.38 (t. J = 6.8 Hz. 2H). |
| 64 | 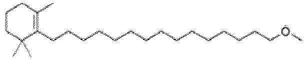 | | NMR(CDCl3) δ ppm 1.00 (s. 6H). 1.25-1.70 (m, 3311), 1.55-2.03 (m. 4H). 3.35 (s. 3H). 3.39 (t. J = 6.6 Hz, 2H), |
| 65 | 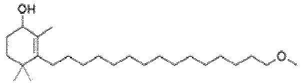 | | NMR(CDCl3) δ ppm 0, 98 (s. 3H). 1.05 (s. 3H). 1.20-2.10 (m. 36H), 3.35 (s. 3H). 3.38 (t. J = G. 7 Hz. 2H). 3. 8.5-4.00 (m. 1H). |
| 66 | 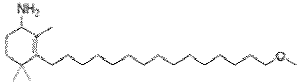 | 380.3 | NMR(CDCl3) δ ppm 0.98 (s. 3H). 1.03 (s. 3H). 1.20-2.10 (m. 37H). 3.02-3. 15 (m. 1H). 3.35 (s. 311). 3.38 (t. J = 6. 7 Hz. 2H). |
| 67 | 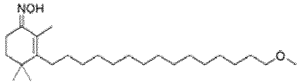 | 394.4 | NMR (CDCI3) δ ppm 1.08 (s. 6H). 1.20-1.65 (m, 28H). 1.82 (s. 3H). 2.10-2.22 (m, 2H). 2.65 (t. J = 6.8 Hz. 2H). 3.35 (s. 3H), 3.39 (t. J = 6.6 Hz. 2H). 7.60-7.90 (br. 2H). |
| 68 | 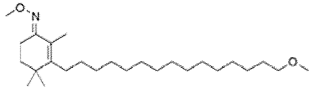 | 408.4 | NMR(CDCl3) δ ppm 1. 06 (s. 6H). 1.22-1.65 (m. 28H), 1.84 (s. 3H). 2.10-2.23 (m. 2H). 2.67 (t. J = 6.8 Hz. 2H). 3.35 (s. 3H). 3.39 (t. J = 6.6 Hz. 2H). 3.91 (s. 2H), |

**Table 7**

| | | | |
|---|---|---|---|
| 69 | 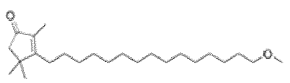 | 365.4 | NMR(CDCl3) δ ppm 1.20 (s. 6H). 1, 25-1.63 (m. 26H), 1.70 (s, 3H). 2.28 4s. 2H). 2.25-2.40 (m. 2H). 3.35 (s. 3H). 3.38 (t. J = 6.7 Hz. 2H). |
| 70 | 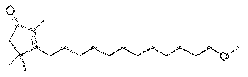 | 322. 3 | NMR(CDCl3) δ ppm 1.21 (s. 6H). 1.25-1.63 (m. 20H). 1. 70 (s, 3H), 2 28 (s, 2H). 2.25-2.40 (m, 2H, 3.35 (s, 3H). 3.38 (t. J = 6.8 Hz. 2H). |
| 71 | 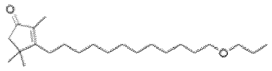 | 351.3 | NMR (CDCI3) δ ppm 0.94 (t, J = 7. 3 Hz. 3H). 1.21 (s. 6H), 1.25-1. 65 (m. 22H). 1.70 (s. 3H) . 2.28 (s. 2H). 2.25-2.40 (m. 2H). 3.38 (t. J = 6.8 Hz. 2H). 3.42 (t, J = 7,3 Hz, 2H). |
| 72 | 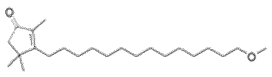 | 351.4 | NMR(CDCl3) δ ppm 1.20 (s, 6H). 1.25-1.63 (m, 24H). 1.70 (s. 3H). 2.28 (s. 2H). 2.25-2.40 (m, 2H), 3. 35 (s. 3H). 3.38 (t. J = 6.8 Hz. 2H). |
| 73 | 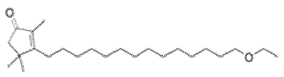 | 365.4 | NMR (CDCI3) δ ppm 1.20 (s. 6H). 1, 22 (t. J = 7. 0 Hz. 3H). 1.25-1.70 (m. 24H). 1.70 (s, 3H). 2 28 (s. 2H). 2.25-2.40 (m, 2H), 3.42 (t, J = 6.8 Hz. 2H). 3.49 (q. J = 7, 0 Hz, 2H). |
| 74 | 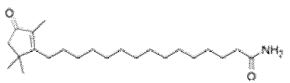 | 364.5 | NMR(CDCl3) δ ppm 1.21 (s. 6H). 1.25-1.80 (m. 24H). 1.70 (s. 3H). 2.18-2.40 (m. 4H) 2.28 (s. 2H). 5.25-5.55 (br. 2H). |
| 15 | 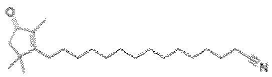 | 346.4 | NMR(CDCl3) δ ppm 1.21 (s. 6H). 1.25-1 80 (m. 24H). 1.70 (s. 3H). 2.25-2.42 (m. 4H) 2.28 (s. 2H). |

### Test Example 1: Pharmacokinetics

The required amounts of compounds were weighed, and drug suspensions were prepared in 0.5% hydroxypropyl methylcellulose. The number of rats in each group was two, and each drug suspension was administered orally to each male rat (Crl: CD(SD)) using an oral sonde (the dose of the compound was 10 mg/kg). At each blood collection point (0.5, 1, 2, 4, 8, and 24 hours after administration), blood was collected from the jugular vein using a syringe and a needle (the anticoagulant was heparin sodium). The collected blood was centrifuged (13000 rpm, 2 min, 4°C) to prepare plasma, followed by deproteinization. Thereafter, the concentration of the compound in the plasma was measured by LC/MS/MS (LCMS-8040 (Shimadzu Corporation) or API 4000 (AB Sciex), LC: 30-A and 20-A series (Shimadzu Corporation) or Waters Acquity (Waters Corporation)).

As shown in Table 8, it was confirmed that the compound 24 had an improvement in area under the curve (AUC) of 1900 times or more and an improvement in maximum blood concentration Cₘₐₓ of 1100 times or more as compared to the compound 4. In addition, it was confirmed that the compound in which R₁ in formula (I) is - CH₂OR₄, the compound in which R₁ is -CH₂OCON(CH₃)₂, the compound in which R₁ is -CONHR₆, and the compound in which R₁ is a cyano group respectively had an improvement in AUC of at least 100 times or more, 1000 times or more, 10 times or more, and 1900 times or more, and an improvement in Cₘₐₓ of at least 30 times or more, 200 times or more, 10 times or more, and 1100 times or more, respectively, as compared to the compound 4. Accordingly, it was shown that the compounds represented by formula (I) had unexpected high pharmacokinetics as compared to the compound 4, which was a known compound.

**Table 8**

| AUC and Cₘₐₓ of each compound | | | |
|---|---|---|---|
| | | AUC_{0-∞} (µM·hr) | Cₘₐₓ(µM) |
| | Compound 4 | 0.003 | 0.002 |
| | Compound 24 | 5.90 | 2.20 |

### Test Example 2: Evaluation of drug efficacy in diabetes mellitus (DM) rat model

To examine the effect of the compound in a diabetes mellitus model, bladder function was analyzed by cystometry based on the method of Saitoh et al. (European Journal of Pharmacology 501 (2004) 143-149).

A streptozotocin (STZ) solution obtained by dissolving STZ in a citrate buffer solution (0.1 M citric acid, pH of 4.2) to a concentration of 32.5 mg/mL was prepared, and administered intraperitoneally (65 mg/kg) to SD rats to induce DM models. The same procedure was performed on sham rats except that a citrate buffer solution was used in place of the STZ solution.

On the day after the model production and 4 weeks later, a blood sample was taken from the tail vein with the rats awake to measure the blood glucose level. AntSense III (Horiba Corporation) was used for the measurement of blood glucose level. Individuals with a blood glucose level of 300 mg/dL or more on the day after the production of the model and 4 weeks later were determined to be established DM models and used in subsequent operations.

The administration of a test substance (compound 4 or 24) was started from the day after the production of the model. A vehicle, or a solution for administering the compound was administered orally twice a day, morning and evening, at a volume of 10 mL/kg for 4 weeks. On the day after the last administration of the test substance, an incision was made in the abdomen of each rat under isoflurane anesthesia, and a catheter (PE-90, Becton Dickinson and Company) was inserted through the apex of the bladder for intravesical pressure measurement. After closing up the abdomen, the rats were placed in a holding cage. At least 30 min after the release of isoflurane anesthesia, a urethane solution was administered subcutaneously (0.8 g/kg).

A pressure transducer (DX-360, Nihon Kohden Kogyo Co., Ltd.) and an infusion pump (TE-331S, Terumo Corporation) were connected via a three-way valve to the other end of a catheter inserted into the bladder. Intravesical pressure during continuous intravesical infusion of a saline solution (infusion rate: 12 mL/hr) was measured using a pressure transducer and polygraph (AP-641G, Nihon Kohden Kogyo Co., Ltd.), and recorded continuously on a computer using a PowerLab (ML866, AD Instruments) (sampling rate: 20/sec). The volume of urination was automatically measured using an electronic balance (GX-200, A&D Company Limited) set directly under the holding cage, and recorded continuously on a computer using a PowerLab (sampling rate: 20/sec). Urinary function parameters were recorded and analyzed using PowerLab analysis software Chart 5 (ver. 5.5.6, AD Instruments).

Immediately after urethane anesthesia, continuous intravesical infusion of saline solution (infusion rate: 3 mL/hr) was performed for about 1.5 hours to familiarize the rats with the cystometry environment. After acclimatization to the cystometry environment, urinary function (intravesical bladder pressure, volume of urination, and residual urine volume) was measured by cystometry (infusion rate: 12 mL/hr).

As shown in Fig. 1, the compound 24 was found to have remarkable drug efficacy as compared to the compound 4.

### Test Example 3: Measurement of nerve growth stimulation effect

To evaluate the nerve growth stimulation effect, neurite bearing assay (proportion of neurite-bearing cells) and neurite length assay (neurite length) were analyzed with reference to the method of Topalli et al. (Brain Research 1030 (2004), pp. 116-124).

An ND3 neuronal cell line (European Collection of Authenticated Cell Cultures) was seeded in a DMEM medium containing 10% fetal bovine serum (0.6 × 10⁴ cells/1 mL) in a 24-well plate. 24 hours later, the medium of seeded cells was removed, and a solution in which the compound of the present disclosure that was sequentially diluted in dimethyl sulfoxide (DMSO) was adjusted in DMEM containing 0.1% bovine serum albumin so that the DMSO final concentration was 0.1%, was added to cells (evaluation performed in duplicate). After six hours from the addition, the proportion of neurite-bearing cells (the number of cells having a neurite longer than the diameter of the cell body/number of total cells), and the neurite length per cell were analyzed. The number of total cells, proportion of neurite-bearing cells, and neurite length per cell were analyzed using a MetaMorph Neurite Outgrowth Application Module (Molecular Devices Japan K.K.). The number of cells was defined as the relative cell number, taking the number of cells per well containing DMSO as 1. The relative activity of each compound when the maximum activity of 3-(15-methoxypentadecyl)-2,4,4-trimethylcyclohexa-2-en-1-one (compound 24) in each test was defined as 1 is shown (Table 9). For the compounds for which multiple tests were conducted, the average value was shown. Data with a number of cells of 0.5 or more was also used. Accordingly, if the number of cells was less than 0.5, the neurite length per cell and the proportion of neurite-bearing cells were defined as "-".

As shown in Table 9, when the compound 24 was added in an amount of 1 to 100 µM, the number of relative cells was 0.5 or more.

**Table 9**

| Number of relative cells in compound 24 of each concentration | | | | | | | |
|---|---|---|---|---|---|---|---|
| | | Number of cells | | | | | |
| | Compound number | 1 µM | 3 µM | 5 µM | 10 µM | 30 µM | 100 µM |
| | 24 | 1.06 | 1.01 | 0.94 | 0.92 | 0.96 | 0.92 |

As shown in Tables 10 to 13, it was confirmed that the compounds of the present disclosure had an excellent nerve growth stimulation effect. Furthermore, it was confirmed that some compounds had a nerve growth stimulation effect equal to or greater than that of the compound 24.

**Table 10**

| | Number of cells | | | | | | Neurite length per cell | | | | | | Proportion of neurite-bearing cells | | | | | |
|---|---|---|---|---|---|---|---|---|---|---|---|---|---|---|---|---|---|---|
| Compound number | 1 µM | 3 µM | 5 µM | 10 µM | 30 µM | 100 µM | 1 µM | 3 µM | 5 µM | 10 µM | 30 µM | 100 µM | 1 µM | 3 µM | 5 µM | 10 µM | 30 µM | 100 µM |
| 1 | 1.03 | 1.05 | 0.99 | 1.03 | 0.99 | 0.09 | 0.19 | 0.18 | 0.21 | 0.30 | 0.67 | - | 0.11 | 0.11 | 0.13 | 0.23 | 0.62 | - |
| 2 | 1.13 | 1.02 | 0.96 | 0.96 | 0.19 | 0.09 | 0.18 | 0.18 | 0.22 | 0.39 | - | - | 0.10 | 0.11 | 0.16 | 0.33 | - | - |
| 3 | 0.98 | 1.05 | 1.10 | 1.08 | 0.11 | 0.13 | 0.22 | 0.24 | 0.33 | 0.54 | - | - | 0.11 | 0.16 | 0.24 | 0.46 | - | - |
| 4 | 1.03 | 0.92 | 0.92 | 1.01 | 0.19 | | 0.26 | 0.33 | 0.39 | 0.71 | - | | 0.24 | 0.34 | 0.41 | 0.70 | - | |
| 5 | 1.11 | 1.09 | 1.05 | 0.83 | 0.49 | 0.40 | 0.22 | 0.36 | 0.56 | 0.92 | - | - | 0.12 | 0.27 | 0.48 | 1.09 | - | - |
| 6 | 0.97 | 0.85 | 0.67 | 0.64 | 0.72 | 0.92 | 0.21 | 0.20 | 0.22 | 0.25 | 0.22 | 0.22 | 0.19 | 0.18 | 0.22 | 0.25 | 0.19 | 0.18 |
| 7 | 1.00 | 1.00 | 0.87 | 0.86 | 0.17 | 0.19 | 0.18 | 0.22 | 0.27 | 0.30 | - | - | 0.15 | 0.20 | 0.25 | 0.28 | - | - |
| 8 | 1.01 | 1.02 | 0.98 | 0.93 | 0.06 | 0.09 | 0.19 | 0.22 | 0.27 | 0.25 | - | - | 0.15 | 0.18 | 0.23 | 0.21 | - | - |
| 11 | 0.96 | 0.96 | 0.83 | 0.82 | 0.62 | 0.70 | 0.30 | 0.37 | 0.54 | 0.91 | 1.66 | 1.83 | 0.27 | 0.37 | 0.61 | 0.98 | 2.10 | 2.10 |
| 12 | 0.96 | 0.90 | 0.77 | 0.69 | 0.11 | 0.03 | 0.32 | 0.41 | 0.58 | 1.55 | - | - | 0.32 | 0.43 | 0.69 | 2.02 | - | - |
| 13 | 0.91 | 0.87 | 0.75 | 0.73 | 0.34 | 0.18 | 0.34 | 0.49 | 0.68 | 1.64 | - | - | 0.34 | 0.59 | 0.84 | 2.24 | - | - |
| 14 | 0.98 | 0.98 | 0.91 | 0.95 | 0.95 | 0.75 | 0.20 | 0.22 | 0.26 | 0.35 | 0.56 | 1.05 | 0.18 | 0.18 | 0.22 | 0.29 | 0.50 | 1.13 |
| 15 | 0.93 | 1.00 | 0.95 | 0.88 | 1.01 | 0.92 | 0.20 | 0.23 | 0.24 | 0.29 | 0.33 | 0.63 | 0.18 | 0.20 | 0.21 | 0.26 | 0.27 | 0.62 |
| 16 | 0.89 | 0.88 | 0.71 | 0.68 | 0.69 | 0.77 | 0.31 | 0.43 | 0.60 | 1.55 | 1.65 | 1.62 | 0.33 | 0.49 | 0.77 | 2.14 | 2.23 | 1.88 |
| 17 | 0.99 | 1.05 | 0. 97 | 1.04 | 0.72 | 0.81 | 0.28 | 0.39 | 0.40 | 1.32 | 1.64 | 1.69 | 0.21 | 0.32 | 0.33 | 1.31 | 1.93 | 1.89 |
| 18 | 1.16 | 1.06 | 0.89 | 0.97 | 0.95 | 0.89 | 0.28 | 0.32 | 0.39 | 0.70 | 1.88 | 1.68 | 0.22 | 0.28 | 0.33 | 0.67 | 2.17 | 1.87 |
| 19 | 0.98 | 1.05 | 0.85 | 0.88 | 1.11 | 1.05 | 0.33 | 0.37 | 0.54 | 0.58 | 0.82 | 1.34 | 0.29 | 0.35 | 0.58 | 0.61 | 0.80 | 1.31 |
| 20 | 1.10 | 1.10 | 0.96 | 0.90 | 1.13 | 1.17 | 0.30 | 0.36 | 0.45 | 0.47 | 0.58 | 0.96 | 0.28 | 0.37 | 0.49 | 0.53 | 0.55 | 0.90 |

**Table 11**

| | Number of cells | | | | | | Neurite length per cell | | | | | | Proportion of neurite-bearing cells | | | | | |
|---|---|---|---|---|---|---|---|---|---|---|---|---|---|---|---|---|---|---|
| Compound number | 1 µM | 3 µM | 5 µM | 10 µM | 30 µM | 100 µM | 1 µM | 3 µM | 5 µM | 10 µM | 30 µM | 100 µM | 1 µM | 3 µM | 5 µM | 10 µM | 30 µM | 100 µM |
| 21 | 1.04 | 1.11 | 1.02 | 1.03 | 0.82 | 0.96 | 0.25 | 0.46 | 0.57 | 1.45 | 1.45 | 1.46 | 0.19 | 0.40 | 0.48 | 1.50 | 1.66 | 1.43 |
| 22 | 1.14 | 1.03 | 0.94 | 1.03 | 0.99 | 0.97 | 0.26 | 0.37 | 0.45 | 0.85 | 1.86 | 1.74 | 0.19 | 0.29 | 0.43 | 0.85 | 2.10 | 1.88 |
| 23 | 1.03 | 1.12 | 0.98 | 0.95 | 0.98 | 1.02 | 0.24 | 0.28 | 0.31 | 0.44 | 0.75 | 1.21 | 0.19 | 0.23 | 0.26 | 0.38 | 0.70 | 1.15 |
| 25 | 0. 97 | 1.03 | 0. 97 | 0. 97 | 0.98 | 0.94 | 0.24 | 0.33 | 0.35 | 0.40 | 0.48 | 0.88 | 0.22 | 0.31 | 0.33 | 0.38 | 0.45 | 0.87 |
| 26 | 1.02 | 1.00 | 0.93 | 0.90 | 0.98 | 1.01 | 0.26 | 0.26 | 0.29 | 0.31 | 0.34 | 0.77 | 0.25 | 0.24 | 0.27 | 0.30 | 0.32 | 0.75 |
| 27 | | 0.90 | 0.87 | 0.83 | 0.83 | 0.88 | | 0.19 | 0.25 | 0.29 | 0.29 | 0.42 | | 0.13 | 0.18 | 0.21 | 0.22 | 0.34 |
| 28 | 0. 92 | 0.86 | 0.71 | 0.68 | 0.77 | 0.86 | 0.20 | 0.21 | 0.26 | 0.30 | 0.32 | 0.45 | 0.17 | 0.18 | 0.27 | 0.30 | 0.30 | 0.40 |
| 29 | 1.07 | 0.98 | 0.96 | 0.92 | 0.97 | 0.93 | 0.25 | 0.29 | 0.28 | 0.36 | 0.46 | 0.60 | 0.18 | 0.24 | 0.24 | 0.31 | 0.42 | 0.54 |
| 30 | 0.88 | 0.91 | 0.83 | 0.87 | 0.95 | 0.98 | 0.27 | 0.31 | 0.36 | 0.39 | 0.39 | 0.76 | 0.22 | 0.26 | 0.37 | 0.41 | 0.37 | 0.70 |
| 31 | 0.99 | 0.93 | 0.75 | 0.77 | 0.84 | 0.93 | 0.29 | 0.27 | 0.33 | 0.32 | 0.34 | 0.40 | 0.28 | 0.28 | 0.38 | 0.34 | 0.36 | 0.40 |
| 32 | 0. 92 | 0.93 | 0.81 | 0.81 | 0.54 | 0.09 | 0.29 | 0.33 | 0.38 | 0.57 | 1.94 | - | 0.25 | 0.27 | 0.36 | 0.50 | 2.28 | - |
| 33 | 0. 92 | 0.90 | 0.83 | 0.86 | 0.91 | 0.80 | 0.26 | 0.30 | 0.36 | 0.53 | 0.59 | 0.94 | 0.19 | 0.26 | 0.34 | 0.51 | 0.58 | 0.95 |
| 34 | 1.06 | 1.01 | 0.96 | 0.98 | 0.98 | 0.97 | 0.28 | 0.37 | 0.53 | 0.76 | 1.21 | 0.51 | 0.24 | 0.33 | 0.54 | 0.75 | 1.29 | 0.48 |
| 35 | 0.94 | 0.94 | 0.99 | 0.82 | 0.16 | 0.31 | 0.26 | 0.33 | 0.50 | 0.82 | - | - | 0.20 | 0.29 | 0.51 | 0. 91 | - | - |
| 36 | 0.91 | 1.07 | 0.97 | 0.94 | 0.98 | 0.86 | 0.31 | 0.54 | 0.63 | 0.69 | 0.94 | 0.86 | 0.28 | 0.51 | 0.61 | 0.63 | 1.01 | 0.92 |
| 37 | 0.99 | 1.02 | 0.84 | 0.89 | 1.07 | 0.97 | 0.29 | 0.34 | 0.36 | 0.43 | 0.68 | 1.11 | 0.28 | 0.37 | 0.42 | 0.49 | 0.72 | 1.14 |
| 38 | 0.96 | 1.01 | 0.91 | 0.86 | 0.80 | 0.52 | 0.20 | 0.24 | 0.31 | 0.63 | 1.17 | 0.95 | 0.16 | 0.21 | 0.29 | 0.55 | 1.20 | 1.10 |
| 39 | 0.83 | 1.00 | 1.08 | 0.97 | 0.97 | 0.49 | 0.20 | 0.21 | 0.24 | 0.40 | 0.98 | - | 0.16 | 0.17 | 0.21 | 0.34 | 1.00 | - |
| 40 | 0.94 | 1.04 | 0.97 | 0.93 | 1.03 | 0.98 | 0.37 | 0.44 | 0.55 | 0.68 | 1.15 | 1.11 | 0.31 | 0.49 | 0.62 | 0.82 | 1.30 | 1.16 |

**Table 12**

| | Number of cells | | | | | | Neurite length per cell | | | | | | Proportion of neurite-bearing cells | | | | | |
|---|---|---|---|---|---|---|---|---|---|---|---|---|---|---|---|---|---|---|
| Compound number | 1 µM | 3 µM | 5 µM | 10 µM | 30 µM | 100 µM | 1 µM | 3 µM | 5 µM | 10 µM | 30 µM | 100 µM | 1 µM | 3 µM | 5 µM | 10 µM | 30 µM | 100 µM |
| 41 | 1.10 | 1.12 | 0.82 | 0.93 | 1.09 | 1.14 | 0.32 | 0.34 | 0.41 | 0.48 | 0.48 | 1.24 | 0.31 | 0.38 | 0.51 | 0.62 | 0.53 | 1.26 |
| 42 | 0.86 | 0.88 | 0.76 | 0.83 | 0.87 | 0.83 | 0.34 | 0.43 | 0.49 | 0.81 | 1.24 | 1.33 | 0.36 | 0.49 | 0.62 | 0.99 | 1.62 | 1.54 |
| 43 | 0. 97 | 0. 97 | 0.85 | 0.86 | 0.96 | 1.01 | 0.28 | 0.31 | 0.39 | 0.50 | 0.53 | 0.57 | 0.27 | 0.33 | 0.44 | 0.55 | 0.54 | 0.59 |
| 44 | 0.83 | 0.90 | 0.82 | 0.75 | 0. 91 | 0.89 | 0.30 | 0.39 | 0.46 | 0.59 | 0.89 | 1.09 | 0.29 | 0.38 | 0.48 | 0.62 | 0. 92 | 1.19 |
| 45 | 0.94 | 0.90 | 0.74 | 0.83 | 0.71 | 0.75 | 0.29 | 0.33 | 0.44 | 0.67 | 1.16 | 1.04 | 0.29 | 0.35 | 0.48 | 0.72 | 1.33 | 1.22 |
| 46 | 0.94 | 0.99 | 0.83 | 0.78 | 1.02 | 0.98 | 0.38 | 0.48 | 0.67 | 0.96 | 1.60 | 1.88 | 0.37 | 0.52 | 0.82 | 1.28 | 1.98 | 2.15 |
| 47 | 0.98 | 0. 91 | 0.85 | 0.89 | 0.88 | 0.87 | 0.27 | 0.28 | 0.31 | 0.34 | 0.33 | 0.39 | 0.27 | 0.28 | 0.31 | 0.34 | 0.35 | 0.42 |
| 48 | 0.85 | 0.93 | 0. 91 | 0.90 | 0.93 | 0.87 | 0.30 | 0.31 | 0.36 | 0.31 | 0.47 | 0.45 | 0.34 | 0.30 | 0.40 | 0.30 | 0.50 | 0.53 |
| 49 | 0.96 | 1.00 | 1.01 | 1.01 | 1.03 | 1.08 | 0.24 | 0.24 | 0.22 | 0.21 | 0.25 | 0.27 | 0.13 | 0.15 | 0.14 | 0.12 | 0.15 | 0.16 |
| 50 | 0. 97 | 0.96 | 0. 91 | 0.86 | 0.93 | 0.94 | 0.21 | 0.21 | 0.19 | 0.20 | 0.21 | 0.21 | 0.11 | 0.12 | 0.10 | 0.12 | 0.13 | 0.12 |
| 51 | 1.07 | 1.03 | 0.93 | 0.93 | 0.96 | 0.94 | 0.20 | 0.19 | 0.18 | 0.18 | 0.20 | 0.24 | 0.12 | 0.11 | 0.10 | 0.11 | 0.12 | 0.13 |
| 52 | 1.05 | 1.00 | 0.95 | 0.96 | 0.90 | 0.96 | 0.22 | 0.22 | 0.21 | 0.21 | 0.25 | 0.26 | 0.13 | 0.12 | 0.11 | 0.12 | 0.15 | 0.16 |
| 53 | 0. 91 | 0.95 | 0.94 | 0.96 | 1.05 | 1.15 | 0.22 | 0.22 | 0.20 | 0.20 | 0.22 | 0.22 | 0.12 | 0.13 | 0.12 | 0.11 | 0.12 | 0.13 |
| 54 | 0.93 | 1.12 | 0.87 | 0.90 | 0.04 | 0.05 | 0.20 | 0.30 | 0.38 | 0.61 | - | - | 0.15 | 0.27 | 0.35 | 0.61 | - | - |
| 55 | 1.05 | 1.02 | 0.85 | 0.85 | 0.69 | 0.25 | 0.21 | 0.33 | 0.44 | 0.53 | 0.62 | - | 0.13 | 0.26 | 0.39 | 0.48 | 0.67 | - |
| 56 | 0.94 | 1.06 | 0.93 | 0.95 | 0.95 | 0.93 | 0.25 | 0.40 | 0.55 | 0.62 | 0.62 | 0.53 | 0.19 | 0.35 | 0.51 | 0.62 | 0.56 | 0.44 |
| 57 | 0.96 | 0.93 | 0.86 | 0.90 | 0. 97 | 1.00 | 0.23 | 0.23 | 0.22 | 0.26 | 0.30 | 0.34 | 0.14 | 0.14 | 0.14 | 0.18 | 0.22 | 0.26 |
| 58 | 1.07 | 0. 92 | 0.88 | 0.13 | 0.03 | 0.43 | 0.23 | 0.30 | 0.25 | - | - | - | 0.20 | 0.34 | 0.24 | - | - | - |
| 59 | 0.90 | 0.93 | 0. 92 | 0.98 | 0.26 | 0.01 | 0.25 | 0.27 | 0.27 | 0.33 | - | - | 0.23 | 0.26 | 0.26 | 0.36 | - | - |
| 60 | 0.90 | 0. 91 | 0.80 | 0.78 | 0.84 | 0.79 | 0.30 | 0.30 | 0.34 | 0.40 | 0.41 | 0.67 | 0.27 | 0.30 | 0.40 | 0.46 | 0.45 | 0.70 |

**Table 13**

| | Number of cells | | | | | | Neurite length per cell | | | | | | Proportion of neurite-bearing cells | | | | | |
|---|---|---|---|---|---|---|---|---|---|---|---|---|---|---|---|---|---|---|
| Compound number | 1 µM | 3 µM | 5 µM | 10 µM | 30 µM | 100 µM | 1 µM | 3 µM | 5 µM | 10 µM | 30 µM | 100 µM | 1 µM | 3 µM | 5 µM | 10 µM | 30 µM | 100 µM |
| 61 | 0.84 | 0.82 | 0.87 | 0.77 | 0. 92 | 0.82 | 0.26 | 0.28 | 0.31 | 0.30 | 0.31 | 0.37 | 0.26 | 0.28 | 0.34 | 0.32 | 0.32 | 0.36 |
| 62 | 0. 92 | 0.89 | 0.83 | 0.78 | 0. 91 | 0. 91 | 0.30 | 0.29 | 0.32 | 0.28 | 0.29 | 0.32 | 0.30 | 0.29 | 0.36 | 0.30 | 0.30 | 0.33 |
| 63 | 1.13 | 1.05 | 0.84 | 0. 91 | 1.11 | 1.16 | 0.33 | 0.38 | 0.41 | 0.44 | 0.42 | 0.47 | 0.33 | 0.40 | 0.48 | 0.54 | 0.44 | 0.45 |
| 64 | 0.80 | 0.93 | 0.96 | 0.88 | 0.96 | 0.53 | 0.26 | 0.26 | 0.30 | 0.45 | 0.89 | 0.72 | 0.21 | 0.22 | 0.27 | 0.38 | 0. 91 | 0.82 |
| 65 | 1.05 | 1.09 | 0.86 | 0.87 | 1.05 | 1.09 | 0.33 | 0.38 | 0.44 | 0.51 | 0.47 | 0.47 | 0.30 | 0.40 | 0.52 | 0.64 | 0.54 | 0.50 |
| 66 | 1.15 | 1.11 | 0.80 | 0.79 | 0.39 | 0.04 | 0.31 | 0.31 | 0.32 | 0.28 | - | - | 0.31 | 0.33 | 0.38 | 0.33 | - | - |
| 67 | 0. 92 | 0.89 | 0.80 | 0.75 | 0.94 | 0. 93 | 0.31 | 0.32 | 0.32 | 0.32 | 0.34 | 0.34 | 0.33 | 0.37 | 0.39 | 0.38 | 0.38 | 0.32 |
| 68 | 0.85 | 0. 92 | 0.73 | 0.71 | 0.95 | 0.89 | 0.29 | 0.30 | 0.30 | 0.29 | 0.28 | 0.29 | 0.31 | 0.35 | 0.36 | 0.35 | 0.30 | 0.29 |
| 69 | 0.89 | 0.89 | 0.75 | 0.80 | 0.70 | 0.47 | 0.40 | 0.56 | 0.60 | 0.90 | 1.77 | - | 0.39 | 0.58 | 0.69 | 0. 97 | 2.18 | - |
| 70 | 0.84 | 0. 91 | 0. 91 | 0.86 | 0.79 | 0.73 | 0.47 | 0.41 | 0.47 | 0.61 | 1.05 | 1.16 | 0.45 | 0.40 | 0.49 | 0.68 | 1.13 | 1.24 |
| 71 | 0.87 | 0.94 | 0.87 | 0.79 | 0.39 | 0.07 | 0.40 | 0.52 | 0.56 | 1.09 | - | - | 0.39 | 0.51 | 0.61 | 1.28 | - | - |
| 72 | 0.84 | 0.85 | 0.78 | 0.80 | 0.41 | 0.38 | 0.44 | 0.49 | 0.62 | 1.03 | - | - | 0.41 | 0.48 | 0.68 | 1.18 | - | - |
| 73 | 0. 91 | 0.86 | 0.84 | 0.82 | 0.61 | 0.09 | 0.43 | 0.55 | 0.66 | 1.39 | 1.84 | - | 0.42 | 0.57 | 0.69 | 1.66 | 2.42 | - |
| 74 | 0.74 | 0.75 | 0.71 | 0.73 | 0.60 | 0.26 | 0.53 | 0.51 | 0.52 | 0.77 | 1.81 | - | 0.57 | 0.51 | 0.60 | 0.84 | 2.34 | - |
| 75 | 0.89 | 0.93 | 0.94 | 0.80 | 0.75 | 0.80 | 0.39 | 0.48 | 0.53 | 0.90 | 1.33 | 1.07 | 0.37 | 0.46 | 0.59 | 0.99 | 1.52 | 1.14 |

The results of this test showed that the compounds of the present disclosure had nerve growth stimulation effects.

## Claims

1. A compound represented by the following formula (I) or a salt thereof: 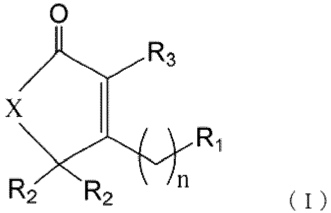 wherein
X represents -CH₂ -, -CH(CH₃)-, -CH₂-CH₂-, or -CH=CH-,
R₁ represents -CH₂OR₄, -CH₂OCON(R₅)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OR₇, or a halogen atom,
R₄ represents CₘH₂ₘ₊₁,
R₅s are identical or different and each represent a hydrogen atom or a methyl group,
R₆ represents a hydrogen atom or a methyl group,
R₇ represents a C1-C3 alkyl group,
m represents an integer of 1 to 14, and
n represents an integer of 1 to 15,
with the proviso that when R₄ is a linear alkyl group, the sum of m and n is an integer of 12 to 17, and when R₄ is a branched alkyl group, the sum of m_{b} and n is an integer of 12 to 17, wherein m_{b} is the number of carbon atoms of the longest linear chain of the branched alkyl group.

2. A compound represented by the following formula (I) or a salt thereof: 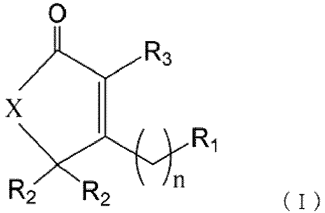 wherein
X represents -CH₂ -, -CH(CH₃)-, -CH₂-CH₂-, or -CH=CH-,
R₁ represents -CH₂OR₄, -CH₂OCON(R₅)₂, -CONHR₆, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OR₇, or a halogen atom,
R₄ represents CₘH₂ₘ₊₁,
R₅s are identical or different and each represent a hydrogen atom or a methyl group,
R₆ represents a hydrogen atom or a methyl group,
R₇ represents a C1-C3 alkyl group,
m represents an integer of 1 to 14, and
n represents an integer of 1 to 14,
with the proviso that when R₄ is a linear alkyl group, the sum of m and n is an integer of 12 to 15, and when R₄ is a branched alkyl group, the sum of m_{b} and n is an integer of 12 to 15, wherein m_{b} is the number of carbon atoms of the longest linear chain of the branched alkyl group.

3. A compound represented by the following formula (I) or a salt thereof: 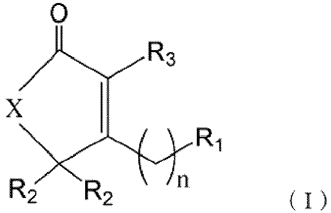 wherein
X represents -CH₂-, -CH(CH₃)-, -CH₂-CH₂-, or -CH=CH-,
R₁ represents -CH₂OR₄, -CH₂OCON(CH₃)₂, -CONHR₆, or a cyano group,
R₂s are identical or different and each represent a hydrogen atom or a methyl group, or two R₂s may form a cyclopropyl group together with the adjacent carbon atom, and
R₃ represents a hydrogen atom, a methyl group, -CH₂OCH₃, or an iodine atom,
with the proviso that when R₂s are identical or different and each represent a hydrogen atom or a methyl group, R₃ represents a hydrogen atom, a methyl group, -CH₂OCH₃, or an iodine atom, and when two R₂s form a cyclopropyl group together with the adjacent carbon atom, R₃ represents a methyl group, -CH₂OCH₃, or an iodine atom,
R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12, and
R₆ represents a hydrogen atom or a methyl group.

4. The compound or a salt thereof according to any one of claims 1 to 3, wherein X represents -CH₂-, -CH₂-CH₂-, or -CH=CHin formula (I).

5. The compound or a salt thereof according to any one of claims 1 to 4, wherein in formula (I),
R₁ represents -CH₂OR₄, -CONH₂, or a cyano group,
R₂s are identical or different and each represent a hydrogen atom or a methyl group, or two R₂s may form a cyclopropyl group together with the adjacent carbon atom,
R₃ represents a hydrogen atom, a methyl group, -CH₂OCH₃, or an iodine atom, with the proviso that when R₃ is a hydrogen atom, each R₂ is a hydrogen atom, when R₃ is a methyl group, -CH₂OCH₃, or an iodine atom, R₂s are identical or different and each represent a hydrogen atom or a methyl group, or two R₂s may form a cyclopropyl group together with the adjacent carbon atom, and
R₄ represents a methyl group, an ethyl group, an n-propyl group, or an isopropyl group, with the proviso that when R₄ is a methyl group, n represents an integer of 11 to 14, when R₄ is an ethyl group or an isopropyl group, n represents an integer of 11 to 13, and when R₄ is an n-propyl group, n is 11 or 12.

6. The compound or a salt thereof according to any one of claims 1 to 5, wherein in formula (I),
X represents -CH₂- or -CH₂-CH₂-,
R₂ represents -CH₂OR₄ or -CONH₂,
each R₂ is a methyl group,
R₃ represents a methyl group or -CH₂OCH₃, and
R₄ represents a methyl group, an ethyl group, or an isopropyl group, with the proviso that when R₄ is a methyl group, n represents an integer of 11 to 14, and when R₄ is an ethyl group or an isopropyl group, n represents an integer of 11 to 13.

7. The compound or a salt thereof according to any one of claims 1 to 6, wherein X represents -CH₂-CH₂- in formula (I) .

8. The compound or a salt thereof according to any one of claims 1 to 7, which is any one of the following compounds (1) to (10) :
(1) 3-(12-methoxydodecyl)-2,4,4-trimethylcyclohex-2-en-1-one,
(2) 3-(12-ethoxydodecyl)-2,4,4-trimethylcyclohex-2-en-1-one,
(3) 3-(12-isopropoxydodecyl)-2,4,4-trimethylcyclohex-2-en-l-one,
(4) 3-(13-methoxytridecyl)-2,4,4-trimethylcyclohex-2-en-1-one,
(5) 3-(13-ethoxytridecyl)-2,4,4-trimethylcyclohex-2-en-1-one,
(6) 3-(13-isopropoxytridecyl)-2,4,4-trimethylcyclohex-2-en-1-one,
(7) 3-(14-methoxytetradecyl)-2,4,4-trimethylcyclohex-2-en-1-one,
(8) 3-(14-ethoxytetradecyl)-2,4,4-trimethylcyclohex-2-en-1-one,
(9) 3-(14-isopropoxytetradecyl)-2,4,4-trimethylcyclohex-2-en-1-one, and
(10) 3-(15-methoxypentadecyl)-2,4,4-trimethylcyclohex-2-en-1-one.

9. The compound or a salt thereof according to any one of claims 1 to 8, wherein the compound is 3-(15-methoxypentadecyl)-2,4,4-trimethylcyclohex-2-en-1-one.

10. A neurite outgrowth agent comprising the compound or a salt thereof of any one of claims 1 to 9 as an active ingredient.

11. A therapeutic agent for a neurodegenerative disease, comprising the compound or a salt thereof of any one of claims 1 to 9 as an active ingredient.

12. A therapeutic agent for amyotrophic lateral sclerosis, comprising the compound or a salt thereof of any one of claims 1 to 9 as an active ingredient.

13. A therapeutic agent for pain, comprising the compound or a salt thereof of any one of claims 1 to 9 as an active ingredient.

14. A therapeutic agent for a lower urinary tract disfunction, comprising the compound or a salt thereof of any one of claims 1 to 9 as an active ingredient.

15. A pharmaceutical composition comprising
the compound or a salt thereof of any one of claims 1 to 9, and
a pharmaceutical carrier.
